# FASCICULE DE BREVET EUROPEEN

(11) **EP 0 839 133 B1**
(45) Date de publication et mention de la délivrance du brevet: **06.10.1999**
(21) Numéro de dépôt: 96924952.3
(22) Date de dépôt: 08.07.1996
(51) Int. Cl.: C07D 209/72, A61K 31/40, C07D 403/06, C07D 401/06, C07F 9/572, C07D 405/12, C07D 409/12, C07D 401/12, C07D 417/12

(54) **DERIVES DE 4,9-ETHANO-BENZO(f)ISOINDOLE COMME INHIBITEURS DE FARNESYL TRANSFERASE**
4,9-ETHANO-BENZO(F)ISOINDOLDERIVATE ALS FARNESYL TRANSFERASE INHIBITOREN
4,9-ETHANO-BENZO(F)ISOINDOLE DERIVATIVES AS FARNESYL TRANSFERASE INHIBITORS

(30) Priorité: 10.07.1995 FR 9508296
(43) Date de publication de la demande: 06.05.1998
(73) Titulaire: RHONE-POULENC RORER S.A., 92160 Antony (FR)
(72) Inventeur: COMMERCON, Alain, F-94400 Vitry-sur-Seine (FR); LEBRUN, Alain, F-91270 Vigneux (FR); MAILLIET, Patrick, F-94120 Fontenay-sous-Bois (FR); PEYRONEL, Jean-François, F-91120 Palaiseau (FR); SOUNIGO, Fabienne, F-75012 Paris (FR); TRUCHON, Alain, F-69003 Lyon (FR); ZUCCO, Martine, F-94320 Thiais (FR)
(86) Numéro de dépôt international: FR9601062
(87) Numéro de publication internationale: WO9703050

(56) Documents cités:
- WO-A-95/12612

## Description

La présente invention concerne de nouveaux produits de formule générale : 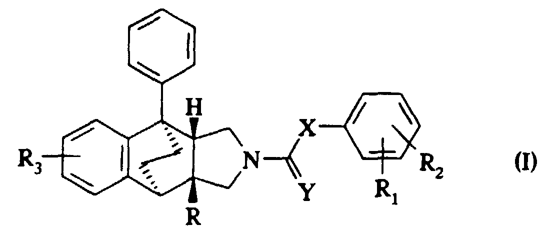 leur préparation et les compositions pharmaceutiques qui les contiennent.

Dans la formule générale (I),
R représente :
   - un radical de formule générale -(CH₂)ₘ-X₁-(CH₂)ₙ-Z dans lequel
      - X₁ représente une simple liaison ou un atome d'oxygène ou de soufre, m représente un nombre entier égal à 0 ou 1 et n représente un nombre entier égal à 0, 1 ou 2, les radicaux méthylènes pouvant être substitués par un radical carboxy, alcoxycarbonyle dont la partie alcoyle contient 1 à 4 atomes de carbone, carbamoyle, alcoylcarbamoyle dont la partie alcoyle contient 1 à 4 atomes de carbone, dialcoylcarbamoyle dont chaque partie alcoyle contient 1 à 4 atomes de carbone, amino, alcoylamino contenant 1 à 4 atomes de carbone, dialcoylamino dont chaque partie alcoyle contient 1 à 4 atomes de carbone et
      - Z représente
         - un radical carboxy,
         - un radical COOR₄, dans lequel R₄ représente un radical alcoyle droit ou ramifié content 1 à 3 atomes de carbone, ou
         - un radical CON(R₅)(R₆) dans lequel R₅ représente un atome d'hydrogène ou un radical alcoyle droit ou ramifié contenant 1 à 6 atomes de carbone et R₆ représente un atome d'hydrogène ou un radical alcoyle droit ou ramifié contenant 1 à 6 atomes de carbone éventuellement substitué par un radical amino, alcoylamino contenant 1 à 4 atomes de carbone, dialcoylamino dont chaque partie alcoyle contient 1 à 4 atomes de carbone, hydroxy, alcoxy contenant 1 à 4 atomes de carbone, mercapto, alcoylthio contenant 1 à 4 atomes de carbone, alcoyloxycarbonyle dont la partie alcoyle contient 1 à 4 atomes de carbone, carboxy, cyano, phényle éventuellement substitué par un ou plusieurs atomes ou radicaux, identiques ou différents, choisis parmi les atomes d'halogène et les radicaux alcoyles contenant 1 à 4 atomes de carbonyle, alcoxy contenant 1 à 4 atomes de carbone, dialcoylamino dont chaque partie alcoyle contient 1 à 4 atomes de carbone, trifluorométhyle ou cyano, naphtyl-1 ou -2, furyl-2 ou -3, thiényl-2 ou -3, imidazolyl-4 ou -5, thiazolyl-2, -4 ou - 5, thiazolidinyl-2, -4 ou -5 ou pyridyl-2, -3 ou -4, ou un radical indanyle ou thiochromanyle ou bien R₅ représente un atome d'hydrogène ou un radical alcoyle droit ou ramifié contenant 1 à 6 atomes de carbone et R₆ représente un radical hydroxy, amino ou alcoyloxy contenant 1 à 6 atomes de carbone en chaîne droite ou ramifiée éventuellement substitué par un radical phényle, ou
         - un radical PO(OR₇)₂ dans lequel R₇ représente un atome d'hydrogène ou un radical alcoyle droit ou ramifié contenant 1 à 6 atomes de carbone, ou
   - un radical -NH-CO-T dans lequel T représente un atome d'hydrogène ou un radical alcoyle droit ou ramifié contenant 1 à 6 atomes de carbone éventuellement substitué par un radical amino, carboxy, alcoyloxycarbonyle dont la partie alcoyle contient 1 à 4 atomes de carbone, hydroxy, alcoyloxy contenant 1 à 4 atomes de carbone, mercapto ou alcoylthio contenant 1 à 4 atoms de carbone, ou bien
   - un radical 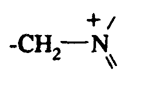 dans lequel 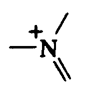 représente un radical pyridinium,
R₁ et R₂, identiques ou différents, représentent un atome d'hydrogène ou d'halogène ou un radical alcoyle contenant 1 à 4 atomes de carbone, alcoyloxy contenant 1 à 4 atomes de carbone eventuellement substitué par un radical dialcoylamino dont chaque partie alcoyle contient 1 à 4 atomes de carbone ou forme avec l'atome d'azote un hetérocycle saturé contenant 5 ou 6 atomes de carbone, alcoylthio contenant 1 à 4 atomes de carbone, alcoyloxycarbonyle dont la partie alcoyle contient 1 à 4 atomes de carbone, ou bien R₁ et R₂, situés en ortho l'un par rapport à l'autre, forment un hétérocycle saturé ou non saturé contenant 1 ou 2 hétéroatomes choisis parmi l'azote et l'oxygène, éventuellement substitue par un atome d'halogène ou par un radical alcoyle contenant 1 à 4 atomes de carbone ou alcoyloxy contenant 1 à 4 atomes de carbone, ou bien R₁ représente un atome d'hydrogène ou d'halogène ou un radical alcoyle contenant 1 à 4 atomes de carbone, alcoyloxy contenant 1 à 4 atomes de carbone éventuellement substitué par un radical dialcoylamino dont chaque partie alcoyle contient 1 à 4 atomes de carbone, alcoylthio contenant 1 à 4 atomes de carbone, alcoyloycarbonyle dont la partie alcoyle contient 1 à 4 atomes de carbone, et R₂ représente un radical thioalcoyle contenant 1 à 4 atomes de carbone, étant entendu que dans ce cas le produit de formule générale (I) se présente sous forme d'un produit doublé par l'intermédiaire d'un pont disulfure,
R₃ représente un atome d'hydrogène ou d'halogène (iode) ou un radical alcoyle contenant 1 à 4 atomes de carbone, alcényle contenant 2 à 4 atomes de carbone, alcoyloxy contenant 1 à 4 atomes de carbone ou alcoylthio contenant 1 à 4 atomes de carbone, carboxy ou alcoyloxycarbonyle dont la partie alcoyle contient de 1 à 4 atomes de carbone,
X représente un atome d'oxygène ou de soufre ou un groupement -NH-, -CO-, méthylène, éthylène, alcoylidène tel que vinylidène ou 1,1-cycloalcoyle contenant 3 à 6 atomes de carbone, et
Y représente un atome d'oxygène ou de soufre,
   sous forme racémique ainsi que les isomères optiques du produit de formule générale (I).

Selon l'invention, les nouveaux produits de formule générale (I) dans laquelle R, R₁, R₂ et R₃ sont définis comme précédemment, Y représente un atome d'oxygène ou de soufre et X représente un groupement -CO-, méthylène, éthylène, alcoylidène ou 1,1-cycloalcoyle, peuvent être obtenus par action d'un acide de formule générale : 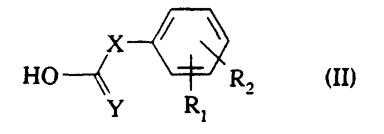 dans laquelle R₁, R₂ et X sont définis comme précédemment et Y représente un atome d'oxygène ou de soufre, de son ester méthylique ou d'un dérivé de cet acide tel qu'un halogénure ou l'anhydride, sur un produit de formule générale : 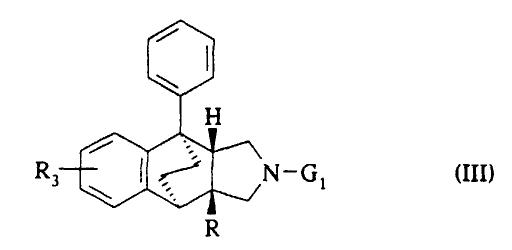 dans laquelle R et R₃ sont définis comme précédemment et G₁ représente un atome d'hydrogène (qui peut être obtenu à partir d'un produit de formule générale (III) dans laquelle G₁ représente un groupement protecteur d'une fonction amino tel qu'un radical benzyle, benzyloxycarbonyle ou tert.butoxycarbonyle par hydrogénolyse en présence d'un catalyseur tel que le palladium sur charbon, lorsque G₁ représente un radical benzyle ou benzyloxycarbonyle, ou par hydrolyse en milieu acide, lorsque G₁ représente un radical tert.butoxycarbonyle ou benzyloxycarbonyle), suivi éventuellement, lorsque R représente ou contient un radical -COOR₄ ou -PO(OR₇)₂ dans lesquels R₄ et R₇ représentent un radical alcoyle, de la saponification du produit obtenu pour obtenir un produit de formule générale (I) dans laquelle R représente ou contient un radical carboxy ou de la transformation au moyen d'agent nucléophile du produit obtenu pour obtenir un produit de formule générale (I) dans laquelle R représente ou contient un radical -PO₃H₂.

Généralement, la réaction du produit de formule générale (II) sous forme acide sur le produit de formule générale (III) est effectuée en opérant dans un solvant organique tel qu'un hydrocarbure aliphatique halogéné tel que le dichlorométhane en présence d'un agent de condensation et/ou d'activation tel que le chlorhydrate de 1-éthyl-3-[3-(diméthylamino) propyl] carbodiimide, l'hydroxybenzotriazole, le 1,3-dicyclohexylcarbodiimide ou l'hexafluorophosphate de benzotriazol-1-yloxytris (diméthylamino)phosphonium à une température comprise entre 0 et 50°C.

Généralement, la réaction du produit de formule générale (II) sous forme d'ester méthylique sur un produit de formule générale (III) est effectuée en opérant dans un solvant organique tel que le dioxane ou un hydrocarbure aliphatique halogéné tel que le dichlorométhane à une température comprise entre 0°C et la température de reflux du mélange réactionnel.

Généralement, la réaction du produit de formule générale (II) sous forme d'halogénure sur le produit de formule générale (III) est effectuée en opérant dans un solvant organique tel qu'un hydrocarbure aliphatique halogéné tel que le dichlorométhane en présence d'une base (amine aliphatique tertiaire) à une température comprise entre 0 et 50°C.

Généralement, la réaction du produit de formule générale (II) sous forme d'anhydride sur le produit de formule générale (III) est effectuée en opérant dans un solvant organique tel qu'un hydrocarbure aliphatique halogéné comme le dichlorométhane en présence d'une base (amine aliphatique tertiaire, pyridine ou 4-diméthylaminopyridine) à une température comprise entre 0 et 50°C.

Généralement la saponification d'un produit de formule générale (I) dans laquelle R représente ou contient un ester de formule générale -COOR₄ en produit de formule générale (I) dans laquelle R représente ou contient un radical carboxy est effectuée au moyen d'une base minérale telle que la soude ou la potasse ou le carbonate de sodium dans un solvant organique tel qu'un alcool comme le méthanol ou l'éthanol.

Généralement la transformation d'un produit de formule générale (I) dans laquelle R représente ou contient un radical PO(OR₇)₂ en produit de formule générale (I) dans laquelle R représente ou contient un radical PO₃H₂ s'effectue par action d'un agent nucléophile tel qu'un halogénune (iodure) de trialcoylsilyle (triméthylsilyle) ou d'un halogénure de sodium ou de lithium (iodure de sodium) en présence d'un trialcoylhalogénosilane (triméthylchlorosilane, triméthylbromosilane) en opérant dans un solvant polaire (tétrachlorure de carbone, acétonitrile) à une température comprise entre 0 et 50°C ou par chauffage avec un halogénure de métal alcalin (iodure de sodium), suivie d'une hydrolyse.

Selon l'invention, les nouveaux produits de formule générale (I) dans laquelle Y représente un atome d'oxygène ou de soufre et X représente un atome d'oxygène peuvent être obtenus par action d'un halogénoformiate ou d'un halogénothioformiate de formule générale : 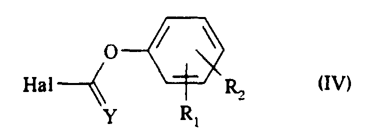 dans laquelle Y représente un atome d'oxygène ou de soufre, R₁ et R₂ sont définis comme précédemment et Hal représente un atome d'halogène, sur un produit de formule générale (III), puis eventuellement, lorsque R représente ou contient un radical -COOR₄ ou -PO(OR₇)₂, saponifie le produit obtenu pour obtenir un produit de formule générale (I) dans laquelle R représente ou contient un radical carboxy ou transforme au moyen d'agent nucléophile le produit obtenu en un produit de formule générale (I) dans laquelle R représente ou contient un radical -PO₃H₂.

Généralement, la réaction de l'halogénure de formule générale (IV) sur le produit de formule générale (III) est effectuée en milieu organique ou hydro-organique tel qu'un mélange dioxane-eau en présence d'une base minérale (soude) ou organique (triéthylamine) à une température comprise entre 0 et 50°C.

Selon l'invention, les nouveaux produits de formule générale (I) dans laquelle Y représente un atome d'oxygène ou de soufre et X représente un groupement NH peuvent être obtenus par action d'un isocyanate ou d'un isothiocyanate de formule générale : 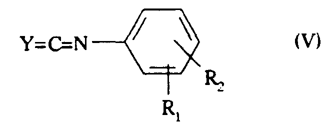 dans laquelle Y représente un atome d'oxygène ou de soufre, R₁ et R₂ sont définis comme précédemment sur un produit de formule générale (III), suivi éventuellement, lorsque R représente ou contient un radical -COOR₄ ou -PO(OR₇)₂ dans lesquels R₄ ou R₇ représentent un radical alcoyle, de la saponification du produit obtenu pour obtenir un produit de formule générale (I) dans laquelle R représente ou contient un radical carboxy ou de la transformation au moyen d'agent nucléophile du produit obtenu pour obtenir un produit de formule générale (I) dans laquelle R représente ou contient un radical -PO₃H₂.

Généralement, la réaction du produit de formule générale (V) sur le produit de formule générale (III) est effectuée dans un solvant organique inerte tel que le tétrahydrofurane ou le toluène en présence d'un agent de condensation tel que la 4-diméthylamino-pyridine ou la 4-pyrrolidino-pyridine à une température comprise entre 0 et 50°C.

Le remplacement éventuel des radicaux -COOR₄ et PO(OR₇)₂ respectivement par des radicaux carboxy et PO₃H₂ s'effectue dans les conditions décrites précédemment.

Selon l'invention, les nouveaux produits de formule générale (I) dans laquelle R₁, R₂ et R₃ sont définis comme précédemment, X représente un atome d'oxygène ou de soufre ou un groupement -NH-, -CO-, méthylène, éthylène, alcoylidène ou 1,1-cycloalcoyle, et Y représente un atome d'oxygène ou de soufre et R représente un radical de formule générale -(CH₂)ₘ-X₁-(CH₂)ₙ -Z dans lequel X₁, m et n sont définis comme précédemment et Z représente un radical -COOR₄ dans lequel R₄ représente un radical alcoyle droit ou ramifié contenant 1 à 3 atomes de carbone peuvent être obtenus par estérification d'un produit de formule générale (I) dans laquelle R₁, R₂, R₃, X, Y, X₁, m et n sont définis comme précédemment et Z représente un radical carboxy.

Généralement, l'estérification est effectuée au moyen d'un alcool de formule générale R₄-OH dans laquelle R₄ est défini comme précédemment en opérant en milieu acide ou au moyen d'un halogénure d'alcoyle de formule générale R₄-Hal dans laquelle Hal représente un atome d'halogène (iode) en opérant en milieu alcalin (carbonate de métal alcalin ou alcalino-terreux tel que le carbonate de césium) en opérant dans un solvant organique tel que le diméthylformamide à une température comprise entre 0 et 50°C.

Selon l'invention, les nouveaux produits de formule générale (I) dans laquelle R₁, R₂ et R₃ sont définis comme précédemment, X représente un atome d'oxygène ou de soufre ou un groupement -NH-, -CO-, méthylène, éthylène, alcoylidène ou 1,1-cycloalcoyle, et Y représente un atome d'oxygène ou de soufre et R représente un radical de formule générale -(CH₂)ₘ-X₁-(CH₂)ₙ -Z dans laquelle X₁, m et n sont définis comme précédemment et Z représente un radical -CON(R₅)(R₆) dans lequel lequel R₅ et R₆, identiques ou différents, représentent un atome d'hydrogène ou un radical alcoyle droit ou ramifié contenant 1 à 6 atomes de carbone et R₆ représente un atome d'hydrogène ou un radical alcoyle droit ou ramifié contenant 1 à 6 atomes de carbone éventuellement substitué par un radical amino, alcoylamino contenant 1 à 4 atomes de carbone, dialcoylamino dont chaque partie alcoyle contient 1 à 4 atomes de carbone, alcoxy contenant 1 à 4 atomes de carbone, alcoylthio contenant 1 à 4 atomes de carbone, alcoyloxycarbonyle dont la partie alcoyle contient 1 à 4 atomes de carbone, carboxy, cyano, phényle éventuellement substitué par un ou plusieurs radicaux identiques ou différents choisis parmi les radicaux alcoxy contenant 1 à 4 atomes de crabone ou trifluorométhyle, naphtyl-1 ou -2, furyl-2 ou -3, thiényl-2 ou -3, imidazolyl-4 ou -5 ou thiazolyl-4 ou -5, pyridyl-2, - 3 ou -4, ou un radical indanyle ou chromanyle, ou bien R₅ représente un atome d'hydrogène ou un radical alcoyle droit ou ramifié contenant 1 à 6 atomes de carbone et R₆ représente un radical hydroxy, amino ou alcoyloxy contenant 1 à 6 atomes de carbone en chaîne droite ou ramifiée éventuellement substitué par un radical phényl, peuvent être obtenus par action d'un produit de formule générale HN(R₅)(R₆) dans laquelle R₅ et R₆ sont définis comme ci-dessus sur un produit de formule générale (I) dans laquelle R₁, R₂, R₃, X, Y, X₁, m et n sont définis comme précédemment et Z représente un radical carboxy.

Lorsque R₅ et R₆ représentent chacun un atome d'hydrogène, il est particulièrement avantageux de faire réagir l'ammoniac éventuellement en solution dans un solvant organique tel qu'un alcool (éthanol) en présence d'un agent de condensation tel que le N,N'-carbonyldiimidazole, le 1,1-dicyclohexylcarbodiimide, le chorhydrate de 1-éthyl-3-[3-(diméthylamino)propyl]carbodiimide, l'hydroxybenzotriazole ou l'hexafluorophosphate de benzotriazol-1-yloxytris(diméthylamino) phosphonium à une température comprise entre 0 et 50°C.

Lorsque R₅ et R₆ ne représentent pas chacun un atome d'hydrogène, il est particulièrement avantageux de faire réagir l'amine de formule générale HN(R₅)(R₆) en présence d'un agent de condensation et/ou d'un agent d'activation tels que le chlorhydrate de 1-éthyl-3-[3-(diméthylamino)propyl]carbodiimide, l'hydroxybenzotriazole, le 1,1-dicyclohexylcarbodiimide, ou l'hexafluorophosphate de benzotriazol-1-yloxytris(diméthylamino)phosphonium en opérant dans un solvant organique tel qu'un hydrocarbure aliphatique halogéné (dichlorométhane) à une température comprise entre 0 et 50°C.

Lorsque l'un au moins des symboles R₅ et R₆ est substitué par un radical amino, il est particulièrement avantageux de le protéger par un groupement protecteur tel qu'un radical tert-butoxycarbonyle, benzyloxycarbonyle ou benzyle préalablement à la condensation de l'amine de formule générale HN(R₅)(R₆) sur l'acide approprié puis à remplacer le groupement protecteur par un atome d'hydrogène par exemple par hydrogénolyse au moyen d'hydrogène en présence d'un catalyseur tel que le palladium sur charbon, lorsqu'il représente un radical benzyle ou benzyloxycarbonyle, ou par hydrolyse en milieu acide, lorsqu'il représente un radical tert.butoxycarbonyle ou benzyloxycarbonyle.

Lorsque l'un au moins des symboles R₅ et R₆ est substitué par un radical carboxy, il est particulièrement avantageux de le protéger par un groupement protecteur tel qu'un radical alcoyle éventuellement substitué par un radical phényle tel que le radical benzyle préalablement à la condensation de l'amine de formule générale HN(R₅)(R₆) sur l'acide approprié puis à remplacer le groupement protecteur par un atome d'hydrogène par exemple par hydrogénolyse au moyen d'hydrogène en présence d'un catalyseur tel que le palladium sur charbon ou par saponification dans les conditions décrites ci-dessus.

Lorsque, dans le produit de formule générale (I), R₅ représente un atome d'hydrogène ou un radical alcoyle contenant 1 à 6 atomes de carbone et R₆ représente un radical alcoyloxy substitué par un radical phényle, le remplacement du radical alcoyloxy substitué par un radical phényle par un radical hydroxy, effectué, par exemple, par hydrogénolyse en présence d'un catalyseur tel que le palladium sur charbon, permet d'obtenir un produit de formule générale (I) dans laquelle R₅ représente un atome d'hydrogène ou un radical alcoyle contenant 1 à 4 atomes de carbone et R₆ représente un radical hydroxy.

Selon l'invention, les nouveaux produits de formule générale (I) dans laquelle R₁, R₂ et R₃ sont définis comme précédemment, X représente un atome d'oxygène ou de soufre ou un groupement -NH-, -CO-, méthylène, éthylène, alcoylidène ou 1,1-cyclopalcoyle, et Y représente un atome d'oxygène ou de soufre et R représente un radical de formule générale -NHCO-T dans laquelle T représente un atome d'hydrogène ou un radical alcoyle (1 à 6 atomes de carbone) éventuellement substitué par un radical amino, carboxy, alcoyloxycarbonyle, hydroxy, alcoyloxy, mercapto ou alcoylthio peuvent être obtenus par action d'un acide de formule générale T-CO-OH dans laquelle T est défini comme ci-dessus sur un produit de formule générale : 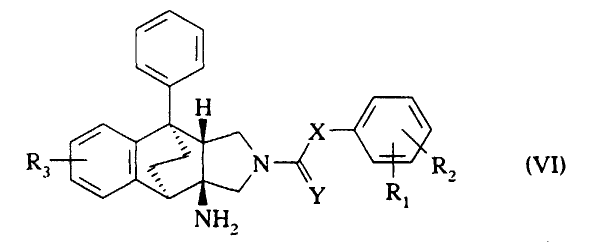 dans laquelle R₁, R₂, R₃, X et Y sont définis comme précédemment, suivi éventuellement du remplacement des fonctions esters ou des fonctions amines protégées portées par T respectivement par des radicaux carboxy ou amino dans les conditions décrites précédemment.

Généralement, la réaction de l'acide de formule générale T-CO-OH sous forme acide sur le produit de formule générale (VI) est effectuée en opérant dans un solvant organique tel qu'un hydrocarbure aliphatique halogéné tel que le dichlorométhane en présence d'un agent de condensation et/ou d'activation tels que le chlorhydrate de 1-éthyl-3-[3-(diméthylamino)propyl]carbodiimide, l'hydroxybenzotriazole, le 1,1-dicyclohexylcarbodiimide ou l'hexafluorophosphate de benzotriazol-1-oxy tris(diméthylaminophosphonium à une température comprise entre 0 et 50°C.

Généralement, la réaction de l'acide de formule générale T-CO-OH sous forme d'halogénure sur le produit de formule générale (VI) est effectuée en opérant dans un solvant organique tel qu'un hydrocarbure aliphatique halogéné tel que le dichlorométhane en présence d'une base (amine aliphatique tertiaire) à une température comprise entre 0 et 50°C.

Généralement, la réaction de l'acide de formule générale T-CO-OH sous forme d'anhydride sur le produit de formule générale (VI) est effectuée en opérant dans un solvant organique tel qu'un hydrocarbure aliphatique halogéné tel que le dichlorométhane en présence d'une base (amine aliphatique tertiaire, pyridine ou 4-diméthylaminopyridine) à une temperature comprise entre 0 et 50°C.

Lorsque T est substitué par un radical amino, il est particulièrement avantageux de le protéger par un groupement protecteur tel qu'un radical benzyloxycarbonyle ou tert-butoxycarbonyle préalablement à la condensation de l'acide de formule générale T-CO-OH sur l'amine appropriée puis à remplacer le groupement protecteur par un atome d'hydrogène par exemple par hydrogénolyse au moyen d'hydrogène en présence d'un catalyseur tel que le palladium sur charbon ou par hydrolyse en milieu acide.

Lorsque T est substitué par un radical carboxy, il est particulièrement avantageux de le protéger par un groupement protecteur tel qu'un radical méthyle, éthyle ou benzyle préalablement à la condensation de l'acide de formule générale T-CO-OH sur l'amine appropriée puis à remplacer le groupement protecteur par un atome d'hydrogène par exemple par hydrogénolyse au moyen d'hydrogène en présence d'un catalyseur tel que le palladium sur charbon ou par saponification dans les conditions décrites précédemment.

Selon l'invention, les nouveaux produits de formule générale (I) dans laquelle R représente un radical de formule 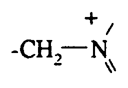 peuvent être obtenus par action d'un excès d'une amine tertiaire et d'un acide fort ou d'un dérivé de cet acide sur un produit de formule générale : 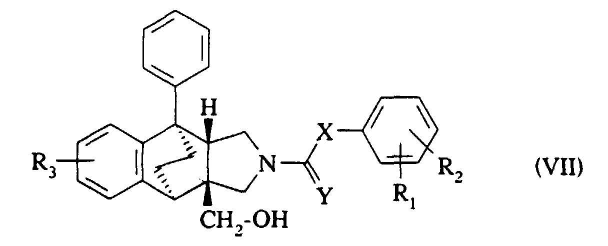 dans laquelle R₁, R₂, R₃, X et Y sont définis comme précédemment.

De préférence, l'amine tertiaire est la pyridine et l'acide fort est l'acide trifluorométhanesulfonique éventuellement sous forme d'anhydride.

Selon l'invention, les nouveaux produits de formule générale (I) dans laquelle Y représente un atome de soufre peuvent être obtenus par thionation d'un produit de formule générale (I) dans laquelle Y représente un atome d'oxygène.

Généralement, la thionation est effectuée dans les conditions habituelles au moyen de pentasulfure de phosphore en opérant dans un solvant organique tel que le tétrahydrofurane à une température comprise entre 0 et 50°C.

Selon l'invention, les produits de formule générale (I) dans laquelle l'un des symboles R₁ ou R₂ représente un radical alcoxycarbonyle peuvent être obtenus par acylation d'un produit de formule générale (I) dans laquelle l'un des symboles R₁ ou R₂ représente un radical hydroxy au moyen d'un acide aliphatique ou d'un dérivé de cet acide tel qu'un halogénure ou l'anhydride dans les conditions habituelles d'estérification.

Les produits de formule générale (III) dans laquelle R représente un radical carboxy ou un radical de formule générale COOR₄ peuvent être obtenus par action de l'acide trifluorométhanesulfonique sur un produit de formule générale : 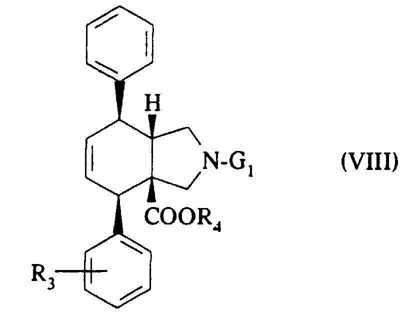 dans laquelle R₃ est défini comme précédemment, G₁ représente un radical benzyle, R₄ représente un radical alcoyle contenant 1 à 3 atomes de carbone, suivie éventuellement de la saponification du produit obtenu, et suivie éventuellement selon le cas du remplacement du radical benzyle par un atome d'hydrogène, par hydrogénolyse dans les conditions décrites ci-dessus puis éventuellement selon le cas du remplacement de l'atome d'hydrogène par un radical tert-butoxycarbonyle, par action de l'anhydride tert-butoxycarbonique dans un solvant organique, ou par un radical benzyloxycarbonyle, par action du chlorure de benzyloxycarbonyle dans un solvant organique.

Le produit de formule générale (VIII) peut être obtenu par action d'une N-trialcoylsilylméthyl-N-alcoxyméthyl-amine portant un groupement protecteur de la fonction amine tel qu'un radical benzyle, telle que la N-triméthylsilylméthyl-N-n.butoxyméthyl-benzylamine qui peut être préparée dans les conditions décrites dis Chem. Pharm. Bull. , 276 (1985), sur un dérivé du cyclohexadiène de formule générale : 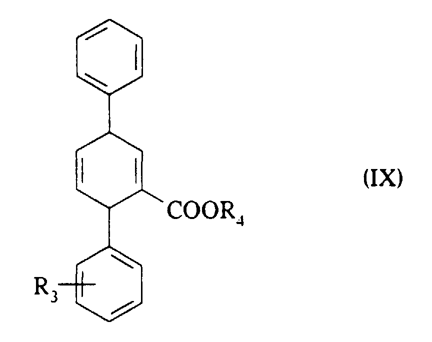 dans laquelle R₃ et R₄ sont définis comme ci-dessus.

Généralement, la réaction est effectuée dans un solvant organique tel qu'un hydrocarbure aliphatique halogéné comme le dichlorométhane en présence d'un acide fort tel que l'acide trifluorométhanesulfonique à une température comprise entre 0 et 50°C.

Les produits de formule générale (III) dans laquelle R représente un radical (CH₂)ₘ-X₁(CH₂)ₙ-Z dans lequel m est égal à 0, X₁ représente une liaison, n est égal à 0 et Z représente un radical -COOR₄ ou -CON(R₅)(R₆) peuvent être obtenus à partir d'un produit de formule générale (VIII) dans laquelle R représente un radical carboxy par estérification et amidification dans les conditions décrites ci-dessus.

Le produit de formule générale (IX) peut être obtenu par réaction de Diels-Alder entre un 1,4-diphényl-butadiène dont un des radicaux phényles est éventuellement substitué par un radical R₃ tel que défini précédemment et l'acide propiolique par chauffage éventuellement en présence d'hydroquinone, suivie de l'estérification du produit obtenu.

Les produits de formule générale (III) dans laquelle m est égal à 1, X₁ représente une liaison, n est égal à 0 et Z représente un radical -COOR₄, -CON(R₅)(R₆) ou PO(OR₇)₂ peuvent être obtenus à partir d'un produit de formule générale : 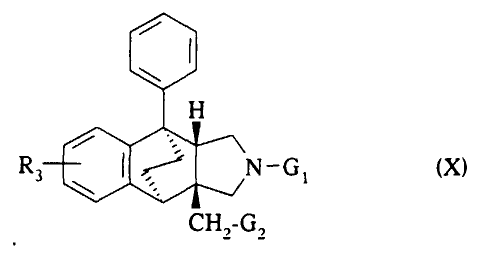 dans laquelle R₃ est défini comme précédemment G₁ représente un groupement protecteur de la fonction amine (benzyle, benzyloxycarbonyle ou tert-butoxycarbonyle) et G₂ représente un groupement partant tel qu'un radical trifluorométhylsulfonyloxy.

Plus particulièrement, pour obtenir un produit de formule générale (III) dans laquelle Z représente un radical carboxy, -COOR₄ dans lequel R₄ représente un radical alcoyle ou -CON(R₅)(R₆), il est particulièrement avantageux de passer par l'intermédiaire du nitrile correspondant, qui peut être obtenu par action d'un cyanure de métal alcalin sur le produit de formule générale (X) en opérant dans un solvant organique polaire tel que le diméthylsulfoxyde à une température comprise entre 0 et 50°C, qui est hydrolysé en acide correspondant qui peut alors être estérifié ou amidifié dans les conditions habituelles.

Plus particulièrement, pour obtenir un produit de formule générale (III) dans laquelle Z représente un radical -PO(OR₇)₂, il est particulièrement avantageux de faire réagir un trialcoylphosphite sur un produit de formule générale (X), puis à transformer éventuellement le phosphonate obtenu en acide phosphonique correspondant.

Les produits de formule générale (III) dans laquelle R représente un radical (CH₂)ₘ-X₁-(CH₂)ₙ-Z dans lequel m est égal à 1, X₁ représente un atome d'oxygène ou de soufre, n est égal à 1 ou 2, G₁ représente un groupement protecteur de la fonction amine et Z représente un radical carboxy, -COOR₄ dans lequel R₄ représente un radical alcoyle ou -CON(R₅)(R₆) peuvent être obtenus par action d'un ester ou d'un amide de formule générale :

H-X₁-(CH₂)ₙ-Z (XI)

dans laquelle X₁, n et Z sont définis comme ci-dessus sur un produit de formule générale (X) en opérant dans un solvant organique anhydre tel que le dioxanne en présence d'un hydrure de métal alcalin tel que l'hydrure de sodium, suivie éventuellement selon le cas de la saponification du produit de formule générale (III) ainsi obtenu.

Les produits de formule générale (III) dans laquelle m est égal à 1, X₁ représente une liaison, n est égal à 1, le groupement méthylène pouvant être substitué par un radical carboxy ou alcoxycarbonyle ou carbamoyle ou alcoylcarbamoyle ou dialcoylcarbamoyle, G₁ représente un groupement protecteur de la fonction amine et Z représente un radical carboxy, -COOR₄ dans lequel R₄ représente un radical alcoyle ou -CON(R₅)(R₆) peuvent être obtenus par action d'un acide malonique préalablement anionisé, ou d'un dérivé de l'acide malonique, de préférence un diester, sur un produit de formule générale (X) en opérant dans un solvant organique anhydre tel que le dioxanne en présence d'un hydrure de métal alcalin tel que l'hydrure de sodium, à une température comprise entre 0°C et la température de reflux du mélange réactionnel, suivie selon le cas de la saponification, de l'estérification, de l'amidification ou de la décarboxylation du produit de formule générale (III) ainsi obtenu.

Les produits de formule générale (III) dans laquelle R représente un radical -NH-CO-T dans lequel T est défini comme précédemment peuvent être obtenus par amidification d'un produit de formule générale (III) dans laquelle R₃ est défini comme précédemment, G₁ représente un groupement protecteur de la fonction amine et R représente un radical amino au moyen d'un acide de formule générale T-CO-OH dans laquelle T est défini comme précédemment dans les conditions décrites ci-dessus pour l'amidification d'un produit de formule générale (VI).

Les produits de formule générale (III) dans laquelle R représente un radical amino ou les produits de formule générale (VI) peuvent être obtenus selon les méthodes qui permettent de transformer un radical carboxy en radical amino sans toucher au reste de la molécule.

Généralement, la fonction carboxy d'un produit de formule générale (III) ou (I) est transformée en radical amino en passant par un isocyanate qui peut être obtenu par pyrolyse de l'azoture d'acide qui peut lui-même être obtenu par action d'un azoture de métal alcalin sur l'halogénure d'acide correspondant.

Les produits de formule générale (III) dans laquelle R représente un radical de formule générale 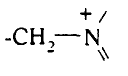 peuvent être obtenus par action d'un excès d'une amine tertiaire et d'un acide fort ou d'un dérivé de cet acide sur un produit de formule générale : 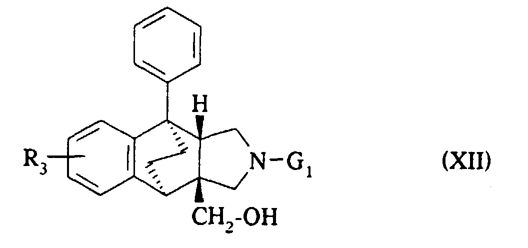 dans laquelle R₃ est défini comme précédemment et G₁ représente un groupement protecteur de la fonction amine.

Les produits de formule générale (VII) ou de formule générale (XII) peuvent être obtenus respectivement par réduction d'un produit de formule générale (I) ou d'un produit de formule générale (III) dans laquelle R représente un radical de formule générale -COOR₄ dans laquelle R₄ représente de préférence un radical alcoyle contenant 1 à 3 atomes de carbone.

Généralement, la réduction est effectuée au moyen d'un hydrure double de lithium et d'aluminium en opérant dans un solvant organique tel qu'un éther comme le tétrahydrofurane à une temperature comprise entre 0 et 50°C.

Les produits de formule générale (III) dans laquelle R₃ représente un atome d'iode et G₁ représente un groupement protecteur de la fonction amine peuvent être obtenus par iodation d'un produit de formule générale (III) dans laquelle R₃ représente un atome d'hydrogène et G₁ représente un groupement protecteur de la fonction amine.

Généralement, l'iodation peut être effectuée, ou bien au moyen d'iode en présence de bis-(trifluoroacétoxy)iodobenzène en opérant dans un solvant organique tel qu'un hydrocarbure aliphatique halogéné comme le dichlorométhane à une température comprise entre 0 et 50°C, ou bien par action de l'iode en présence de nitrate d'ammonium cérique dans l'acide acétique ou le méthanol, ou bien par action de l'iode en présence de trifluoroacétate d'argent, ou bien par action du N-iodosuccinimide en présence d'hydroxy toxyloxy iodobenzène (réactif de Koser) dans le méthanol, ou bien par action du dichloroiodure de benzyltriméthylammonium en présence de chlorure de zinc dans l'acide acétique.

Les produits de formule générale (III) dans laquelle R₃ représente un radical alcoyle, alcényle, alcoyloxy, alcoylthio, carboxy ou alcoyloxycarbonyle peuvent être obtenus selon les méthodes connues qui permettent de remplacer un atome d'iode par un radical alcoyle, alcényle, alcoyloxy, alcoylthio, carboxy ou alcoyloxycarbonyle.

Les produits de formule générale (III) dans laquelle R₃ représente un radical alcoyle peuvent être obtenus par action d'un alcoylure de métal alcalin sur un produit de formule générale (III) dans laquelle R₃ représente un atome d'iode en opérant dans un solvant organique inerte tel qu'un éther.

Les produits de formule générale (III) dans laquelle R₃ représente un radical alcényle peuvent être obtenus par action d'un acide boronique de formule générale R₃-B(OH)₂ ou d'un stannane de formule générale R₃-Sn(R')₃, dans lequel R' représente un radical alcoyle contenant de 1 à 4 atomes de carbone, en présence d'un catalyseur tel que le palladium associé à un ligand (triphénylphosphine) dans un solvant aromatique (benzène, toluène, xylène) à une température comprise entre 0 et 100°C, sur un produit de formule générale (III) dans laquelle R₃ représente un atome d'iode.

Les produits de formule générale (III) dans laquelle R₃ représente un radical alcoyloxy ou alcoylthio peuvent être obtenus par action d'un alcoolate ou d'un sulfénate de métal alcalin sur un produit de formule générale (III) dans laquelle R₃ représente un atome d'iode.

Les produits de formule générale (III) dans laquelle R₃ représente un radical carboxy peuvent être obtenus par oxydation, par exemple au moyen de perborate de sodium, d'un produit de formule formule générale (III) dans laquelle R₃ représente un radical formyle qui peut être lui-même obtenu par ozonolyse d'un produit de formule générale (III) dans laquelle R₃ représente un radical alcényle.

Les produits de formule générale (III) dans laquelle R₃ représente un radical alcoyloxycarbonyle peuvent être obtenus par estérification d'un produit de formule générale (III) dans laquelle R₃ représente un radical carboxy.

Les isomères des produits de formule générale (I) peuvent être obtenus selon les méthodes habituelles de séparation à partir du poduit racémique correspondant. Il est particulièrement avantageux d'effectuer la séparation par chromatographie en phase liquide à haute performance en utilisant une phase stationnaire chirale de type Pirkle modifiée en éluant avec un solvant convenable.

Comme phase stationnaire chirale peut être utilisée de préférence une phase dont le sélecteur chiral, qui est de préférence la 3,5-dinitro-phénylalanine, est éloigné de la silice par un bras aminoalcoyle contenant 3 à 14 atomes de carbone fixé sur les fonctions amines d'une silice aminopropyle et dont les fonctions silanols libres sont bloquées par des radicaux trialcoylsilyles.

Cette phase chirale peut être définie par la structure suivante : 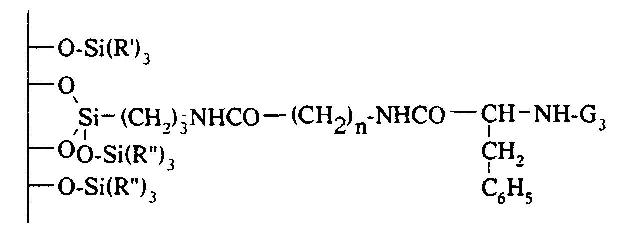 dans laquelle les symboles R', identiques ou différents, et R'', identiques ou différents, représentent des radicaux alcoyles contenant 1 à 10 atomes de carbone, G₃ représente un groupe électo-attracteur et n représente un nombre entier compris entre 3 et 13 inclusivement, dont la porosité est voisine de 100 Å

La phase chirale peut être préparée par action sur une silice aminopropyle de l'anhydride d'un acide aminoalcanoïque contenant 4 à 14 atomes de carbone dont la fonction amine est protégée par un groupement protecteur tel que le radical tert.butoxycarbonyle, suivie du blocage d'une partie des fonctions silanols par des radicaux Si(R')₃ tels que définis précédemment, puis, après élimination des groupements protecteurs de la fonction amine, de l'amidification au moyen d'un aminoacide de formule générale : 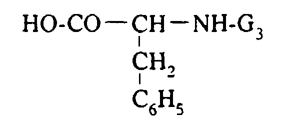 dans laquelle G₃ est défini comme précédemment, et enfin blocage des fonctions silanols résiduelles par des radicaux Si(R'')₃ tels que définis précédemment.

Généralement, l'action de l'anhydride d'un acide aminoalcanoïque protégé sur la silice aminopropyle est effectuée en opérant dans un solvant organique anhydre tel que le diméthylformamide à une température voisine de 20°C.

Le blocage des fonctions silanols par des groupements -Si(R'₃) tels que définis précédemment est effectuée par action d'un halogénotrialkylsilane sur la silice aminopropyle greffée par des restes aminoalcanoyles en opérant dans un solvant organique tel que le chlorure de méthylène en présence d'un agent basique tel que la pyridine.

L'élimination des groupements protecteurs des restes aminoalcanoyles s'effectue généralement, lorsque le groupement protecteur est un radical tert.butoxycarbonyle, par action de l'acide trifluoroacétique dans un solvant organique tel que le chlorure de méthylène.

L'amidification au moyen de phénylalanine dont la fonction amine est protégée est effectuée en présence d'un agent de condensation tel que la N-éthoxycarbonyl éthoxy-2 dihydro-1,2 quinoléine en opérant dans un solvant organique anhydre tel que le diméthylformamide.

Le blocage des fonctions silanols résiduelles par des radicaux -Si(R'')₃ tels que définis précédemment est généralement effectué au moyen de trialkylsilylimidazole en opérant dans un solvant organique tel que le chlorure de méthylène.

La silice aminopropyle peut être préparée par action de l'aminopropyl-triéthoxysilane sur une silice dont la porosité est voisine de 100 Å en opérant en présence d'imidazole dans un solvant organique anhydre tel qu'un hydrocarbure aromatique comme le toluène.

Les exemples suivants illustrent la présente invention.

### EXEMPLE 1

450 g (2,2 moles) de (E, E)-1,4-diphénylbutadiène, 210 g (3 moles) d'acide propiolique et 12,1 g (0,11 mole) d'hydroquinone sont chauffés au reflux, pendant 24 heures sous atmosphère d'azote, dans 2,5 litres de xylène. Les cristaux formés par refroidissement sont essorés, lavés au xylène puis à l'éther de pétrole, et séchés sous pression réduite. On obtient ainsi 454 g (76 %) d'acide cis 3,6-diphényl-1-4-cyclohexadiène carboxylique sous forme d'une poudre beige-clair dont les caractéristiques sont les suivantes :
- point de fusion = 179°C
- spectre de R.M.N. ¹H (250 MHz, (CD₃)₂SO d6, δ en ppm) : 4,40 (mt, 2H : CHAr) ; 5,78 et 6,92 (2 dmt, J = 9 Hz, 1H chacun : CH=CH) ; 7,02 (mt, 1H : CH=) ; de 7,15 à 7,50 (mt, 10H : H aromatiques des deux phényles) ; 12,35 (mf, 1H : COOH).

200 g (0,73 mole) d'énantiomère de l'acide cis 3,6-diphényl-1-4-cyclohexadiène carboxylique sont chauffés à reflux, pendant 24 heures, dans 1,5 litres de méthanol en présence de 7,15 g (0,073 mole) d'acide sulfurique à 96 %. Après refroidissement, le méthanol est concentré sous pression réduite. Le résidu est repris par 2 litres de dichlorométhane, lavé par 750 cm3 d'une solution aqueuse saturée d'hydrogénocarbonate de sodium puis à l'eau jusqu'à neutralité. La phase organique est séchée sur sulfate de magnésium puis agitée pendant 30 minutes avec 2 kg de gel de silice (70-230 mesh). Après filtration de la silice et concentration sous pression réduite, on obtient 185 g (88 %) d'ester méthylique de l'acide cis 3,6-diphényl-1-4-cyclohexadiène carboxylique sous forme d'une huile jaune-orangée dont les caractéristiques sont les suivantes :
- spectre de masse (D.C.I. : NH₃) M/Z = 291 (MH⁺).

116,2 g (0,4 mole) d'ester méthylique de l'acide cis 3,6-diphényl-1-4-cyclohexadiène carboxylique et 9,12 g (0,08 mole) d'acide trifluoroacétique sont chauffés à reflux dans 600 cm3 de dichlorométhane. On ajoute alors, goutte à goutte au reflux en 2 heures environ, 335,4 g (1,2 mole) de N-triméthylsilylméthyl-N-n.butoxyméthyl-benzylamine, qui peut être obtenue selon Chem. Pharm. Bull., 276 (1985). L'agitation est maintenue pendant une nuit en laissant la température remonter au voisinage de 20°C. Le mélange réactionnel est agité avec une solution aqueuse 2N d'acide chlorhydrique jusqu'à ce que le pH de la phase aqueuse atteigne 1. La phase organique est séchée sur sulfate de sodium et concentrée sous pression réduite. L'huile résiduelle brune est reprise dans 400 cm3 de toluène. Après 48 heures de repos, les cristaux formés sont essorés. On obtient 123 g (67 %) de chlorhydrate de l'ester méthylique de l'acide 2-benzyl-4,7-diphényl-2,3,3a,4,7,7a-hexahydro-1H-isoindole-3a-carboxylique sous forme d'une poudre beige-clair dont les caractéristiques sont les suivantes :
- point de fusion = 208-212°C
- spectre de R.M.N. ¹H (250 MHz, CDCl₃, δ en ppm) : de 2,55 à 2,80 (mt, 3H : CH₂ en 1 et H en 7a) ; 2,87 et 3,20 (2 d, J = 10 Hz, 1H chacun : CH₂ en 3) ; 3,30 (mt, 1H : H en 7) ; 3,38 (s, 3H : COOCH₃) ; 3,60 (mt, 1H : H en 4) ; 3,68 (s, 2H : NCH₂Ar) ; 6,08 (AB limite, 2H : CH=CH) ; de 7,20 à 7,45 (mt, 15H : H aromatiques des deux phényles en 4 et en 7 et H aromatiques du benzyle en 2).

A une solution, refroidie à 0-5°C, de 92 g (0,22 moles) d'ester méthylique de l'acide 2-benzyl-4,7-diphényl-2,3,3a,4,7,7a-hexahydro-1H-isoindole-3a-carboxylique dans 1,4 litres de dichlorométhane sec, on ajoute, goutte à goutte en 2 heures environ, 200 cm3 d'acide trifluorométhanesulfonique en maintenant la température inférieure à 5°C. Après la fin de l'addition, on laisse la température du mélange réactionnel revenir au voisinage de 20°C puis on l'agite pendant 20 heures. Après refroidissement à 0°C, le mélange est hydrolysé par 130 cm3 d'eau puis 460 cm3 de soude 4N. Après décantation, la phase aqueuse est extraite au dichlorométhane, puis les phases organiques réunies sont lavées à l'eau jusqu'à neutralité. Le résidu orange, obtenu par concentration du dichlorométhane, cristallise par agitation dans 800 cm3 de pentane. Après filtration, les cristaux sont purifiés par chromatographie haute pression sur 2 kg de gel de silice (15-40 mM), en éluant avec un mélange cyclohexane-acétate d'éthyle (98-2 en volumes). On obtient ainsi 44 g (47 %) d'ester méthylique de l'acide 2-benzyl-4,9-éthano-9-phényl-2,3,3a,4,9,9a-hexahydro-1H-benzo[f]isoindole-3a-carboxylique-(3aRS, 4SR, 9SR, 9aRS) sous forme d'une poudre beige-clair dont les caractéristiques sont les suivantes :
- point de fusion = 125°C
- spectre de R.M.N. ¹H (300 MHz, CDCl₃, δ en ppm) : 1,45 - 1,73 et 2,50 (3 mts, respectivement 1H - 1H et 2H : CH₂CH₂) ; 2,38 et 3,25 (2 d, J = 10 Hz, 1H chacun : CH₂ en 3) ; 2,36 et 2,75 (respectivement dd et d, J = 11 et 9 Hz et J = 11 Hz, 1H chacun : CH₂ en 1) ; 3,30 (d large, J = 9 Hz, 1H : H en 9a) ; 3,35 et 3,70 (2 d, J = 12,5 Hz, 1H chacun : NCH₂Ar) ; 3,42 (mt, 1H : H en 4) ; 3,58 (s, 3H : COOCH₃) ; 6,58 (d large, J = 7,5 Hz, 1H : H en 8) ; de 6,95 à 7,15 (mt, 3H : H en 5 - H en 6 et H en 7) ; de 7,20 à 7,50 (mt, 10H : H aromatiques du phényle en 9 et H aromatiques du benzyle en 2).

15 g (35 mmoles) d'ester méthylique de l'acide 2-benzyl-4,9-éthano-9-phényl-2,3,3a,4,9,9a-hexahydro-1H-benzo[f]isoindole-3a-carboxylique-(3aRS, 4SR, 9SR, 9aRS), en solution dans 360 cm3 de méthanol, sont réduits par 6,62 g de formiate d'ammonium en présence de 5,1 g de palladium sur charbon à 5 % (p/p) en chauffant au reflux pendant 3 heures. Après refroidissement, le catalyseur est séparé par filtration et le méthanol concentré sous pression réduite. Par agitation dans 200 cm3 de pentane, l'huile obtenue cristallise. Par filtration, on obtient 11,3 g (97 %) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), sous forme de cristaux blancs dont les caractéristiques sont les suivantes :
- point de fusion = 129-30°C
- spectre de R.M.N. ¹H (200 MHz, (CD₃)₂SO d6, δ en ppm) : 1,36 - 1,64 et de 2,15 à 2,45 (3 mts, respectivement 1H - 1H et 2H : CH₂CH₂) ; 2,65 et 2,77 (2 dd, respectivement J = 11,5 et 3 Hz et J = 11,5 et 7,5 Hz, 1H chacun : CH₂ en 1) ; 2,92 et 3,35 (2 d, J = 11,5 Hz, 1H chacun : CH₂ en 3) ; de 3,10 à 3,25 (mt, 1H : H en 9a) ; 3,45 (mt, 1H : H en 4) ; 3,52 (s, 3H : COOCH₃) ; 6,41 (d large, J = 7,5 Hz, 1H : H en 8) ; 7,04 (dt, J = 7,5 et 2 Hz, 1H : H en 7) ; de 7,05 à 7,20 (mt, 2H : H en 5 et H en 6) ; de 7,30 à 7,60 (mt, 6H : H aromatiques du phényle en 9 et NH).

2 g (6 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS) et 1 g (6 mmoles) d'acide 2-méthoxyphénylacétique, en solution dans 50 cm3 de dichlorométhane, sont condensés pur agitation en présence de 1,27 g (6,6 mmoles) de dichlorhydrate de 1-éthyl-3-[3-(diméthylamino)propyl]-carbodiimide et 0,16 g (1,2 mmoles) d'hydroxybenzotriazole pendant 18 heures sous atmosphère d'azote. Après lavage par décantation avec 2 fois 40 cm3 d'eau, la phase organique est séchée sur sulfate de sodium puis le dichlorométhane est évaporé sous pression réduite. Le résidu est purifié par flash-chromatographie sur gel de silice (70-230 mesh) en éluant avec un mélange cyclohexane-acétate d'éthyle (70-30 en volumes). On obtient ainsi 2,18 g (75 %) d'ester méthylique de l'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) sous forme d'une poudre beige-clair dont les caractéristiques sont les suivantes :
- point de fusion = 152-3°C
- spectre de R.M.N. ¹H (400 MHz, (CD₃)₂SO d6, à une température de 393 K, δ en ppm) : 1,41 - 1,66 - 1,94 et 2,09 (4 mts, 1H chacun : CH₂CH₂) ; de 3,30 à 3,65 (mt, 6H : CH₂ en 1 - COCH₂Ar - 1H du CH₂ en 3 et H en 9a) ; 3,48 (mt, 1H : H en 4) ; 3,55 (s, 3H : COOCH₃) ; 3,70 (mf, 3H : OCH₃) ; 4,18 (d, J = 12,5 Hz, 1H : l'autre H du CH₂ en 3) ; 6,43 (d large, J = 7,5 Hz, 1H : H en 8) ; 6,90 (t large, J = 7,5 Hz, 1H : H aromatique en para du OCH₃) ; 6,96 (d large, J = 7,5 Hz, 1H : H aromatique en ortho du OCH₃) ; 7,06 (dt, J = 7,5 et 2 Hz, 1H : H en 7) ; de 7,10 à 7,25 (mt, 4H : H en 5 - H en 6 et H aromatiques en méta du OCH₃) ; 7,39 (mt, 3H : H aromatiques en ortho et para du phényle en 9) ; 7,50 (t, J = 7,5 Hz, 2H : H aromatiques en méta du phényle en 9).

### EXEMPLE 2

2,1 g (4,35 mmoles) d'ester méthylique de l'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) sont chauffés au reflux pendant 18 heures dans 27 cm3 de méthanol et 45 cm3 de soude 1N. Après refroidissement, les cristaux formés sont essorés, puis repris dans 200 cm3 d'acide chlorhydrique 1N et extraits par 2 fois 100 cm3 d'acétate d'éthyle. Après concentration du solvant sous pression réduite, le solide obtenu est purifie par recristallisation dans 50 cm3 d'éther isopropylique. On obtient ainsi 1,36 g (67 %) d'acide 4,9-éthano-2-(2-méthoxyphényl)acétyl-9-phényl-2,3,3a,4,9,9a-hexahydro-1H-benzo[f]isoindole-3a- carboxylique-(3aRS, 4SR, 9SR, 9aRS) sous forme de cristaux blancs dont les caractéristiques sont les suivantes :
- point de fusion = 242-4°C
- spectre de R.M.N. ¹H (400 MHz, (CD₃)₂SO d6, à une température de 396 K, δ en ppm) : 1,41 - 1,66 - 1,94 et 2,09 (4 mts, 1H chacun : CH₂CH₂) ; de 3,30 à 3,65 (mt, 6H : CH₂ en 1 - COCH₂Ar - 1H du CH₂ en 3 et H en 9a) ; 3,48 (mt, 1H : H en 4) ; 4,18 (d, J = 12 Hz, 1H : l'autre H du CH₂ en 3) ; 3,70 (mf, 3H : OCH₃) ; 6,42 (d large, J = 7,5 Hz, 1H : H en 8) ; 6,90 (t large, J = 7,5 Hz, 1H : H aromatique en para du OCH₃) ; 6,96 (d large, J = 7,5 Hz, 1H : H aromatique en ortho du OCH₃) ; 7,06 (dt, J = 7,5 et 2 Hz, 1H : H en 7) ; de 7,10 à 7,25 (mt, 4H : H en 5 - H en 6 et H aromatiques en méta du OCH₃) ; 7,39 (mt, 3H : H aromatiques en ortho et paru du phényle en 9) ; 7,50 (t, J = 7,5 Hz, 2H : H aromatiques en méta du phényle en 9).

### EXEMPLE 3

467 mg (1 mmole) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) et 200 mg (1,2 mmoles) de N,N'-carbonyldiimidazole sont dissous dans 20 cm3 de dichlorométhane sec. On ajoute ensuite 10 cm3 d'une solution 0,5N d'ammoniac dans l'éthanol puis on agite pendant 16 heures à une temperature voisine de 20°C. Après dilution avec 50 cm3 de dichlorométhane, le mélange réactionnel est lavé successivement, par décantation, avec 50 cm3 d'une solution 4N d'acide chlorhydrique puis pur 2 fois 25 cm3 d'une solution saturée de chlorure de sodium, séché sur sulfate de sodium et concentré sous pression réduite. Le résidu obtenu est recristallisé dans 13 cm3 d'acétate d'éthyle. On obtient ainsi 350 mg (75 %) de 4,9-éthano-2-(2-méthoxyphényl)acétyl-9-phényl-2,3,3a,4,9,9a-hexahydro-1H-benzo[f]isoindole-3a-carboxamide-(3aRS, 4SR, 9SR, 9aRS) sous forme de cristaux blancs dont les caractéristiques sont les suivantes :
- point de fusion = 262-4°C
- spectre de R.M.N. ¹H (250 MHz, (CD₃)₂SO d6, à une température de 383 K, δ en ppm) : 1,36 - 1,60 - 1,88 et 2,05 (4 mts, 1H chacun : CH₂CH₂) ; de 3,20 à 3,65 (mt, 7H : CH₂ en 1 - COCH₂Ar - 1H du CH₂ en 3 - H en 4 et H en 9a) ; 3,67 (mf, 3H : OCH₃) ; 4,22 (d, J = 12,5 Hz, 1H : l'autre H du CH₂ en 3) ; 6,37 (d large, J = 7,5 Hz, 1H : H en 8) ; 6,75 (mf, 2H : CONH₂) ; 6,88 (t large, J = 7,5 Hz, 1H : H aromatique en para du OCH₃) ; 6,94 (d large, J = 7,5 Hz, 1H : H aromatique en ortho du OCH₃) ; 7,02 (dt, J =7,5 et 2 Hz, 1H : H en 7) ; de 7,05 à 7,30 (mt, 4H : H en 5 - H en 6 et H aromatiques en méta du OCH₃) ; 7,39 (mt, 3H : H aromatiques en ortho et para du phényle en 9) ; 7,48 (t, J = 7,5 Hz, 2H : H aromatiques en méta du phényle en 9).

### EXEMPLE 4

A partir de 500 mg (1,07 mmoles) d'acide 4,9-éthano-2-(2-méthoxyphényl) acétyl-9-phényl-2,3,3a,4,9,9a-hexahydro-1H-benzo[f]isoindol-3a-carboxylique-(3aRS, 4SR, 9SR, 9aRS), de 113 mg (1,28 mmoles) de N,N-diméthyléthylénediamine, de 246 mg (1,28 mmoles) de chlorhydrate de 1-éthyl-3-(3-dimé-thylaminopropyl)-carbodiimide et de 14 mg (0,1 mmole) d'hydroxybenzotriaxole en solution dans 30 cm3 de dichlorométhane, on obtient, après 18 heures d'agitation à une température voisine de 20°C et recristallisation du produit brut obtenu dans 10 cm3 d'un mélange cyclohexane-acétate d'éthyle (40-60 en volumes), 530 mg (92 %) N-(2-diméthylaminoéthyl)-4,9-éthano-2-(2-méthoxyphényl)acétyl-9-phényl-2,3,3a,4,9,9a-hexahydro-1H-benzo[f]isoindole-3a-carboxamide-(3aRS, 4SR, 9SR, 9aRS) sous forme de cristaux beiges clairs dont les caractéristiques sont les suivantes :
- point de fusion = 99°C
- spectre de masse : (D.I.C. NH₃) M/Z = 538 (MH⁺).

### EXEMPLE 5

A partir de 500 mg (1,07 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 95 mg (1,28 mmoles) de N-méthyléthylènediamine, de 246 mg (1,28 mmoles) de chlorhydrate de 1-éthyl-3-(3-diméthylamino-propyl)-carbodiimide et de 14 mg (0,1 mmole) d'hydroxybenzotriazole en solution dis 30 cm3 de dichlorométhane, on obtient, après 18 heures d'agitation à une température voisine de 20°C et recristallisation du produit brut obtenu dans 10 cm3 d'un mélange cyclohexane-acétate d'éthyle (50-50 en volumes), 525 mg (95 %) N-(2-méthylaminoéthyl)-4,9-éthano-2-(2-méthoxyphényl)acétyl-9-phényl-2,3,3a,4,9,9a-hexahydro-1H-benzo[f]isoindole-3a-carboxamide-(3aRS, 4SR, 9SR, 9aRS) sous forme de cristaux jaunes clairs dont les caractéristiques sont les suivantes :
- point de fusion = 107°C
- spectre de masse : (D.I.C. : NH₃) M/Z = 524 (MH⁺).

### EXEMPLE 6

A partir de 500 mg (1,07 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 735 mg (1,5 mmoles) de p.toluènesulfonate du diester de benzyle de l'acide aspartique, de 290 mg (1,5 mmoles) de chlorhydrate de 1-éthyl-3-(3-diméthylamino-propyl)-carbodiimide, de 14 mg (0,1 mmole) d'hydroxybenzotriazole et de 152 mg (1,5 mmoles) de triéthylamine en solution dans 30 cm3 de dichlorométhane, on obtient, après 18 heures d'agitation à une temperature voisine de 20°C puis chromatographie du produit brut obtenu sur gel de silice (70-230 mesh), en éluant avec un mélange cyclohexane-acétate d'éthyle (70-30 en volumes) , 490 mg (67 %) de diester de benzyle de l'acide N-[4,9-éthano-2-(2-méthoxyphényl)acétyl-9-phényl-2,3,3a,4,9,9a-hexahydro-1H-benzo[f] isoindolyl-3a-carbonyl]-aspartique-(3aRS, 4SR, 9SR, 9aRS) sous forme d'une poudre blanche fondant a 118°C.

470 mg (0,63 mmole) de diester de benzyle de l'acide N-[4,9-éthano-2-(2-méthoxyphényl)acétyl-9-phényl-2,3,3a,4,9,9a-hexahydro-1H-benzo[f]isoindolyl-3a-carbonyl]-aspartique-(3aRS, 4SR, 9SR:, 9aRS) en solution dans 15 cm3 d'éthanol sont réduits par action de l'hydrogène à pression atmosphérique et en présence de 50 mg d'hydroxyde de palladium sur charbon à 20 % (p/p), pendant 5 heures. Après filtration du catalyseur et concentration de l'éthanol sous pression réduite, le résidu est recristallisé dis le toluène. On obtient ainsi 310 mg (83 %) d'acide N-[4,9-éthano-2-(2-méthoxyphényl)acétyl-9-phényl-2,3,3a,4,9,9a-hexahydro-1H-benzo[f] isoindolyl-3a-carbonyl]-aspartique-(3aRS, 4SR, 9SR, 9aRS), sous forme de cristaux blancs dont les caractéristiques sont les suivantes :
- point de fusion = 194°C
- spectre de masse (E.I.) M/Z = 582 (M⁺).

### EXEMPLE 7

A partir de 500 mg (1,07 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 750 mg (1,5 mmoles) de p.toluènesulfonate de l'ester dibenzylique de l'acide glutamique, de 290 mg (1,5 mmoles) de chlorhydrate de 1-éthyl-3-(3-diméthylamino-propyl)-carbodiimide, de 14 mg (0,1 mmole) d'hydroxybenzotriazole et de 152 mg (1,5 mmoles) de triéthylamine en solution dans 30 cm3 de dichlorométhane, on obtient, après 18 heures d'agitation à une température voisine de 20°C puis chromatographie du produit brut obtenu sur gel de silice (70-230 mesh) en éluant avec un mélange cyclohexane-acétate d'éthyle (80-20 en volumes), 750 mg (96 %) de l'ester dibenzylique de l'acide N-[4,9-éthano-2-(2-méthoxyphényl)acétyl-9-phényl-2,3,3a, 4,9,9a-hexahydro-1H-benzo[f]isoindolyl-3a-carbonyl]-glutamique-(3aRS, 4SR, 9SR, 9aRS) sous forme d'une huile qui se solidifie vers 30°C.

A partir de 730 mg (0,96 mmole) de l'ester dibenzylique de l'acide N-[4,9-éthano-2-(2-méthoxyphényl)acétyl-9-phényl-2,3,3a,4,9,9a-hexahydro-1H-benzo[f] isoindolyl-3a-carbonyl]-glutamique-(3aRS, 4SR, 9SR, 9aRS), en présence de 100 mg d'hydroxyde de palladium sur charbon à 20 % (p/p), on obtient, après recristallisation dans un mélange méthanol-pentane (50-50 en volumes), 200 mg (35 %) d'acide N-[4,9-éthano-2-(2-méthoxyphényl)acétyl-9-phényl-2,3,3a,4,9,9a-hexahydro-1H-benzo[f]isoindolyl-3a-carbonyl]-glutamique-(3aRS, 4SR, 9SR, 9aRS), sous forme de cristaux blancs dont les caractéristiques sont les suivantes :
- point de fusion = 168°C
- spectre de masse (E.I.) M/Z = 596 (M⁺).

### EXEMPLE 8

A partir de 570 mg (1,2 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 370 mg (1,54 mmoles) de dichlorhydrate de l'ester méthylique de l'histidine, de 300 mg (1,54 mmoles) de chlorhydrate de 1-éthyl-3-(3-diméthylamino-propyl)-carbodiimide, de 18 mg (0,12 mmole) d'hydroxybenzotriazole et de 280 mg (2,8 mmoles) de triéthylamine en solution dans 70 cm3 de dichlorométhane, on obtient, après 18 heures d'agitation à une température voisine de 20°C et chromatographie du produit brut obtenu sur gel de silice (70-230 mesh), en éluant avec un mélange dichlorométhane-méthanol-acide acétique (96-2-2 en volumes), 660 mg (89 %) d'ester méthylique de la N-[4,9-éthano-2-(2-méthoxyphényl)acétyl-9-phényl-2,3,3a,4,9,9a-hexahydro-1H-benzo[f] isoindolyl-3a-carbonyl]-histidine-(3aRS, 4SR, 9SR, 9aRS), sous forme d'une poudre blanche dont les caractéristiques sont les suivantes :
- point de fusion = 200-204°C
- spectre de masse (D.I.C. : NH₃) M/Z = 619 (MH⁺).

A partir de 640 mg (1,06 mmoles) d'ester de méthyle de la N-[4,9-éthano-2-(2-méthoxyphényl)acétyl-9-phényl-2,3,3a,4,9,9a-hexahydro-1H-benzo[f]isoindolyl-3a-carbonyl]-histidine-(3aRS, 4SR, 9SR, 9aRS) dans 1,3 cm3 de soude 1N et 30 cm3 d'éthanol, on obtient, après chauffage au reflux pendant 18 heures et deux recristallisations du produit brut obtenu dans une solution aqueuse 0,25 N d'acide chlorhydrique, 200 mg (29 %) de chlorhydrate de N-[4,9-éthano-2-(2-méthoxyphényl)acéty]-9-phényl-2,3,3a,4,9,9a-hexahydro-1H-benzo[f]isoindolyl-3a-carbonyl]-histidine-(3aRS, 4SR, 9SR, 9aRS), sous forme de cristaux blancs dont les caractéristiques sont les suivantes :
- point de fusion = 245°C
- spectre de masse (E.I.) M/Z = 604 (M⁺).

### EXEMPLE 9

A partir de 200 mg (0,42 mmole) de l'énantiomère dextrogyre de l'acide 4,9-éthano-2-[(2-méthoxyphényl)-2-méthylènyl-acétyl]-9-phényl-2,3,3a,4,9,9a-hexahydro-1H-benzo[f]-isoindole-3a-carboxylique-(3aRS, 4SR, 9SR, 9aRS) (exemple 40), de 60 mg (0,5 mmoles) de 1(R)-phényl-éthylamine, de 96 mg (0,5 mmole) de chlorhydrate de 1-éthyl-3-(3-diméthylamino-propyl)-carbodiimide et de 6 mg (0,045 mmole) d'hydroxybenzotriazole en solution dans 10 cm3 de dichlorométhane, on obtient, après 18 heures d'agitation à une température voisine de 20°C et chromatogaphie du produit brut obtenu sur une plaque de silice préparative, en éluant avec un mélange cyclohexane-acétate d'éthyle (50-50 en volumes), 170 mg (71 %) de l'énantiomère dextrogyre de N-[1(R)-phényl-éthyl)]-[4,9-éthano-2-(2-méthoxyphényl)-2-méthylènyl-acétyl]-9-phényl-2,3,3a,4,9,9a-hexahydro-1H-benzo[f]isoindole-3a-carboxamide-(3aRS, 4SR, 9SR, 9aRS) sous forme d'une poudre blanche dont les caractéristiques sont les suivantes :
- point de fusion = 235°C
- spectre de masse (E.I.) M/Z = 582 (M⁺).

### EXEMPLE 10

A une solution de 300 mg (0,64 mmole) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) dans 5 cm3 de diméthylformamide, on ajoute 470 mg de carbonate de césium. Apres 30 minutes d'agitation à une température voisine de 20°C, on ajoute une solution de 820 mg (5 mmoles) d'iodure d'éthyle dans 2,5 cm3 de diméthylformamide puis on agite à une température voisine de 20°C pendant 18 heures. Après dilution avec 100 cm3 d'eau, on extrait 2 fois avec 50 cm3 d'acétate d'éthyle. Après concentration du solvant sous pression réduite, le résidu obtenu est purifié par flash-chromatographie sur gel de silice (70-230 mesh) en éluant avec un mélange cyclohexane-acétate d'éthyle (70-30 en volumes). On obtient ainsi 170 mg (54 %) d'ester éthylique de l'acide 4,9-éthano-2-(2-méthoxyphényl) acétyl-9-phényl-2,3,3a,4,9,9a-hexahydro-1H-benzo[f]isoindol-3a-carboxylique-(3aRS,4SR,9SR,9aRS) sous forme d'une poudre blanche dont les caractéristiques sont les suivantes :
- point de fusion = 196-7°C
- spectre de masse (E.I.) M/Z = 495 (M⁺).

### EXEMPLE 11

A une solution de 3,43 g (7,4 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS,4SR,9SR,9aRS) dans 20 cm3 d'acétone et 6 cm3 d'eau, refroidie à 0-2°C et maintenue sous atmosphère d'azote, on ajoute successivement 0,9 g (8,9 mmoles) de triéthylamine et 1,085 g (10 mmoles) de chloroformiate d'éthyle en solution dans 40 cm3 d'acétone. Après 30 minutes d'agitation à 0-5°C, on ajoute goutte à goutte une solution de 0,77 g (7,4 mmoles) d' azoture de sodium dans 15 cm3 d'eau, puis on maintient l'agitation à 0-5°C pendant 1 heure. Le mélange réactionnel est alors versé dans 200 cm3 d'eau, et l'acylazide est extrait par 5 fois 40 cm3 d'éther éthylique. Les phases organiques réunies sont lavées à l'eau puis séchées sur sulfate de magnésium. Après évaporation de l'éther sous pression réduite, on ajoute 50 cm3 de toluène. On chauffe alors progressivement à 85-90°C, puis au reflux pendant 1 heure après la fin du dégagement gazeux. Les cristaux formés par refroidissement sont essorés, puis lavés à l'éther de pétrole. On obtient ainsi 2,98 g (88 %) de 4,9-éthano-3a-isocyanate-2-(2-méthoxyphényl)acétyl-9 phényl-2,3,3a,4,9,9a-hexahydro-1H-benzo[f]isoindole-(3aRS,4SR,9SR,9aRS) sous forme d'une poudre blanche dont les caractéristiques sont les suivantes :
- point de fusion = 185°C
- spectre de masse (E.I.) M/Z = 464 (M⁺).

2,95 g (6,5 mmoles) de 4,9-éthano-3a-isocyanate-2-(2-méthoxyphényl) acétyl-9-phényl-2,3,3a,4,9,9a-hexahydro-1H-benzo-[f]isoindole-(3aRS, 4SR, 9SR, 9aRS) sont chauffés à 60°C pendant 12 heures avec 760 mg (7 mmoles) d'alcool benzylique et 123 mg (0,65 mmole) d'acide p.toluènesulfonique dans 100 cm3 de toluène. On obtient ainsi 3,63 g (100 %) de 3a-benzyloxycarbonylamino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), sous forme d'une poudre blanche, dont les caractéristiques sont les suivantes :
- point de fusion = 114°C
- spectre de masse (D.C.I. : NH₃) = 573 (MH⁺).

3,6 g (6,48 mmoles) de 3a-benzyloxycarbonylamino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS) en solution dans 30 cm3 d'un mélange dichlorométhane-méthanol (1-2 en volumes), sont réduits par action de l'hydrogène à pression atmosphérique en présence de 0,3 g de palladium sur charbon à 5 % (p/p) pendant 5 heures. Après filtration du catalyseur et concentration du solvant sous pression réduite, le résidu est purifé par flash-chromatographie sur gel de silice (70-230 mesh) en éluant avec un mélange dichlorométhane-éthanol (9-1 en volumes). On obtient ainsi 2,42 g (88 %) de 3a-amino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), sous forme d'une poudre blanche dont les caractéristiques sont les suivantes :
- point de fusion = 92°C
- spectre de R.M.N. ¹H (300 MHz, (CD₃)₂SO d6, δ en ppm) : à température ambiante, nous observons le mélange de deux rotamères dans les proportions 50/50. De 1,30 à 2,05 (mts, 6H : CH₂CH₂ et NH₂) ; 2,35 (mt, 1H : H en 9a) ; de 2,85 à 3,75 (mt, 6H : CH₂ en 1 - COCH₂Ar - 1H du CH₂ en 3 et H en 4) ; 3,55 et 3,80 (2s, 3H en totalité : OCH₃) ; 4,10 et 4,17 (d, J = 12,5 Hz, 1H en totalité : l'autre H du CH₂ en 3) ; 6,38 et 6,43 (2d large, J= 7,5 Hz, 1H en totalité : H en 8) ; de 6,85 à 7,55 (mt, 12 H : H en 5 - H en 6 - H en 7 - H aromatiques en ortho - méta et para du OCH₃ et H aromatiques du phényle en 9).

On ajoute, goutte à goutte et à température inférieure à 10°C, 1 cm3 d'anhydride acétique dans 3 cm3 d'acide formique concentré, puis on chauffe pendant 1 heure à 50°C. Après refroidissement, on ajoute 438 g (1 mmole) de 3a-amino-4,9-éthano-2-(2-méthoxyphényl)acétyl-9-phényl-2,3,3a,4,9,9a-hexahydro- 1H-benzo-[f]isoindole-(3aRS, 4SR, 9SR, 9aRS) et on maintient l'agitation à une température voisine de 20°C pendant 12 heures. Le mélange réactionnel est ensuite repris par 50 cm3 d'eau et 50 cm3 de dichlorométhane. La phase oganique est séparée par décantation, lavée à l'eau puis séchée sur sulfate de sodium. Les cristaux formés par addition de 50 cm3 d'éther isopropylique sont essorés. On obtient ainsi 215 mg (46 %) de 3a-formylamino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), sous forme de cristaux blancs, dont les caractéristiques sont les suivantes :
- spectre de masse (D.C.I. : NH₃) M/Z = 429 (MH⁺)
- point de fusion = 236°C

### EXEMPLE 12

A partir de 400 mg (0,91 mmole) de 3a-amino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), de 200 mg (0,91 mmole) de N-benzyloxycarbonyl-glycine, de 200 mg (1,05 mmoles) de chlorhydrate de 1-éthyl-3-(3-diméthylamino-propyl)-carbodiimide et de 14 mg (0,1 mmole) d'hydroxybenzotriazole en solution dans 20 cm3 de dichlorométhane, on obtient, après 18 heures d'agitation à une température voisine de 20°C et purification du produit brut obtenu par chromatographie sur silice (70-230 mesh) en éluant avec de l'acétate d'éthyle, 350 mg (62 %) de 3a-(N-benzyloxycarbonyl-glycyl)amino-[4,9-éthano-2-(2-méthoxyphényl)acetyl-9-phényl-2,3,3a,4,9,9a-hexahydro-1H-benzo[f]isoindole-(3aRS, 4SR, 9SR, 9aRS), sous forme d'une poudre blanche dont les caractéristiques sont les suivantes :
- point de fusion = 118°C
- spectre de masse (D.I.C. : NH₃) M/Z = 630 (MH⁺).

100 mg (0,16 mmole) de 3a-(N-benzyloxycarbonyl-glycyl)amino-[4,9-éthano-2-(2-méthoxyphényl)acétyl-9-phényl-2,3,3a,4,9,9a-hexahydro-1H-benzo[f] isoindole-(3aRS, 4SR, 9SR, 9aRS), en solution dans 5 cm3 de méthanol, sont traités par de l'hydrogène à pression atmosphérique en présence de 10 mg de palladium sur charbon à 10 % (p/p) pendant 4 heures. Après séparation du catalyseur par filtration et évaporation du solvant, le résidu est purifié par chromatographie sur une plaque de silice préparative en éluant avec un mélange acétate d'éthyle-méthanol (98-2 en volumes). On obtient ainsi 42 mg (51 %) de 3a-glycylamin-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), sous forme d'une poudre blanche, dont les caractéristiques sont les suivantes :
- point de fusion = 210°C
- spectre de masse (D.I.C. : NH₃) M/Z = 496 (MH⁺).

### EXEMPLE 13

A partir de 600 mg (1,37 mmoles) de 3a-amino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), de 400 mg (1,60 mmoles) de N-t.butoxycarbonylméthionine, de 303 mg (1,60 mmoles) de chlorhydrate de 1-éthyl-3-(3-diméthylamino-propyl)-carbodiimide et de 20 mg (0,14 mmole) d'hydroxybenzotriazole en solution dans 30 cm3 de dichlorométhane, on obtient, après 18 heures d'agitation à une température voisine de 20°C et purification du produit brut obtenu par chromatographie sur gel de silice (70-230 mesh) en éluant avec un mélange cyclohexane-acétate d'éthyle (30-70 en volumes), 670 mg (73 %) de 3a-(N-t.butoxycarbonyl-méthinonyl)amino-[4,9-éthano-2-(2-méthoxyphényl) acétyl-9-phényl-2,3,3a,4,9,9a-hexahydro-1H-benzo[f]isoindole-(3aRS, 4SR, 9SR, 9aRS), sous forme d'une poudre blanche dont les caractéristiques sont les suivantes :
- point de fusion = 92°C
- spectre de masse (D.I.C. / NH₃) M/Z = 670 (MH⁺).

400 mg (0,6 mmole) de 3a-(N-t.butoxycarbonyl-méthionyl)amino-[4,9-éthano-2-(2-méthoxyphényl)acétyl-9-phényl-2,3,3a,4,9,9a-hexahydro-1H-benzo[f] isoindole-(3aRS, 4SR, 9SR, 9aRS) sont chauffés à 50°C pendant 3 heures dans 40 cm3 de méthanol et 4 cm3 d'acide chlorhydrique 4N. Après dilution avec 100 cm3 d'eau, neutralisation à pH = 8 par une solution de soude 4N, extraction par 2 fois 100 cm3 d'acétate d'éthyle, séchage sur sulfate de magnésium et concentration à sec, on obtient une huile qui est purifiée par flash-chromatographie sur gel de silice (70-230 mesh), en éluant avec un mélange dichlorométhane-éthanol (92-8 en volumes). On obtient ainsi 80 mg (22 %) de 3a-méthionylamino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS) sous forme d'une poudre blanche dont les caractéristiques sont les suivantes :
- point de fusion supérieur à 260°C
- spectre de masse (D.I..C. : NH₃) M/Z = 569 (MH⁺).

### EXEMPLE 14

A partir de 500 mg (1,14 mmoles) de 3a-amino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), de 280 mg (1,37 mmoles) de N-t.butoxycarbonylsérine, de 262 mg (1,37 mmoles) de chlorhydrate de 1-éthyl-3-(3-diméthylamino-propyl)-carbodiimide et de 15,5 mg (0,114 mmole) d'hydroxybenzotriazole en solution dans 20 cm3 de dichlorométhane, on obtient, après 18 heures d'agitation à une température voisine de 20°C et purification du produit brut obtenu par chromatographie sur gel de silice (70-230 mesh) en éluant avec de l'acétate d'éthyle, 200 mg (28 %) de 3a-(N-t.butoxycarbonyl-séryl)amino-[4,9-éthano-2-(2-méthoxyphényl)acétyl-9-phényl-2,3,3a,4,9,9a-hexahydro-1H-benzo[f]isoindole-(3aRS, 4SR, 9SR, 9aRS) sous forme d'une poudre blanche dont les caractéristiques sont les suivantes :
- point de fusion = 110°C
- spectre de masse (D.I.C. : NH₃) M/Z = 626 (MH⁺).

### EXEMPLE 15

A partir de 600 mg (1,37 mmoles) de 3a-amino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS), de 220 mg (1,60 mmoles) de monoester éthylique de l'acide malonique, de 300 mg (1,60 mmoles) de chlorhydrate de 1-éthyl-3-(3-diméthylamino-propyl)-carbodiimide et de 20 mg (0,14 mmole) d'hydroxybenzotriazole en solution dans 20 cm3 de dichlorométhane, on obtient, après 18 heures d'agitation à une température voisine de 20°C et purification du produit brut obtenu par recristallisation dans le toluène, 680 mg (91 %) de 4,9-éthano-3a-éthoxycarbonylacétylamino-2-(2-méthoxyphényl)acétyl-9-phényl-2,3,3a,4,9,9a-hexahydro-1H-benzo[f]isoindole-(3aRS, 4SR, 9SR, 9aRS) sous forme d'une poudre blanche dont les caractéristiques sont les suivantes :
- point de fusion = 110°C
- spectre de masse (D.I.C. : NH₃) M/Z = 553 (MH⁺).

### EXEMPLE 16

A partir de 490 mg (0,89 mmoles) de 4,9-éthano-3a-éthoxycarbonylacétylamino-2-(2-méthoxyphényl)acétyl-9-phényl-2,3,3a,4,9,9a-hexahydro-1H-benzo[f]isoindole-(3aRS, 4SR, 9SR, 9aRS) dans 0,9 cm3 de soude 1N et 10 cm3 d'éthanol au reflux pendant 5 heures, on obtient, après recristallisation dans l'éthanol à 90 %, 320 mg (70 %) de 4,9-éthano-3a-carboxylacétylamino-2-(2-méthoxyphényl)acétyl-9-phényl-2,3,3a,4,9,9a-hexahydro-1H-benzo[f]isoindole-(3aRS, 4SR, 9SR, 9aRS) sous forme de cristaux blancs dont les caractéristiques sont les suivantes :
- point de fusion = 228°C
- spectre de masse (D.I.C. : NH₃) M/Z = 539 (MH⁺).

### EXEMPLE 17

A une solution de 310 mg (0,7 mmole) de 3a-amino-4,9-éthano-2-(2-méthoxyphényl)acétyl-9-phényl-2,3,3a,4,9,9a-hexahydro-1H-benzo[f]isoindole-(3aRS, 4SR, 9SR, 9aRS) dans 20 cm3 de dichlorométhane, on ajoute 90 mg (0,9 mmole) d'anhydride succinique, puis on agite pendant 3 heures à température voisine de 20°C. Après concentration des solvants sous pression réduite, le résidu obtenu est dissous dans 50 cm3 de soude 0,1N puis la phase aqueuse est lavée par 2 fois 20 cm3 d'acétate d'éthyle. Après acidification à pH=2 par addition d'acide chlorydrique 0,5 N, le précipité formé est essoré puis recristallisé dans l'éthanol à 50 %. On obtient ainsi 200 mg (53 %) de 4,9-éthano-3a-(3-carboxy-propionyl) amino-2-(2-méthoxyphényl)acétyl-9-phényl-2,3,3a,4,9,9a-hexahydro-1H-benzo[f] isoindole-(3aRS, 4SR, 9SR, 9aRS) sous forme de cristaux écrus dont les caractéristiques sont les suivantes :
- point de fusion = 140°C
- spectre de masse (D.I.C. : NH₃) M/Z = 481 [(M- COO + H)⁺]

### EXEMPLE 18

On ajoute, goutte à goutte de manière à maintenir la température inférieure à 40°C, une solution de 13,7 g (32 mmoles) d'ester méthylique de l'acide 2-benzyl-4,9-éthano-9-phényl-2,3,3a,4,9,9a-hexahydro-1H-benzo[f]isoindole-3a-carboxylique-(3aRS, 4SR, 9SR, 9aRS), obtenu dans les conditions de l'exemple 1, dans 130 cm3 de tétrahydrofurane à une suspension de 1,23 g (32 mmoles) d'hydrure mixte d'aluminium et de lithium dans 200 cm3 de tétrahydrofurane sec, puis on maintient l'agitation à 40°C pendant 1 heure. Après refroidissement, on ajoute lentement 12 cm3 de soude 1N puis du sulfate de magnésium. Après filtration des sels minéraux et concentration à sec, le résidu obtenu est purifié sur gel de silice (70-230 mesh), en éluant avec un mélange cyclohexane-acétate d'éthyle (30-70 en volumes), on obtient 12,15 g (96 %) de 2-benzyl-4,9-éthano-9-phényl-2,3,3a,4,9,9a-hexahydro-1H-benzo[f]isoindole-3a-méthanol-(3aRS, 4SR, 9SR, 9aRS) sous forme d'une meringue blanche dont les caractéristiques sont les suivantes :
- spectre de R.M.N. ¹H (300 MHz, CDCl₃, δ en ppm) : 1,43 - 1,80 et de 2,35 à 2,55 (3 mts, respectivement 1H - 1H et 2H : CH₂CH₂) ; de 2,25 à 2,45 et 2,62 (respectivement mt et dd, J = 9,5 et 8 Hz, 2H et 1H : CH₂ en 1 et H en 9a) ; 2,65 et 2,83 (2 d, J = 10 Hz, 1H chacun : CH₂ en 3) ; 2,87 (mt, 1H : H en 4) ; de 2,95 à 3,15 (mf étalé, 1H : OH) ; 3,03 et 3,32 (2 d, J = 10,5 Hz, 1H chacun : CH₂O) ; 3,50 et 3,60 (2 d, J = 13,5 Hz, 1H chacun : NCH₂Ar) ; 6,52 (d large, J = 7,5 Hz, 1H : H en 8) ; de 6,95 à 7,20 (mt, 3H : H en 5 - H en 6 et H en 7) ; de 7,20 à 7,60 (mt, 10H : H aromatiques du phényle en 9 et H aromatiques du benzyle en 2).

A partir de 3,1 g (7,8 mmoles) de 2-benzyl-4,9-éthano-9-phényl-2,3,3a,4,9,9a-hexahydro-1H-benzo[f]isoindole-3a-méthanol-(3aRS, 4SR, 9SR, 9aRS) en solution dans 50 cm3 de méthanol et en présence de 300 mg d'hydroxyde de palladium à 20 % (p/p), on obtient, après 2 heures sous hydrogène à pression atmosphérique, filtration du catalyseur et concentration à sec sous pression réduite, 2,26 g (95 %) de 4,9-éthano-9-phényl-2,3,3a,4,9,9a-hexahydro-1H-benzo[f] isoindole-3a-méthanol-(3aRS, 4SR, 9SR, 9aRS) sous forme d'une huile incolore très visqueuse dont les caractéristiques sont les suivantes :
- spectre de masse (E.I.) M/Z = 305 (M⁺).

A partir de 2,25 g (7,4 mmoles) de 4,9-éthano-9-phényl-2,3,3a,4,9,9a-hexahydro-1H-benzo[f]isoindole-3a-méthanol-(3aRS, 4SR, 9SR, 9aRS), de 1,23 g (7,4 mmoles) d'acide 2-méthoxyphénylacétique, de 1,42 g (7,4 mmoles) de chlorhydrate de 1-éthyl-3-(3-diméthylamino-propyl)-carbodiimide et de 100 mg (0,74 mmole) d'hydroxybenzotriazole en solution dans 60 cm3 de dichlorométhane, on obtient, après 18 heures d'agitation à une température voisine de 20°C et recristallisation du produit brut obtenu dans un mélange toluène-éther isopropylique (1-1 en volumes), 2,57 g (90 %) de 4,9-éthano-2-(2-méthoxyphényl)acétyl-9-phényl-2,3,3a,4,9,9a-hexahydro-1H-benzo[f]isoindole-3a-méthanol-(3aRS, 4SR, 9SR, 9aRS) sous forme de cristaux beige clair dont les caractéristiques sont les suivantes :
- point de fusion = 208°C
- spectre de R.M.N. ¹H (250 MHz, (CD₃)₂SO d6 avec ajout de qulques gouttes de CD₃COOD d4, à une température de 393 K, δ en ppm) : 1,35 - 1,68 - de 1,90 à 2,15 (3 mts, respectivement 1H - 1H et 2H : CH₂CH₂) ; 2,35 (mt, 1H : H en 9a) ; 2,95 (AB limite, J = 10 Hz, 2H : CH₂O) ; 3,10 (mt, 1H : H en 4) ; de 3,20 à 3,50 (mt, 2H : CH₂ en 1) ; 3,58 (s large, 2H : COCH₂Ar) ; de 3,55 à 3,75 (mt, 1H : 1H du CH₂ en 3) ; 3,70 (mf, 3H : OCH₃) ; 3,90 (d, J = 12,5 Hz, 1H : l'autre H du CH₂ en 3) ; 6,41 (d large, J = 7,5 Hz, 1H : H en 8) : de 6,85 à 7,00 (mt, 2H : H aromatiques en para et en ortho du OCH₃) ; 7,05 (dt, J = 7,5 et 2 Hz, 1H : H en 7) ; de 7,10 à 7,30 (mt, 4H : H en 5 - H en 6 et H aromatiques en méta du OCH₃) ; de 7,30 à 7,55 (mt, 5H : H aromatiques du phényle en 9).

On dissout, à une température voisine de 20°C sous atmosphère d'azote, 500 mg (1,1 mmoles) de 4,9-éthano-2-(2-méthoxyphényl)acétyl-9-phényl-2,3,3a, 4,9,9a-hexahydro-1H-benzo[f]isoindole-3a-méthanol-(3aRS, 4SR, 9SR, 9aRS) dans 10 cm3 de pyridine séchée sur potasse, puis on ajoute 336 mg (1,2 mmoles) d'anhydride trifluorométhanesulfonique. Après 3 heures d'agitation à une température voisine de 20°C, on ajoute 25 cm3 d'eau et on extrait par 3 fois 25 cm3 de dichlorométhane. Les phases organiques réunies sont lavées par de l'acide chlorhydrique 0,1 N puis à l'eau, séchées sur sulfate de magnésium et concentrées à sec sous pression réduite. Le résidu est purifié par recristallisation dans un mélange méthanol-éther isopropylique (60-40 en volumes). On obtient ainsi 310 mg (42 %) de trifluorométhanesulfonate de N-[4,9-éthano-2-(2-méthoxyphényl)acétyl-9-phényl-2,3,3a,4,9,9a-hexahydro-1H-benzo[f]isoindolyl-3a-méthyl]pyridinium-(3aRS, 4SR, 9SR, 9aRS) sous forme de cristaux jaune pâle dont les caractéristiques sont les suivantes :
- point de fusion = 244°C
- spectre de R.M.N. ¹H (250 MHz, (CD₃)₂SO d6, à une température de 393 K, δ en ppm) : 1,37 - 1,67 - 1,98 et 2,12 (4 mts, 1H chacun : CH₂CH₂) ; 2,80 (mt, 1H : H en 9a) ; 3,20 (mt, 1H : H en 4) ; 3,38 (AB limite, 2H : CH₂ en 1) ; 3,52 (s large, 2H : COCH₂Ar) ; 3,56 et 4,02 (2 d, J = 12,5 Hz, 1H chacun : CH₂ en 3) ; 3,68 (mf, 3H : OCH₃) ; 4,38 et 4,50 (2 d, J = 12,5 Hz, 1H chacun : CH₂N⁺) ; 6,51 (d large, J = 7,5 Hz, 1H : H en 8) ; 6,88 (t large, J = 7,5 Hz, 1H : H aromatique en para du OCH₃) ; 6,95 (d large, J = 7,5 Hz, 1H : H aromatique en ortho du OCH₃) ; 7,15 (dt, J = 7,5 et 2 Hz, 1H : H en 7) ; de 7,20 à 7,45 (mt, 4H : H en 5 - H en 6 et H aromatiques en méta du OCH₃) ; de 7,35 à 7,60 (mt, 5H : H aromatiques du phényle en 9) ; 8,15 (t large, J = 7 Hz, 2H : H en 3 et H en 5 du pyridyle) ; 8,65 (t large, J = 7 Hz, 1H : H en 4 du pyridyle) ; 8,91 (d large, J = 7 Hz, 2H : H en 2 et H en 6 du pyridyle).

### EXEMPLE 19

On dissout, à une température voisine de 20°C et sous atmosphère d'azote, 9 g (23 mmoles) de 2-benzyl-4,9-éthano-9-phényl-2,3,3a,4,9,9a-hexahydro-1H-benzo[f]isoindole-3a-méthanol-(3aRS, 4SR, 9SR, 9aRS) dans 100 cm3 de dioxane sec et 2,53 mg (25 mmoles) de triéthylamine, puis on ajoute 7,05 g (25 mmoles) d'anhydride trifluorométhanesulfonique. Après 3 heures d'agitation à une température voisine de 20°C, on dilue par 200 cm3 d'eau et on extrait avec 3 fois 250 cm3 d'acétate d'éthyle. Les phases organiques sont lavées à l'eau, séchées sur sulfate de magnésium, puis concentrées sous pression réduite. On obtient ainsi 12,5 g (95 %) 2-benzyl-4,9-éthano-9-phényl-3a-(trifluorométhylsulfonyloxy)méthyl-2,3,3a, 4,9,9a-hexahydro-1H-benzo[f]isoindole-(3aRS, 4SR, 9SR, 9aRS) sous forme d'une huile incolore dont les caractéristiques sont les suivantes :
- spectre de masse (E.I.) M/Z = 527 (M⁺).

870 mg (1,65 mmoles) de 2-benzyl-4,9-éthano-9-phenyl-3a-(trifluorométhylsulfonyloxy)méthyl-2,3,3a,4,9,9a-hexahydro-1H-benzo[f]isoindole-(3aRS, 4SR, 9SR, 9aRS) et 323 mg (6,6 mmoles) de cyanure de sodium sont agités pendant 18 heures à une température voisine de 20°C dans 20 cm3 de diméthylsulfoxyde. Après dilution avec 100 cm3 d'eau, on extrait 3 fois avec 50 cm3 d'éther éthylique. Les phases organiques réunies sont lavées à l'eau, séchées sur sulfate de magnésium et concentrées à sec sous pression réduite. On obtient ainsi 640 mg (96 %) 2-benzyl-3a-cyanométhyl-4,9-éthano-9-phényl-2,3,3a,4,9,9a-hexahydro-1H-benzo[f] isoindole-(3aRS, 4SR, 9SR, 9aRS) sous forme d'une laque blanche dont les caractéristiques sont les suivantes :
- spectre de masse (E.I.) M/Z = 495 (M⁺).

970 mg (2,4 mmoles) de 2-benzyl-3a-cyanométhyl-4,9-éthano-9-phényl-2,3,3a,4,9,9a-hexahydro-1H-benzo[f]isoindole(3aRS, 4SR, 9SR, 9aRS) sont chauffés au reflux pendant 5 heures dans 4 cm3 d'acide sulfurique aqueux à 50 %. Après refroidissement, le mélange réactionnel est versé dans 50 cm3 d'eau glacée. Le précipité formé est essoré, lavé avec 2 fois 10 cm3 d'eau glacée et séché sous pression réduite à 50°C, puis repris dans 20 cm3 de méthanol en présence de 1 cm3 d'acide sulfurique concentré et chauffé à reflux pendant une nuit. Le méthanol est évaporé, puis le résidu est dilué par 20 cm3 d'eau et extrait 2 fois par 25 cm3 d'acétate d'éthyle. Les phases organiques sont lavées à l'eau, séchées sur sulfate de magnésium, filtrées sur 10 g de silice et concentrées à sec sous pression réduite. On obtient ainsi 600 mg (59 %) d'ester méthylique de l'acide 2-benzyl-4,9-éthano-9-phényl-2,3,3a,4,9,9a-hexahydro-1H-benzo[f]isoindole-3a-acétique-(3aRS, 4SR, 9SR, 9aRS) sous forme d'une poudre blanche dont les caractéristiques sont les suivantes :
- point de fusion = 84-86°C
- spectre de masse (E.I.) M/Z = 437 (M⁺).

A partir de 800 mg (18 mmoles) d'ester méthylique de l'acide 2-benzyl-4,9-éthano-9-phényl-2,3,3a,4,9,9a-hexahydro-1H-benzo[f]isoindole-3a-acétique-(3aRS, 4SR, 9SR, 9aRS) en solution dans 20 cm3 de méthanol et en présence de 100 mg d'hydroxyde de palladium à 20 % (p/p), on obtient, après 2 heures d'agitation sous hydrogène à pression atmosphérique et à 40°C, filtration du catalyseur et concentration à sec sous pression réduite, 520 mg (83 %) d'ester méthylique de l'acide 4,9-éthano-9-phényl-2,3,3a,4,9,9a-hexahydro-1H-benzo[f]isoindole-3a-acétique-(3aRS, 4SR, 9SR, 9aRS) sous forme d'une poudre blanche dont les caractéristiques sont les suivantes :
- point de fusion = 71-73°C
- spectre de masse (E.I.) M/Z = 347 (M⁺).

A partir de 500 mg (1,44 mmoles) d'ester méthylique de l'acide 4,9-éthano-9-phényl-2,3,3a,4,9,9a-hexahydro-1H-benzo[f]isoindole-3a-acétique-(3aRS, 4SR, 9SR, 9aRS), de 260 mg (1,6 mmoles) d'acide 2-méthoxyphénylacétique, de 290 mg (1,6 mmoles) de chlorhydrate de 1-éthyl-3-(3-diméthylamino-propyl)-carbodiimide et de 20 mg (0,14 mmole) d'hydroxybenzotriazole en solution dans 12 cm3 de dichlorométhane, on obtient, après 18 heures d'agitation à une température voisine de 20°C et purification par flash-chromatographie sur gel de silice (70-230 mesh), en éluant par un mélange cyclohexane-acétate d'éthyle (1-1 en volumes), 430 mg (61 %) d'ester méthylique de l'acide 4,9-éthano-2-(2-méthoxyphényl)acétyl-9-phényl-2,3,3a,4,9,9a-hexahydro-1H-benzo[f]isoindole-3a-acétique-(3aRS, 4SR, 9SR, 9aRS) sous forme d'une poudre blanche dont les caractéristiques sont les suivantes :
- point de fusion = 95°C
- spectre masse (E.I.) M/Z = 495 (M⁺).

A partir de 394 mg (0,8 mmole) d'ester méthylique de l'acide 4,9-éthan-2-(2-méthoxyphényl)acétyl-9-phényl-2,3,3a,4,9,9a-hexahydro-1H-benzo[f]isoindol-3a-acetique-(3aRS, 4SR, 9SR, 9aRS) chauffés au reflux pendant 4 heures dans 8 cm3 de soude 1N et 4 cm3 de méthanol, on obtient, après purification par recristallisation dans l'éther isopropylique, 200 mg (52 %) d'acide 4,9-éthano-2-(2-méthoxyphényl)acétyl-9-phényl-2,3,3a,4,9,9a-hexahydro-1H-benzo[f]isoindol-3a-acétique-(3aRS, 4SR, 9SR, 9aRS) sous forme d'une poudre blanche, dont les caractéristiques sont les suivantes :
- point de fusion = 171°C
- spectre de R.M.N. ¹H (250 MHz, (CD₃)₂SO d6, à une tempe'rature de 393 K, δ en ppm) : 1,37 - 1,64 - de 1,85 à 2,15 (3 mts, respectivement 1H - 1H et 2H : CH₂CH₂) ; 2,00 et 2,14 (2 d, J = 15,5 Hz, 1H chacun : CH₂COO) ; 2,50 (mt, 1H : H en 9a) ; 3,20 (mt, 1H : H en 4) ; de 3,20 à 3,50 (mt, 2H : CH₂ en 1) ; de 3,55 à 3,70 (mt, 1H : 1H du CH₂ en 3) ; 3,56 (s large, 2H : COCH₂Ar) ; 3,71 (mf, 3H : OCH₃) ; 4,12 (d, J = 12,5 Hz, 1H : l'autre H du CH₂ en 3) ; 6,43 (d large, J = 7,5 Hz, 1H : H en 8) ; 6,90 (t, J = 7,5 Hz, 1H : H aromatique en para du OCH₃) ; 6,95 (d, J = 7,5 Hz, 1H : H aromatique en ortho du OCH₃) ; de 7,05 à 7,30 (mt, 5H : H en 5 - H en 6 - H en 7 et H aromatiques en méta du OCH₃) ; de 7,30 à 7,55 (mt, 5H : H aromatiques du phényle en 9).

### EXEMPLE 20

A partir de 300 mg (0,6 mmole) d'acide 4,9-éthano-2-(2-méthoxyphényl)acétyl-9-phényl-2,3,3a,4,9,9a-hexahydro-1H-benzo[f]isoindole-3a-acétique-(3aRS, 4SR, 9SR, 9aRS), de 112,5 mg (0,9 mmole) d'ester méthylique de la glycine, de 180 mg (0,9 mmoles) de chlorhydrate de 1-éthyl-3-(3-diméthylamino-propyl)-carbodiimide, de 8 mg (0,06 mmole) d'hydroxybenzotriazole et de 101 mg (1 mmole) de triéthylamine en solution dans 10 cm3 de dichlorométhane, on obtient, après 18 heures d'agitation à une température voisine de 20°C et purification du produit brut obtenu par flash-chromatographie sur gel de silice (70-230 mesh), en éluant avec un mélange cyclohexane-acétate d'éthyle (4-6 en volumes), 150 mg (45 %) d'ester méthylique de la N-[4,9-éthano-2-(2-méthoxyphényl)acetyl-9-phényl-2,3,3a,4,9,9a-hexahydro-1H-benzo[f]isoindol-3a-yl]acétyl-glycine-(3aRS, 4SR, 9SR, 9aRS) sous forme d'une poudre blanche dont les caractéristiques sont les suivantes :
- point de fusion = 95°C
- spectre de masse (D.I.C. : NH₃) M/Z = 553 (MH⁺).

A partir de 290 mg (0,525 mmole) d'ester méthylique de la N-[4,9-éthano-2-(2-méthoxyphényl)acétyl-9-phényl-2,3,3a,4,9,9a-hexahydro-1H-benzo[f]isoindol-3a-yl]acétyl-glycine-(3aRS, 4SR, 9SR, 9aRS) chauffés au reflux pendant 4 heures dans 10 cm3 de soude 1N et 5 cm3 de méthanol, on obtient, après purification par recristallisation dans l'éther isopropylique, 180 mg (64 %) de N-[4,9-éthano-2-(2-méthoxyphényl)acétyl-9-phényl-2,3,3a,4,9,9a-hexahydro-1H-benzo[f]isoindol-3a-yl] acétyl-glycine-(3aRS, 4SR, 9SR, 9aRS) sous forme d'une poudre blanche dont les caractéristiques sont les suivantes :
- point de fusion = 172°C
- spectre de masse (D.I.C. : NH₃) M/Z = 539 (MH⁺).

A partir de 300 mg (0,6 mmole) d'acide 4,9-éthano-2-(2-méthoxyphényl) acétyl-9-phényl-2,3,3a,4,9,9a-hexahydro-1H-benzo[f]isoindole-3a-acétique-(3aRS, 4SR, 9SR, 9aRS), de 190 mg (0,9 mmole) d'ester méthylique de la méthionine, de 180 mg (0,9 mmoles) de chlorhydrate de 1-éthyl-3-(3-diméthylamino-propyl)-carbodiimide, de 8 mg (0,06 mmole) d'hydroxybenzotriazole et de 101 mg (1 mmole) de triéthylamine en solution dans 10 cm3 de dichlorométhane, on obtient, après 18 heures d'agitation à une température voisine de 20°C et après purification par flash-chromatographie sur gel de silice (70-230 mesh) en éluant avec un mélange cyclohexane-acétate d'éthyle (4-6 en volumes), 200 mg (53 %) d'ester méthylique de la N-[4,9-éthano-2-(2-méthoxyphényl)acétyl-9-phényl-2,3,3a,4,9,9a-hexahydro-1H-benzo[f]isoindol-3a-yl]acétyl-méthionine-(3aRS, 4SR, 9SR, 9aRS) sous forme d'une poudre blanche dont les caractéristiques sont les suivantes :
- point de fusion = 96°C
- spectre de masse (D.I.C. : NH₃) M/Z = 627 (MH⁺).

A partir de 860 mg (1,4 mmole) d'ester méthylique de la N-[4,9-éthano-2-(2-méthoxyphényl)acétyl-9-phényl-2,3,3a,4,9,9a-hexahydro-1H-benzo[f]isoindol-3a-yl]acétyl-méthionine-(3aRS, 4SR, 9SR, 9aRS) chauffés au reflux pendant 12 heures dans 28 cm3 de soude 1N et 14 cm3 de méthanol, on obtient après purification par cristallisation dans l'éther éthylique, 590 mg (69 %) de N-[4,9-éthano-2-(2-méthoxyphényl)acétyl-9-phényl-2,3,3a,4,9,9a-hexahydro-1H-benzo[f]isoindole-3a-yl]acétyl-méthionine-(3aRS, 4SR, 9SR, 9aRS) sous forme d'une poudre blanche dont les caractéristiques sont les suivantes :
- point de fusion = 135°C
- spectre de masse (D.I.C. : NH₃) M/Z = 613 (MH⁺).

### EXEMPLE 22

On chauffe lentement à 100°C une solution de 5 g (9,5 mmoles) de 2-benzyl-4,9-éthano-9-phényl-3a-(trifluorométhylsulfonyloxy)méthyl-2,3,3a,4,9,9a-hexahydro-1H-benzo[f]isoindole-(3aRS, 4SR, 9SR, 9aRS), obtenu dans les conditions de l'exemple 19, dans 50 cm3 de triéthylphosphite. A ce moment une vigoureuse réaction se produit et la température interne atteint 145-150°C. L'agitation est poursuivie pendant 1 heure en maintenant la température entre 100 et 110°C, puis pendant 18 heures en laissant la température descendre au voisinage de 20°C. Le mélange réactionnel est repris par 100 cm3 d'eau et 200 cm3 d'acétate d'éthyle. La phase organique est séparée par décantation, lavée à l'eau, séchée sur sulfate de magnésium et concentrée à sec sous pression réduite. Le résidu obtenu est purifié par flash-chromatographie sur gel de silice (70-230 mesh) en éluant avec un mélange cyclohexane-acétate d'éthyle (1-1 en volumes). On obtient ainsi 1,46 g (29 %) de l'ester diéthylique de l'acide (2-benzyl-4,9-éthano-9-phényl-2,3,3a,4,9,9a-hexahydro-1H-benzo[f]isoindol-3a-yl)méthyl-phosphonique-(3aRS, 4SR, 9SR, 9aRS), sous forme d'une huile jaune-pâle dont les caractéristiques sont les suivantes :
- spectre de masse (E.I.) M/Z = 515 (M⁺).

A partir de 460 mg (0,9 mmoles) de l'ester diéthylique de l'acide (2-benzyl-4,9-éthano-9-phényl-2,3,3a,4,9,9a-hexahydro-1H-ben-zo[f]isoindol-3a-yl)méthyl-phosphonique-(3aRS, 4SR, 9SR, 9aRS) en solution dans 20 cm3 d'éthanol et en présence de 100 mg d'hydroxyde de palladium à 20 % (p/p), on obtient après 6 heures d'agitation sous hydrogène à pression atmosphérique et à 60°C, filtration du catalyseur et concentration à sec sous pression réduite, 370 mg (97 %) de l'ester diéthylique de l'acide (4,9-éthano-9-phényl-2,3,3a,4,9,9a-hexahydro-1H-benzo[f] isoindol-3a-yl)méthyl-phosphonique-(3aRS, 4SR, 9SR, 9aRS) sous forme d'une huile incolore dont les caractéristiques sont les suivantes :
- spectre de masse (D.I.C. : NH₃) M/Z = 425 (MH⁺).

A partir de 600 mg (1,41 mmoles) de l'ester diéthylique de l'acide (4,9-éthano-9-phényl-2,3,3a,4,9,9a-hexahydro-1H-benzo[f]isoindol-3a-yl)méthyl-phosphonique-(3aRS, 4SR, 9SR, 9aRS), de 260 mg (1,56 mmoles) d'acide 2-méthoxyphénylacétique, de 300 mg (1,56 mmoles) de chlorhydrate de 1-éthyl-3-(3-diméthylamino-propyl)-carbodiimide et de 19 mg (0,14 mmole) d'hydroxybenzotriazole en solution dans 20 cm3 de dichlorométhane, on obtient, après 18 heures d'agitation à une température voisine de 20°C et purification du produit brut obtenu par flash-chromatographie sur gel de silice (70-230 mesh) en éluant avec un mélange cyclohexane-acétate d'éthyle (1-9 en volumes), 350 mg (43 %) de l'ester diéthylique de l'acide (4,9-éthano-2-(2-méthoxyphényl)acétyl-9-phényl-2,3,3a,4,9,9a-hexahydro-1H-benzo[f]isoindol-3a-yl)méthyl-phosphonique-(3aRS, 4SR, 9SR, 9aRS) sous forme d'une poudre blanche dont les caractéristiques sont les suivantes :
- point de fusion = 130°C
- spectre de R.M.N. ¹H (400 MHz, (CD₃)₂SO d6 avec ajout de quelques gouttes de CD₃COOD d4, à une température de 403 K, δ en ppm) : 1,23 et 1,25 (2 t, J = 7 Hz, 6H : CH₃ de l'éthyle) ; 1,37 - 1,64 - 1,90 et 2,05 (4 mts, 1H chacun : CH₂CH₂) ; de 1,55 à 1,85 (mt, 2H : CH₂P) ; 2,62 (mt, 1H : H en 9a) ; 3,28 (mt, 1H : H en 4) ; de 3,30 à 3,50 (mt, 2H : CH₂ en 1) ; 3,58 (s large, 2H : COCH₂Ar) ; de 3,65 à 3,80 (mt, 1H : 1H du CH₂ en 3) ; 3,70 (mf, 3H : OCH₃) ; de 3,85 à 4,10 (mt, 4H : OCH₂ de l'éthyle) ; 4,09 (d, J = 13 Hz, 1H : l'autre H du CH₂ en 3) ; 6,44 (d large, J = 7,5 Hz, 1H : H en 8) ; 6,88 (t, J = 7,5 Hz, 1H : H aromatique en para du OCH₃) ; 6,93 (d, J = 7,5 Hz, 1H : H aromatique en ortho du OCH₃) ; 7,07 (dt, J = 7,5 et 1,5 Hz, 1H : H en 7) ; de 7,15 à 7,35 (mt, 4H : H en 5 - H en 6 et H aromatiques en méta du OCH₃) ; 7,35 (mt, 3H : H aromatiques en ortho et en para du phényle en 9) ; 7,47 (t, J = 7,5 Hz, 2H : H aromatiques en méta du phényle en 9).

### EXEMPLE 23

On dissout 850 mg (1,5 mmoles) de l'ester diéthylique de l'acide (4,9-éthano-2-(2-méthoxyphényl)acétyl-9-phényl-2,3,3a,4,9,9a-hexahydro-1H-benzo[f] isoindol-3a-yl)méthyl-phosphonique-(3aRS, 4SR, 9SR, 9aRS) dans 10 cm3 de tétrachlorure de carbone sec puis on refroidit vers -20°C et on ajoute 600 mg (3 mmoles) d'iodure de triméthylsilane. On laisse remonter progressivement la température à une température voisine de 20°C en 2-3 heures puis on agite encore pendant 1 heure. On ajoute alors 2 cm3 d'eau et 2 cm3 d'une solution aqueuse 0,1 N de thiosulfate de sodium, puis on extrait à l'éther. La phase organique est lavée à l'eau, séchée sur sulfate de sodium et concentrée à sec sous pression réduite. L'huile résiduelle est cristallisée dans 20 cm3 d'éthanol aqueux à 80 % (en volumes), puis empâtée à reflux dans 25 cm3 d'éthanol. On obtient ainsi 230 mg (30 %) d'acide (4,9-éthano-2-(2-méthoxyphényl)acétyl-9-phényl-2,3,3a,4,9,9a-hexahydro-1H-benzo [f]isoindol-3a-yl)méthyl-phosphonique-(3aRS, 4SR, 9SR, 9aRS) sous forme d'une poudre blanche dont les caractéristiques sont les suivantes :
- point de fusion supérieur à 260°C
- spectre de masse (D.I.C. : NH₃) M/Z = 518 (MH⁺).

### EXEMPLE 24

On ajoute, goutte à goutte, une solution de 436 mg (3,3 mmoles) de malonate de méthyle dans 10 cm3 de dioxane sec à une suspension de 160 mg (3,3 mmoles) d'hydrure de sodium (à 50 % dans l'huile) dans 20 cm3 de dioxane sec. On agite à une température voisine de 20°C pendant 30 minutes après la fin du dégagement d'hydrogène, puis on ajoute lentement une solution de 1,52 g (2,9 mmoles) de 2-benzyl-4,9-éthano-9-phényl-3a-(trifluorométhylsulfonyloxy) méthyl-2,3,3a,4,9,9a-hexahydro-1H-benzo[f]isoindole-(3aRS, 4SR, 9SR, 9aRS), obtenu dans les conditions de l'exemple 19, dans 20 cm3 de dioxane sec. Le mélange réactionnel est chauffé pendant 1 heure au reflux, puis agité pendant 18 heures à une température voisine de 20°C. Après dilution avec 100 cm3 d'eau, on extrait 3 fois avec 50 cm3 d'acétate d'éthyle. Les phases organiques sont lavées à séchées sur sulfate de sodium, filtrées sur 10 g de silice et concentrées à sec sous pression réduite. On obtient ainsi 1,21 g (82 %) de diester méthylique de l'acide 2-(2-benzyl-4,9-éthano-9-phényl-2,3,3a,4,9,9a-hexahydro-1H-benzo[f]isoindol-3a-yl)méthyl-malonique-(3aRS, 4SR, 9SR, 9aRS) sous forme d'une huile incolore dont les caractéristiques sont les suivantes :
- spectre de masse (D.I.C. : NH₃) M/Z = 510 (MH⁺).

A partir de 1,2 g (2,3 mmoles) de diester méthylique de l'acide 2-(2-benzyl-4,9-éthano-9-phényl-2,3,3a,4,9,9a-hexahydro-1H-benzo[f]isoindol-3a-yl)méthyl-malonique-(3aRS, 4SR, 9SR, 9aRS) en solution dans 20 cm3 de méthanol et en présence de 250 mg d'hydroxyde de palladium à 20 % (p/p), on obtient, après 6 heures sous hydrogène à pression atmosphérique et à 60°C filtration du catalyseur et concentration à sec sous pression réduite, 930 mg (96 %) de diester méthylique de l'acide 2-(4,9-éthano-9-phényl-2,3,3a,4,9,9a-hexahydro-1H-benzo[f]isoindol-3a-yl) méthyl-malonique-(3aRS, 4SR, 9SR, 9aRS) sous forme d'une huile incolore dont les caractéristiques sont les suivantes :
- spectre de masse (D.I.C. NH₃) M/Z = 420 (MH⁺).

A partir de 530 mg (1,27 mmoles) de diester méthylique de l'acide 2-(4,9-éthano-9-phényl-2,3,3a,4,9,9a-hexahydro-1H-benzo[f]isoindol-3a-yl)méthyl-malonique-(3aRS, 4SR, 9SR, 9aRS), de 232 mg (1,4 mmoles) d'acide 2-méthoxyphénylacétique, de 270 mg (1,4 mmoles) de chlorhydrate de 1-éthyl-3-(3-diméthylamino-propyl)-carbodiimide et de 17 mg (0,13 mmole) d'hydroxybenzotriazole en solution dans 20 cm3 de dichlorométhane, on obtient, après 18 heures d'agitation à une température voisine de 20°C et purification du produit brut obtenu par flash-chromatographie sur gel de silice (70-230 mesh) en éluant avec un mélange cyclohexane-acétate d'éthyle (1-9 en volumes), 350 mg (47 %) de diester méthylique de l'acide 2- [4,9-éthano-2-(2-méthoxyphényl)acétyl-9-phényl-2,3,3a,4, 9,9a-hexahydro-1H-benzo[f]isoindol-3a-yl]méthyl-malonique-(3aRS, 4SR, 9SR, 9aRS) sous forme d'une poudre blanche dont les caractéristiques sont les suivantes :
- point de fusion = 75°C
- spectre de masse (D.I.C. : NH₃) M/Z = 568 (MH⁺).

A partir de 280 mg (0,5 mmole) d'ester diméthylique de l'acide 2-[4,9-éthano-2-(2-méthoxyphényl)acétyl-9-phényl-2,3,3a,4,9,9a-hexahydro-1H-benzo[f] isoindol-3a-yl]méthyl-malonique-(3aRS, 4SR, 9SR, 9aRS) chauffés au reflux pendant 4 heures dans 10 cm3 de soude 1N et 5 cm3 de méthanol, on obtient après purification par cristallisation dans l'éther isopropylique, 110 mg (41 %) d'acide 2-(4,9-éthano-[(2-méthoxyphényl)acétyl]-9-phényl-1,2,3,3a,9,9a-hexahydro-1H-benzo[f]isoindole-3a-}méthyl-malonique-(3aRS, 4SR, 9SR, 9aRS) sous forme d'une poudre blanche dont les caractéristiques sont les suivantes :
- point de fusion = 200°C
- spectre de masse (D.I.C. : NH₃) M/Z = 539 (MH⁺).

### EXEMPLE 25

On chauffe une solution de 770 mg (1,4 mmoles) d'acide 2-[4,9-éthano-2-(2-méthoxyphényl)acétyl-9-phényl-1,2,3,3a,9,9a-hexahydro-1H-benzo[f]isoindol-3a-yl]méthyl-malonique-(3aRS, 4SR, 9SR, 9aRS) dans 20 cm3 d'acétonitrile en présence de 19,6 mg (0,14 mmole) d'oxyde cuivreux vers 80-90°C jusqu'à l'apparition d'un dégagement de dioxyde de carbone. Lorsque le dégagement gaseux cesse, on maintient le chauffage pendant 2 minutes, puis on refoidit rapidement. On dilue avec 50 cm3 d'acétate d'éthyle et 2 cm3 d'une solution 1N d'ammoniaque. Le gel obtenu est filtré successivement sur célite puis sur silice. Après élimination des solvants, le résidu est repris par 20 cm3 de dichlorométhane. La phase organique est lavée avec de l'acide chlorhydrique aqueux 0,5N séchée sur sulfate de magnésium et concentrée à sec sous pression réduite. Par cristallisation dans l'éther isopropylique, on obtient 220 mg (32 %) d'acide 3-[4,9-éthano-2-(2-méthoxyphényl)acétyl-9-phényl-1,2,3,3a,9,9a-hexahydro-1H-benzo[f]isoindol-3a-yl]-propionique (3aRS, 4SR, 9SR, 9aRS) sous forme d'une poudre blanche dont les caractéristiques sont les suivantes :
- point de fusion = 200°C
- spectre de R.M.N. ¹H (250 MHz, (CD₃)₂SO d6, à une température de 393 K, δ en ppm) : 1,25 et 1,38 (2 mts, 1H chacun : CH₂ du propionyloxy) ; 1,38 - 1,67 - 1,90 et 2,06 (4 mts, 1H chacun : CH₂CH₂) ; 2,20 et 2,35 (2 mts, 1H chacun : CH₂COO du propionyloxy) ; 2,38 (mt, 1H : H en 9a) ; 3,06 (mt, 1H : H en 4) ; de 3,30 à 3,55 (mt, 2H : CH₂ en 1) ; de 3,40 à 3,70 (mt, 1H : 1H du CH₂ en 3) ; 3,58 (s large, 2H : COCH₂Ar) ; 3,70 (mf, 3H : OCH₃) ; 4,00 (d, J = 12,5 Hz, 1H : l'autre H du CH₂ en 3) ; 6,44 (d large, J = 7,5 Hz, 1H : H en 8) ; 6,90 (t, J = 7,5 Hz, 1H : H aromatique en para du OCH₃) ; 6,95 (d, J = 7,5 Hz, 1H : H aromatique en ortho du OCH₃) ; 7,07 (dt, J = 7,5 et 1,5 Hz, 1H : H en 7) ; de 7,15 à 7,35 (mt, 4H : H en 5 - H en 6 et H aromatiques en méta du OCH₃) ; 7,35 (mt, 3H : H aromatiques en ortho et en para du phényle en 9) ; 7,48 (t large, J = 7,5 Hz, 2H : H aromatiques en méta du phényle en 9).

### EXEMPLE 26

A partir de 436 mg (3,3 mmoles) d'ester méthylique de l'acide 3-mercapto-propanoïque dans 10 cm3 de dioxane sec, de 160 mg (3,3 mmoles) d'hydrure de sodium (à 50 % dans l'huile) dans 20 cm3 de dioxane et de 1,52 g (2,9 mmoles) de 2-benzyl-4,9-éthano-9-phényl-3a-(trifluorométhylsulfonyloxy)méthyl-2,3,3a,4,9, 9a-hexahydro-1H-benzo[f]isoindole-(3aRS, 4SR, 9SR, 9aRS), obtenu dans les conditions de l'exemple 19, dans 20 cm3 de dioxane chauffés pendant 1 heure au reflux, puis agités pendant 18 heures à une température voisine de 20°C, on obtient 1,34 g (93 %) d'ester méthylique de l'acide 3-(2-benzyl-4,9-éthano-9-phényl-2,3,3a,4,9,9a-hexahydro-1H-benzo[f]isoindol-3a-yl)méthylthio-propanoïque-(3aRS, 4SR, 9SR, 9aRS) sous forme d'une huile jaune-pâle dont les caractéristiques sont les suivantes :
- spectre de masse (E.I.) M/Z = 497 (M⁺).

A une solution, refroidie à -10°C de 600 mg (1,2 mmoles) d'ester méthylique de l'acide 3-(2-benzyl-4,9-éthano-9-phényl-2,3,3a,4,9,9a-hexahydro-1H-benzo[f]isoindol-3a-yl)méthylthio-propanoïque-(3aRS, 4SR, 9SR, 9aRS) dans 10 cm3 de dichlorométhane sec, on ajoute, goutte à goutte, sous atmosphère d'azote et en maintenant la température inférieure à 0°C, une solution de 257 mg de chloroformiate de 1-chloroéthyle dans 5 cm3 de dichlorométhane sec. Après 30 minutes d'agitation à 0°C puis 1 heure à une température voisine de 20°C, le mélange réactionnel est concentré à sec, repris par 10 cm3 de méthanol chauffé au reflux pendant 2 heures puis agité à une température voisine de 20°C pendant 18 heures. Après concentration du méthanol sous pression réduite, le résidu est repris par 20 cm3 de dichlorométhane. La phase organique est lavée à l'eau, séchée sur sulfate de sodium et concentrée à sec. On obtient ainsi 400 mg (82 %) d'ester méthylique de l'acide 3-(4,9-éthano-9-phényl-2,3,3a,4,9,9a-hexahydro-1H-benzo[f] isoindol-3a-yl)méthylthio-propanoique-(3aRS, 4SR, 9SR, 9aRS) sous forme d'une huile jaune-pâle.

A partir de 390 mg (0,96 mmole) d'ester méthylique de l'acide 3-(4,9-éthano-9-phényl-2,3,3a,4,9,9a-hexahydro-1H-benzo[f]isoindol-3a-yl)méthylthio-propanoïque-(3aRS, 4SR, 9SR, 9aRS), de 190 mg (1,1 mmoles) d'acide 2-méthoxyphénylacétique, de 220 mg (1,1 mmoles) de chlorhydrate de 1-éthyl-3-(3-diméthylamino-propyl)-carbodiimide et de 14 mg (0,1 mmole) d'hydroxybenzotriazole en solution dans 20 cm3 de dichlorométhane, on obtient, après 18 heures d'agitation à une température voisine de 20°C et purification du produit brut obtenu par flash-chromatographie sur gel de silice (70-230 mesh), en éluant avec un mélange cyclohexane-acétate d'éthyle (6-4 en volumes), 210 mg (39 %) d'ester méthylique de l'acide 3-[4,9-éthano-2-(2-méthoxyphényl)acétyl-9-phényl-2,3,3a,4,9, 9a-hexahydro-1H-benzo[f]isoindol-3a-yl]-méthylthio-propanoïque-(3aRS, 4SR, 9SR, 9aRS) sous forme d'une laque blanche dont les caractéristiques sont les suivantes :
- point de fusion = 30-35°C
- spectre de masse (D.I.C. : NH₃) M/Z = 555 (MH⁺).

A partir de 180 mg (0,32 mmole) d'ester méthylique de l'acide 3-[4,9-éthano-2-(2-méthoxyphényl)acétyl-9-phényl-2,3,3a,4,9,9a-hexahydro-1H-benzo[f] isoindol-3a-yl]-méthylthio-propanoïque-(3aRS, 4SR, 9SR, 9aRS) chauffés au reflux pendant 4 heures dans 6,4 cm3 de soude 1N et 3,2 cm3 de méthanol, on obtient, après purification par cristallisation dans l'éther isopropylique, 120 mg (69 %) d'acide 3-[4,9-éthano-2-(2-méthoxyphényl)acétyl-9-phényl-2,3,3a,4,9,9a-hexahydro-1H-benzo[f]isoindol-3a-yl]-méthylthio-propanoique-(3aRS, 4SR, 9SR, 9aRS) sous forme d'une poudre blanche dont les caractéristiques sont les suivantes :
- point de fusion = 120°C
- spectre de R.M.N. ¹H (250 MHz, (CD₃)₂SO d6, à une température de 393 K, δ en ppm) : 1,38 - 1,67 et de 1,85 à 2,15 (3 mts, respectivement 1H -1H et 2H : CH₂CH₂) ; 2,26 et 2,41 (2 d, J = 12 Hz, 1H chacun : CH₂S) ; 2,40 (t, J = 7 Hz, 2H : CH₂COO) ; 2,54 (mt, 1H : H en 9a) ; 2,66 (t, J = 7 Hz, 2H : CH₂S) ; 3,16 (mt, 1H : H en 4) ; de 3,30 à 3,75 (mt, 3H : CH₂ en 1 et 1H du CH₂ en 3) ; 3,58 (s large, 2H : COCH₂Ar) ; 3,72 (mf, 3H : OCH₃) ; 4,00 (d, J = 12,5 Hz, 1H : l'autre H du CH₂ en 3) ; 6,44 (d large, J = 7,5 Hz, 1H : H en 8) ; 6,90 (t, J = 7,5 Hz, 1H : H aromatique en para du OCH₃) ; 6,95 (d, J = 7,5 Hz, 1H : H aromatique en ortho du OCH₃) ; 7,09 (dt, J = 7,5 et 1,5 Hz, 1H : H en 7) ; de 7,15 à 7,35 (mt, 4H : H en 5 - H en 6 et H aromatiques en méta du OCH₃) ; de 7,35 7,45 (mt, 3H : H aromatiques en ortho et en para du phényle en 9) ; 7,49 (t large, J = 7,5 Hz, 2H : H aromatiques en méta du phényle en 9).

### EXEMPLE 27

A partir de 1,2 g (3,6 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), obtenu dans les conditions de l'exemple 1, chauffés au reflux pendant 4 heures dans 36 cm3 de soude 2N et 36 cm3 d'éthanol, on obtient, après purification du produit brut obtenu après concentration par dissolution dans la soude N et reprécipitation par neutralisation à pH=6,5 avec une solution d'acide chlorhydrique N, on obtient 1,12 g (97 %) d'acide 4,9-éthano-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion supérieur à 260°C
- spectre de masse (E.I.) M/Z= 319 (M⁺).

On dissout 500 mg (1,56 mmoles) d'acide 4,9-éthano-9-phényl-1,2,3,3a,9,9a-hexahydro-1H-benzo[f]isoindole-3a-carboxylique-(3aRS, 4SR, 9SR, 9aRS) dans 50 cm3 de soude 0,1N et 50 cm3 de dioxane par chauffage à 60°C. Après refroidissement à une température voisine de 20°C, on ajoute 580 mg (3,12 mmoles) de chloroformiate de (2-méthoxyphényl)méthyle, puis on agite pendant 4 jours. à une température voisine de 20°C. Après concentration à sec, le résidu est puriifé par chromatographie sur plaque préparative de silice, en éluant avec un mélange dichlorométhane-méthanol-acide acétique (90-6-4 en volumes). On obtient ainsi 175 mg (24 %) d'acide 4,9-éthano-2-(2-méthoxyphényl)oxycarbonyl-9-phényl-1,2,3,3a,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 251°C
- spectre de R.M.N. ¹H (300 MHz, (CD₃)₂SO d6, δ en ppm) : à température ambiante, nous observons un mélange de rotamères dans les proportions 50/50. 1,47 - 1,78 et de 2,00 à 2,50 (3 mts, respectivement 1H - 1H et 2H : CH₂CH₂) ; de 3,25 à 3,65 (mt, 5H : CH₂ en 1- 1H du CH₂ en 3 - H en 4 et H en 9a) ; 3,75 et 3,77 (2 s, 3H en totalité : OCH₃) ; 4,10 et 4,25 (2 d, J = 11,5 Hz, 1H en totalité : l'autre H du CH₂ en 3) ; 6,40 (d large, J = 7,5 Hz, 1H : H en 8) ; 6,93 (dt, J = 7,5 et 2 Hz, 1H : H en 7) ; de 6,90 à 7,65 (mt, 11H : H en 5 - H en 6 - H aromatiques en ortho -méta et para du OCH₃ et H aromatiques du phényle en 9).

### EXEMPLE 28

A partir de 3 g (9 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 2,32 g (10,8 mmoles) d'acide 2-bromophénylacétique, de 2,07 g (10,8 mmoles) de chlorhydrate de 1-éthyl-3-(3-diméthylamino-propyl)-carbodiimide et de 121 g (0,9 mmole) d'hydroxybenzotriazole en solution dans 100 cm3 de dichlorométhane, on obtient, après 18 heures d'agitation à une température voisine de 20°C et purification du produit brut obtenu par flash-chromatographie sur gel de silice (70-230 mesh), en éluant avec un mélange cyclohexane-acétate d'éthyle (5-5 en volumes), 286 g (60 %) d'ester méthylique de l'acide 4,9-éthano-2-(2-bromophényl)acétyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 128-130°C
- spectre de masse (D.C.I. : NH₃) M/Z = 530 (MH⁺).

A partir de 1 g (1,82 mmoles) d'ester méthylique de l'acide 4,9-éthano-2-(2-bromophényl)acétyl-9-phényl-2,3,3a,4,9,9a-hexahydro-1H-benzo[f]isoindole-3a-carboxylique-(3aRS, 4SR, 9SR, 9aRS) chauffé au reflux pendant 4 heures dans 36 cm3 de soude 1N et 36 cm3 d'éthanol, on obtient après purification par flash-chromatographie sur gel de silice (70-230 mesh), en éluant avec un mélange dichlorométhane-méthanol-acide acétique (95-4-1 en volumes), 430 mg (44 %) d'acide 4,9-éthano-2-(2-bromophényl)acétyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 167°C
- spectre de masse (D.C.I. : NH₃) M/Z = 516 (MH⁺).

### EXEMPLE 29

On dissout 200 mg (0,44 mmole) d'acide 4,9-éthano-2-(2-hydroxyphényl)acétyl-9-phényl-2,3,3a,4,9,9a-hexahydro-1H-benzo[f]isoindole-3a-carboxylique-(3aRS, 4SR, 9SR, 9aRS), obtenu dans les conditions de l'exemple 60 ci-après, 106 mg ( 1,32 mmoles) de pyridine et 11 mg (0,09 mmole) de diméthylamino-4 pyridine dans 30 cm3 de dichlorométhane, puis on ajoute 225 mg (2,2 mmoles) d'anhydride acétique. Après 18 heures d'agitation à une température voisine de 20°C, l'excès d'anhydride acétique est neutralisé par addition de soude aqueuse 1N. La phase organique séparée par décantation est lavée successivement à l'eau, avec une solution 0,1N d'acide chlorhydrique et puis à l'eau, séchée sur sulfate de magnésium et concentratée à sec sous pression réduite Le résidu obtenu est purifié par chromatographie sur une plaque préparative de silice en éluant avec un mélange dichlorométhane-méthanol-acide acétique (95-4,5-0,5 en volumes). On obtient ainsi 78 mg (36 %) d'acide 4,9-éthano-2-(2-acétoxyphényl)acétyl-9-phényl-2,3,3a,4,9,9a-hexahydro-1H-benzo [f]isoindole-3a-carboxylique-(3aRS, 4SR, 9SR, 9aRS), sous forme d'une poudre blanche dont les caractéristiques sont les suivantes :
- point de fusion = 157-158°C
- spectre de masse (E.I.) M/Z = 496 (M⁺).

### EXEMPLE 30

A partir de 901 mg (2,7 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), 600 mg (3,3 mmoles) d'acide (2-méthylthiophényl)acétique, qui peut être obtenu selon J. Organomet. Chem., 178, 800 (1979), de 630 mg (3,3 mmoles) de chlorhydrate de 1-éthyl-3-(3-diméthylamino-propyl)-carbodiimide et de 36 mg (0,27 mmole) d'hydroxybenzotriazole en solution dans 30 cm3 de dichlorométhane, on obtient, après 18 heures d'agitation à une température voisine de 20°C et purification du produit brut obtenu par flash-chromatographie sur gel de silice (70-230 mesh), en éluant avec un mélange cyclohexane-acétate d'éthyle (7-3 en volumes), 1,25 g (92 %) d'ester méthylique de l'acide 4,9-éthano-2-(2-méthylthiophényl)acétyl-9-phényl-2,3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 155°C
- spectre de masse (D.C.I. : NH₃) M/Z = 498 (MH⁺).

A partir de 1,25 g (2,5 mmoles) d'ester méthylique de l'acide 4,9-éthano-2-(2-méthylthiophényl)acétyl-9-phényl-2,3,3a,4,9,9a-hexahydro-1H-benzo[f]isoindole-3a-carboxylique-(3aRS, 4SR, 9SR, 9aRS) chauffé au reflux pendant 4 heures dans 31,5 cm3 de soude 1N et 31,5 cm3 d'éthanol, on obtient, après purification par cristallisation dans l'éther isopropylique, 780 mg (64 %) d'acide 4,9-éthano-2-(2-méthylthiophényl)acétyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 237°C
- spectre de masse (D.C.I. / NH₃) M/Z = 484 (MH⁺).

### EXEMPLE 31

774 mg (1,6 mmoles) d'acide 4,9-éthano-2-(2-méthylthio-phényl)acétyl-9-phényl-2,3,3a,4,9,9a-hexahydro-1H-benzo[f]isoindol-3a-carboxylique-(3aRS, 4SR, 9SR, 9aRS) sont dédoublés sur une colonne de silice chirale, porteuse de greffons (3,5-dinitrobenzyl)phénylalanine en éluant par un mélange dichlorométhane-isopropanol-n.heptane (85-10-5 en volumes). En recueillant la deuxième fraction éluée (temps de rétention = 15,4 minutes.), on obtient 330 mg d'énantiomère dextrogyre de l'acide 4,9-éthano-2-(2-méthylthiophényl)acétyl-9-phényl-2,3,3a,4,9, 9a-hexahydro-1H-benzo[f]isoindole-3a-carboxylique-(3aRS, 4SR, 9SR, 9aRS), énantiomère dextrogyre, sous forme de cristaux blancs dont les caractéristiques sont les suivantes :
- point de fusion = 133°C
- pouvoir rotatoire [α]₃₆₅²⁰ = +187,2 ± 3 (c = 0,5 ; dichlorométhane)
- spectre de masse (D.C.I. : NH₃) M/Z = 484 (MH⁺).

La silice chirale peut être préparée de la manière suivante :
Dans un tricol de 6 litres, on met en suspension 948 g de silice amino-propyle (100 Å - 10 µm - NH₂ ; Macherey-Nagel) dans 3 litres de diméthylformamide. On ajoute 180 g d'anhydride de l'acide N-tert-butoxy-carbonylamino-11-undécanoïque et on agite le mélange réactionnel pendant 18 heures à une température voisine de 20°C. La silice est séparée par filtration et lavée successivement par 2 fois 2500 cm3 de dichlorométhane puis 2 fois 2500 cm3 de diméthylformamide. La silice ainsi lavée est remise en suspension dans 3 litres de diméthylformamide et on ajoute 180 g d'anhydride de l'acide N-tert-butoxycarbonylamino-11-undécanoïque puis on agite le mélange réactionnel pendant 18 heures à une température voisine de 20°C. La silice est séparée par filtration, lavée successivement par 2 fois 2500 cm3 de dichlorométhane, 2 fois 2500 cm3 de tétrahydrofuranne, 2 fois 2500 cm3 de méthanol et 2 fois 2500 cm3 d'oxyde de diéthyle puis séchée sous pression réduite à une température voisine de 20°C. On obtient ainsi 971 g de silice désignée par l'appellation "BOC-C₁₁-C₃-silice" sous forme de poudre blanche dont la structure est confirmée par le spectre infra-rouge et dont l'analyse élémentaire (trouvée) est : C % = 9,85 ; H % = 2,05 ; N % = 1,05.

Dans un tricol de 6 litres, on met en suspension 971 g de silice "BOC-C₁₁-C₃-silice" dans 2,5 litres de dichlorométhane et 470 g d'imidazole. On ajoute goutte à goutte 850 cm3 de diméthyloctylchlorosilane et on agite le mélange réactionnel pendant 16 heures à une température voisine de 20°C. Le solide obtenu est séparé par filtration et lavé successivement par 2 fois 2,5 litres de dichlorométhane, deux fois 2,5 litres de méthanol, 2 fois 2,5 litres de tétrahydrofurane, deux fois 2,5 litres de dichlorométhane et 2 fois 2,5 litres d'oxyde de diéthyle puis séché sous pression réduite à une température voisine de 20°C. On obtient ainsi 1179 g de silice désignée par l'appellation "BOC-C₁₁-C₃-silice-O-Si(CH₃)₂(CH₂)₇CH₃" sous forme de poudre blanche dont la structure est confirmée par le spectre infra-rouge et dont l'analyse élémentaire (trouvée) est : C % = 13,9 ; H % =2,83 ; N % = 1,16.

Dans un tricol de 6 litres, on met en suspension 1178 g de silice "BOC-C₁₁-C₃-silice-O-Si(CH₃)₂(CH₂)₇CH₃" dans 2500 cm3 d'une solution à 5 % en volume d'acide trifluoroacétique dans le dichlorométhane. On agite 20°C. La silice est séparée par filtration et lavée successivement par 2 fois 2,5 litres de dichlorométhane, 2 fois 2,5 litres d'un mélange dichlorométhane-diisopropyl-éthylamine (70-30 en volumes), 2,5 litres de dichlorométhane, 2 fois 2,5 litres de tétrahydrofurane, 2 fois 2 litres de méthanol et 2 fois 2 litres d'oxyde de diéthyle puis séchée sous pression réduite à une température voisine de 50°C. On obtient ainsi 1080,5 g de silice désignée par l'appellation "C₁₁-C₃-silice-O-Si(CH₃)₂(CH₂)₇CH₃" sous forme de poudre blanche dont la structure est confirmée par le spectre infra-rouge et dont l'analyse élémentaire (trouvée) est : C % = 12,6 : H % = 2,44 ; N % = 1,05.

Dans un ballon tricol de 6 litres, on met en suspension 1080 g de silice "C₁₁-C₃- (silice)-O-Si(CH₃)₂(CH₂)₇CH₃" dans 2500 cm3 de diméthylformamide séché sur tamis moléculaire 4Å. On ajoute 108 g de N-benzoyl-3,5-dinitro-L-phénylalanine et 75 g de N-éthoxycarbonyl-2-éthoxy-1,2-dihydro-quinoléine. On agite le mélange réactionnel pendant 1 nuit. La silice est séparée par filtration sur verte fritté puis est lavée par 2 fois 2500 cm3 de dichlorométhane, 2 fois 2500 cm3 de tétrahydrofurane, 2500 cm3 de diméthylformamide et 2500 cm3 de dichlorométhane. La silice ainsi lavée est remise en suspension dans 2500 cm3 de diméthylformamide. On ajoute successivement 108 g de N-benzoyl-3,5-dinitro-L-phénylalanine et 75 g de N-éthoxycarbonyl-2-éthoxy-1,2-dihydro-quinoléine puis on agite pendant une nuit à 20°C. La silice est séparée par filtration sur verte fritté et est lavée successivement par 2 fois 2500 cm3 de dichlorométhane, 2 fois 2500 cm3 de tétrahydrofurane, 2 fois 2500 cm3 de méthanol et 2 fois 2500 cm3 d'éther éthylique. Après séchage à 60°C sous pression réduite (20 mm de mercure : 2,7 kPa), on obtient 1093,6 g de silice désignée par l'appellation "DNB-L-Phe-C₁₁-C₃ (silice)-O-Si(CH₃)₂(CH₂)₇CH₃", sous forme d'une poudre jaune pâle dont la structure est confirmée par son spectre infra-rouge, et dont l'analyse élémentaire (trouvée) est C % = 14,5 H % = 2,4, N % = 1,68.

Dans un ballon tricol de 4 litres, on met en suspension 519 g de silice "DNB-L-Phe-C₁₁-C₃ (silice)-O-Si(CH₃)₂(CH₂)₇CH₃" dans 3000 cm3 de diméthylformamide séché sur tamis moléculaire 4 Å. On ajoute 450 cm3 de triméthylsilylimidazole en 15 minutes, puis on agite pendant une nuit. La silice est séparée par filtration et est lavée successivement par 2 fois 1500 cm3 de tétrahydrofurane, 2 fois 1500 cm3 de méthanol, 2 fois 1500 cm3 d'acétone et 2 fois 1500 cm3 de dichlorométhane. Après séchage à une température de 60°C sous pression réduite (2,7 kPa), on obtient 519 g de silice désignée par l'appellation "DNB-L-Phe-C₁₁-C₃ (silice)-[O-Si(CH₃)₂(CH₂)₇CH₃]-[O-Si(CH₃)₃]" sous forme d'une poudre jaune pâle dont la structure est confirmée par son spectre infra-rouge, et dont l'analyse élémentaire (trouvée) est C % = 15,3 H % = 1,8, N % = 2,6.

L'anhydride de l'acide N-tert.butoxycarbonyl-11-amino-undécanoïque peut être préparé de la manière suivante :
On dissout 30,1 g d'acide N-tert.butoxycarbonyl-11-amino-undécanoïque dans 480 cm3 d'acétate d'éthyle. On refroidit cette solution à 5°C puis, en la maintenant à cette température, on ajoute en 10 minutes une solution de 10,63 g de dicyclohexyl-carbodiimide dans 120 cm3 d'acétate d'éthyle. On agite le mélange réactionnel pendant 1 heure à 5°C puis pendant 16 heures à une température voisine de 20°C. On sépare le précipité formé par filtration et le lave par 30 cm3 d'acétate d'éthyle. Le filtrat est concentré à sec sous pression réduite (20 mm de mercure : 2,7 kPa) à 30°C. Le solide obtenu est séché à 20°C sous pression réduite (20 mm de mercure ; 2,7 kPa). On obtient ainsi, avec un rendement voisin de 100 %, 31 g d'anhydride de l'acide N-tert.butoxycarbonyl-11-amino-undécanoïque.

L'acide N-tert.butoxycarbonyl-11-amino-undécanoïque peut être préparé de la manière suivante :
Dans un tricol de 4 litres muni d'une agitation mécanique et d'un réfrigérant, on introduit successivement 160 g d'acide-11-amino-undécanoïque, 2 litres de dioxane, 1,3 litre d'eau distillée, 208 g de carbonate de sodium et 173 g de di-tert.butyl dicarbonate. Le mélange réactionnel est chauffé à l'ébullition pendant 16 heures. On obtient ainsi une solution limpide. Après refroidissement à 20°C, le mélange réactionnel est versé sur 800 g de glace puis acidifié à pH=3-4 par addition d'acide chlorhydrique 4N. On obtient ainsi un précipité blanc qui est séparé par filtration, lavé par 300 cm3 d'eau et séché à 20°C sous pression réduite (20 mm de mercure : 2,7 kPa). On obtient ainsi, avec un rendement de 95 %, 232 g d'acide N-tert.butoxycarbonyl-11-amino-undécanoïque dont le point de fusion (68°C) est en accord avec celui qui est donné dans J. Org. Chem., 41, 1350 (1976).

### EXEMPLE 32

A partir de 1 g (3 mmoles) d'ester méthylique de l'acide 4,9-éthano-9-phényl-2,3,3a,4,9,9a-hexahydro-1H-benzo[f]isoindole-3a-acétique-(3aRS, 4SR, 9SR, 9aRS), de 947 mg (3,6 mmoles) de chlorhydrate de l'acide 2-(3-pyrrolidinyl-propyl)oxyphénylacétique, qui peut être obtenu selon le brevet européen EP 0 514 275, de 690 mg (3,6 mmoles) de chlorhydrate de 1-éthyl-3-(3-diméthylamino-propyl)-carbodiimide et de 40 mg (0,3 mmole) d'hydroxybenzotriazole en solution dans 20 cm3 de dichlorométhane, on obtient, après 18 heures d'agitation à une température voisine de 20°C un produit brut qui précipite. Le précipité formé est essoré et lavé à l'eau, puis remis en suspension dans 25 cm3 d'eau et 25 cm3 d'acétate d'éthyle. La suspension est amenée à pH=8 par addition de soude 1N. Après décantation de la phase organique, la phase aqueuse est extraite avec 2 fois 20 cm3 d'acétate d'éthyle. Les phases organiques réunies sont lavées à l'eau, séchées sur sulfate de magnésium et concentrées à sec sous pression réduite. On obtient ainsi 1,63 g (94 %) d'ester méthylique de l'acide 4,9-éthano-2-[2-(3-pyrrolidinylpropyl) oxyphényl]acétyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 118°C
- spectre de masse (D.C.I. : NH₃) M/Z = 579 (MH⁺).

A partir de 1,60 g (2,77 mmoles) d'ester méthylique de l'acide 4,9-éthano-2-[2-(3-pyrrolidinylpropyl)oxyphényl]acétyl-9-phényl-2,3,3a,4,9,9a-hexahydro-1H-benzo[f]isoindole-3a-acétique-(3aRS, 4SR, 9SR, 9aRS) chauffé au reflux pendant 4 heures dans 55 cm3 de soude 1N et 55 cm3 d'éthanol, on obtient, après concentration à sec sous pression réduite, un résidu qui est repris par 10 cm3 d'eau glacée et 5 cm3 d'acide chlorhydrique 1N. Le chlorhydrate ainsi formé est extrait par 3 fois 50 cm3 de dichlorométhane. Les phases organiques réunies sont lavées par 2 fois 5 cm3 d'eau glacée, séchées sur sulfate de magnésium et concentrées à sec sous pression réduite. Par recristallisation dans un mélange dichlorométhane-éther éthylique (1-1 en volumes), on obtient 1,24 g (75 %) de chlorhydrate d'acide 4,9-éthano-2-[2-(3-pyrrolidinylpropyl)oxyphényl]acétyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 135°C
- spectre de masse (D.C.I. / NH₃) M/Z = 565 (MH⁺).

### EXEMPLE 33

A partir de 1,67 g (5 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 1,08 g (6 mmoles) de l'acide (R)-2-(2-méthoxyphényl)propanoïque, qui peut être obtenu selon le brevet européen EP-0 430 771, de 1,15 g (6 mmoles) de chlorhydrate de 1-éthyl-3-(3-diméthylamino-propyl)-carbodiimide et de 68 mg (0,5 mmole) d'hydroxybenzotriazole en solution dans 20 cm3 de dichlorométhane, on obtient après 18 heures d'agitation à une température voisine de 20°C, un mélange de diastéréoisomères qui sont séparés par chromatographie liquide haute pression (9 bars) sur gel de silice 12 µm., en éluant avec un mélange cyclohexane-acétate d'éthyle (8-2 en volumes). On obtient ainsi successivement :
670 mg (27 %) du diastéréoisomère A de l'ester méthylique de l'acide 4,9-éthano-2-(R)-2-(2-méthoxyphényl)propanoyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 82°C
- pouvoir rotatoire [α]₃₆₅²⁰ = -20,3 ± 0,6 (c = 0,5 ; dichlorométhane)
- spectre de masse (E.I.) M/Z = 495 (M⁺).

690 mg (30 %) du diastéréoisomère B de l'ester méthylique de l'acide 4,9-éthano-2-(R)-2-(2-méthoxyphényl)propanoyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 162°C
- pouvoir rotatoire [α]₃₆₅²⁰ = -480,5 ±3,9 (c = 0,5 ; dichlorométhane).

On opère comme à l'exemple 2 à partir de 570 mg (1,15 mmoles) du diastéréoisomère A de l'ester de méthyle de l'acide 4,9-éthano-2-(R)-2-(2-méthoxyphényl)propanoyl-9-phényl-2,3,3a,4,9,9a-hexahydro-1H-benzo[f]isoindole-3a-carboxylique-(3aRS, 4SR, 9SR, 9aRS) en chauffant au reflux pendant 18 heures dans 14,4 cm3 de soude 0,1N et 14,4 cm3 d'éthanol. Après cristallisation dans l'éther isopropylique, on obtient 410 mg (76 %) du diastéréoisomère A de l'acide 4,9-éthano-2-(R)-2-(2-méthoxyphényl)propanoyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 231°C
- pouvoir rotatoire [α]₃₆₅²⁰ = -28,4 ± 0,7 (c= 0,5 ; dichlorométhane)
- spectre de masse (D.I.C : NH₃) M/Z = 482 (MH⁺)

### EXEMPLE 34

A partir de 1,24 g (3,7 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 800 mg (4,44 mmoles) d'acide (S)-2-(2-méthoxyphényl) propanoïque, qui peut être obtenu selon le brevet européen EP-0 430 771, de 850 mg (4,44 mmoles) de chlorhydrate de 1-éthyl-3-(3-diméthylamino-propyl)-carbodiimide et de 50 mg (0,37 mmole) d'hydroxybenzotriazole en solution dans 20 cm3 de dichlorométhane, on obtient, après 18 heures d'agitation à une température voisine de 20°C, les diastéréoisomères qui sont séparés par chromatographie liquide haute pression (15 bars) sur gel de silice 12 µm. en éluant avec un mélange cyclohexane-acétate d'éthyle (8-2 en volumes). On obtient ainsi successivement :
880 mg (48 %) du diastéréoisomère A de l'ester méthylique de l'acide 4,9-éthano-2-(S)-2-(2-méthoxyphényl)propionyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 78-80°C
- pouvoir rotatoire [α]₃₆₅²⁰ = +19,7 ± 0,7 (c = 0,5 ; dichlorométhane)
- spectre de masse (D.I.C. : NH₃) M/Z = 496 (MH⁺).

860 mg (47 %) du diastéréoisomère B de l'ester méthylique de l'acide 4,9-éthano-2-(S)-2-(2-méthoxyphényl)propionyl-9-phényl-2,3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 160-162°C
- pouvoir rotatoire [α]₃₆₅²⁰ = +478,3 ±4,1 (c = 0,5 ; dichlorométhane).

A partir de 720 mg (1,45 mmole) du diastéréoisomère B de l'ester méthylique de l'acide 4,9-éthano-2-(S)-2-(2-méthoxyphényl)propionyl-9-phényl-2,3,3a,4,9,9a-hexahydro-1H-benzo[f]isoindole-3a-carboxylique-(3aRS, 4SR, 9SR, 9aRS) chauffés au reflux pendant 18 heures dans 18 cm3 de soude 0,1N et 18 cm3 d'éthanol, on obtient après cristallisation dans l'éther isopropylique, 510 mg (76 %) du diastéréoisomère B de l'acide 4,9-éthano-2-(S)-2-(2-méthoxyphényl) propionyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 160°C
- pouvoir rotatoire [α]₃₆₅²⁰ = +448 ± 4,5 (c = 0,5 ; dichlorométhane)
- spectre de masse (D.I.C. : NH₃) M/Z = 482 (MH⁺).

### EXEMPLE 35

A partir de 920 mg (2,8 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 854 mg (3,36 mmoles) d'acide 5-bromo-indole-3-acétique, de 644 mg (3,36 mmoles) de chlorhydrate de 1-éthyl-3-(3-diméthylamino-propyl)-carbodiimide et de 40 mg (0,28 mmole) d'hydroxybenzotriazole en solution dans 100 cm3 de dichlorométhane, on obtient, après 18 heures d'agitation à une température voisine de 20°C, des cristaux qui sont séparés par filtration et lavés 2 fois par 2 cm3 de dichlorométhane glacé. On obtient ainsi 1,13 g (71 %) d'ester méthylique de l'acide 4,9-éthano-2-(5-bromo-3-indolyl)acétyl-9-phényl-2,3,3a,4,9, 9a-hexahydro-1H-benzo[f]-isoindole-3a-carboxylique-(3aRS, 4SR, 9SR, 9aRS) sous forme d'une poudre blanche dont les caractéristiques sont les suivantes :
- point de fusion = 230°C
- spectre de masse (E.I.) M/Z = 569(M⁺).

A partir de 600 mg (1,05 mmoles) d'ester méthylique de l'acide 4,9-éthano-(5-bromo-3-indolyl)acétyl-9-phényl-2,3,3a,4,9,9a-hexahydro-1H-benzo[f]isoindole-3a-carboxylique-(3aRS, 4SR, 9SR, 9aRS) chauffés au reflux pendant 18 heures dans 13,2 cm3 de soude 0,1N et 13,2 cm3 d'éthanol, on obtient, après recristallisation dans l'acétate d'éthyle, 150 mg (26 %) d'acide 4,9-éthano-2-(5-bromo-3-indolyl) acétyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 262°C
- spectre de masse (E.I.) M/Z = 555 (M⁺).

### EXEMPLE 36

A partir de 500 mg (1,5 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 328 mg (1,65 mmoles) d'acide (2,3-diméthoxyphényl)acétique, préparé selon J. Org. Chem., 53,548 (1949), de 320 mg (1,65 mmoles) de chlorhydrate de 1-éthyl-3-(3-diméthylamino-propyl)-carbodiimide et de 20 mg (0,15 mmole) d'hydroxybenzotriazole en solution dans 20 cm3 de dichlorométhane, on obtient, après 18 heures d'agitation à une température voisine de 20°C et purification par flash-chromatographie sur gel de silice (70-230 mesh), en éluant avec un mélange cyclohexane-acétate d'éthyle (4-6 en volumes), 640 mg (83 %) d'ester méthylique de l'acide 4,9-éthano-2-(2,3-diméthoxyphényl)acétyl-9-phényl-2,3,3a,4,9,9a-hexahydro-1H-benzo[f]-isoindole-3a-carboxylique-(3aRS, 4SR, 9SR, 9aRS), sous forme d'une poudre blanche dont les caractéristiques sont les suivantes :
- point de fusion = 196-197°C
- spectre de masse (E.I.) M/Z = 511 (M⁺).

A partir de 320 mg (0,625 mmoles) d'ester méthylique de l'acide 4,9-éthano-2-(2,3-diméthoxyphényl)acétyl-9-phényl-2,3,3a,4,9,9a-hexahydro-1H-benzo[f]isoindole-3a-carboxylique-(3aRS, 4SR, 9SR, 9aRS) chauffés au reflux pendant 6 heures dans 12,5 cm3 de soude 1N et 12,5 cm3 d'éthanol, on obtient, après cristallisation dans l'éther isopropylique, 150 mg (50 %) d'acide 4,9-éthano-2-(2,3-diméthoxy-phényl)acétyl-9-phényl-2,3,3a,4,9,9a-hexahydro-1H-benzo[f] isoindole-3a-carboxylique-(3aRS, 4SR, 9SR, 9aRS) sous forme de cristaux blancs dont les caractéristiques sont les suivantes :
- point de fusion = 230°C
- spectre de masse (E.I.) M/Z = 497 (M⁺).

### EXEMPLE 37

A une suspension, refroidie à 0°C, de 3,2 g (19 mmoles) d'acide (2-méthoxyphényl)acétique et de 0,9 g (30 mmoles) de p.formaldéhyde, on ajoute, très lentement en maintenant la température inférieure à 5°C, 40 cm3 d'acide chlorhydrique concentré. On laisse la température remonter au voisinage de 20°C et on agite pendant 18 heures. Le mélange réactionnel est versé sur 200 g de glace et est extrait 3 fois avec 200 cm3 d'acétate d'éthyle. Les phases organiques réunies sont lavées à l'eau, séchées sur sulfate de magnésium et concentrées à sec sous pression réduite. Après purification par flash-chromatographie sur gel de silice (70-230 mesh), en éluant avec un mélange cyclohexane-acétate d'éthyle, on obtient 2,5 g (61 %) d'acide (2-méthoxy-5-chlorométhyl-phényl)acétique sous forme d'une poudre jaune-pâle dont les caractéristiques sont les suivantes :
- point de fusion = 75-78°C

A partir de 2 g (6 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 1,54 g (7,2 mmoles) d'acide (2-méthoxy-5-chlorométhyl-phényl) acétique, de 1,38 g (7,2 mmoles) de chlorhydrate de 1-éthyl-3-(3-diméthylamino-propyl)-carbodiimide et de 81 mg (0,6 mmole) d'hydroxybenzotriazole en solution dans 20 cm3 de dichlorométhane, on obtient, après 18 heures d'agitation à une température voisine de 20°C et purification par flash-chromatographie sur gel de silice (70-230 mesh), en éluant avec un mélange cyclohexane-acétate d'éthyle (3-7 en volumes), 2,92 g (92 %) d'ester méthylique de l'acide 4,9-éthano-2-(2-méthoxy-5-chlorométhyl-phényl)acétyl-9-phényl-2,3,3a,4,9,9a-hexahydro-1H-benzo[f]-isoindole-3a-carboxylique-(3aRS, 4SR, 9SR, 9aRS), qui contient un peu de dérivé 5-hydroxyméthylé.

A une solution de 2,9 g (∼5,4 mmoles) d'ester méthylique de l'acide 4,9-éthano-2-(2-méthoxy-5-chlorométhyl-phényl)acétyl-9-phényl-2,3,3a,4,9,9a-hexahydro-1H-benzo[f]isoindole-3a-carboxylique-(3aRS, 4SR, 9SR, 9aRS) obtenu précédemment, dans 25 cm3 de diméthylformamide et 25 cm3 de dioxane, on ajoute 960 mg (6 mmoles) d'éthylxanthate de potassium, puis on agite pendant 24 heures à une temperature voisine de 20°C. Après concentration à sec sous pression réduite, le résidu est versé dans 100 cm3 d'eau glacée. Le précipité beige-clair formé est essoré, puis redissous dans 100 cm3 d'acétate d'éthyle. La phase organique est lavèe à l'eau jusquà ce que le lavage aqueux soit parfaitement limpide. Après séchage sur sulfate de magnésium et concentration à sec sous pression réduite, on obtient 2,73 g (96 %) d'une poudre contenant essentiellement l'ester méthylique de l'acide 4,9-éthano-2-(2-méthoxy-5-mercaptométhyl-phény])acétyl-9-phényl-2,3,3a,4,9,9a-hexahydro-1H-benzo[f]isoindole-3a-carboxylique-(3aRS, 4SR, 9SR, 9aRS) sous forme de disulfure.

A partir de 2,72 g (2,6 mmoles) de disulfure de l'ester méthylique de l'acide 4,9-éthano-2-(2-méthoxy-5-mercaptométhyl-phényl)acétyl-9-phényl-2,3,3a,4,9,9a-hexahydro-1H-benzo[f]isoindole-3a-carboxylique-(3aRS, 4SR, 9SR, 9aRS) obtenu précédemment, chauffés au reflux pendant 12 heures dans 5,2 cm3 de soude 1N et 25 cm3 d'éthanol, on obtient après purification par flash-chromatographie sur gel de silice (70-230 mesh), en éluant avec un mélange dichlorométhane-acétate d'éthyle (1-1 en volumes), puis chromatographie sur plaque préparative de silice, en éluant avec un mélange dichlorométhane-acétate d'éthyle-acide acétique (19-80-1 en volumes), 180 mg (7 %) de disulfure de l'acide 4,9-éthano-2-(2-méthoxy-5-mercaptométhyl-phényl)acétyl-9-phényl-2,3,3a,4,9,9a-hexahydro-1H-benzo[f] isoindole-3a-carboxylique-(3aRS, 4SR, 9SR, 9aRS) sous forme d'une poudre blanche dont les caractéristiques sont les suivantes :
- point de fusion = 140°C
- spectre infra-rouge (en comprimé avec KBr) : principales bandes d'absorption caractéristiques exprimées en cm⁻¹ à 3065 et 3022 (n CH aromatiques) ; 2952 et 2874 (nₐ et nₐₛ CH₂); 2387 (n CH des OCH₃) ; 3000-2300 (n OH de l'acide) ; 1732 (n C=O de l'acide) ; 1646 et 1610 (n C=O de l'amide) ; 1610, 1503, 1457 et 1444 (vibrations des noyaux aromatiques) ; 1254 (nₐₛ C-O de l'éther) ; 1031 (nₛ C-O de l'éther) ; 788 (g CH du phényle trisubstitué) ; 754 (g CH du phényle disubsitué) ; 701 (g CH du phényle monosubstitué).

### EXEMPLE 38

A une solution de 25 g (0,15 mole) d'acide 2-méthoxyphénylacétique dans de l'éthanol absolu, sont ajoutés lentement 13,5 cm3 (0,18 mole) de chlorure de thionyle. Le mélange est agité pendant 3 heures au reflux. Après concentration à sec, on obtient 30 g (100 %) d'ester éthylique de l'acide 2-méthoxyphénylacétique sous forme d'un liquide dont les caractéristiques sont les suivantes :
- spectre de masse (E.I.) M/Z = 180 (M⁺).

24 g (0,12 mole) d'ester éthylique de l'acide 2-méthoxyphénylacétique, 5,2 g (0,17 mole) de paraformaldéhyde, 0,68 g (1,84 mmoles) de iodure de tetrabutyl-ammonium et 28,9 g (0,21 mole) de carbonate de potassium sont chauffés au reflux dans le toluène pendant 20 heures. Après lavage à l'eau distillée, le solvant est éliminé par concentration à sec. On obtient 19 g (90 %) de l'ester éthylique de l'acide 2-(2-méthoxyphényl)-acrylique sous forme d'un liquide dont les caractéristiques sont les suivantes :
- spectre de masse (E.I.) M/Z = 192 (M⁺).

A partir de 6 g (29 mmoles) d'ester éthylique de l'acide 2-(2-méthoxy-phényl)-acrylique chauffés au reflux dans 58 cm3 (58 mmoles) d'hydroxyde de sodium 1N pendant 4 heures, on obtient, après acidification du mélange réactionnel et filtration du précipité obtenu, 3,4 g (66 %) d'acide 2-(2-méthoxyphényl)-acrylique dont les caractéristiques sont les suivantes :
- point de fusion = 144°C
- spectre de masse (E.I.) M/Z = 178 (M⁺).

A partir de 4,1 g (12,3 mmoles) d'ester méthylique de l'acide 4,9-éthano-9-phényl-2,3,3a,4,9,9a-hexahydro-1H-benzo[f]isoindole-3a-carboxylique-(3aRS 4SR, 9SR, 9aRS), de 2 g (11,2 mmoles) d'acide 2-(2-méthoxyphényl)-acrylique, de 2,36 g (12,3 mmoles) de chlorhydrate de 1-éthyl-3-(3-diméthylamino-propyl)-carbodiimide et de 0,17 g (1,12 mmoles) d'hydroxybenzotriazole dans le dichlorométhane agités pendant 24 heures à température ambiante, on obtient, après purification par flash-chromatographie sur gel de silice (70-230 mesh) en éluant avec un mélange cyclohexane-acétate d'éthyle (60-40 en volumes), 3,1 g (56 %) d'ester méthylique de l'acide 4,9-éthano-2-[2-(2-méthoxyphényl)prop-2-én-1-oyl]-9-phényl-2,3,3a,4,9,9a-hexahydro-1H-benzo[f]isoindole-3a-carboxylique-(3aRS, 4SR, 9SR, 9aRS) sous forme de meringue dont les caractéristiques sont les suivantes :
- point de fusion = 80 - 85°C
- spectre de masse (D.I.C. : NH₃) M/Z = 494 (MH⁺).

A partir de 3 g (6,08 mmoles) d'ester méthylique de l'acide 4,9-éthano-2-[2-(2-méthoxyphényl)prop-2-én-1-oyl]-9-phényl-2,3,3a,4,9,9a-hexahydro-1H-benzo[f] isoindole-3a-carboxylique-(3aRS, 4SR, 9SR, 9aRS) chauffés au reflux dans 7,5 cm3 (7,30 mmoles) d'hydroxyde de sodium 1N et de l'éthanol, on obtient, après acidification du mélange réactionnel avec de l'acide chlorhydrique 1N, une huile qui relargue et qui est extraite avec de l'acétate d'éthyle. La phase organique est lavée avec de l'eau distillée, séchée puis concentrée à sec sous pression réduite. On obtient ainsi 2,5 g (86 %) d'acide 4,9-éthano-2-[2-(2-méthoxyphényl)prop-2-én-1-oyl]-9-phényl-2,3,3a,4,9,9a-hexahydro-1H-benzo[f]isoindole-3a-carboxylique-(3aRS, 4SR, 9SR, 9aRS) dont les caractéristiques sont les suivantes :
- point de fusion = 102-108°C
- spectre de R.M.N. ¹H (250 MHz, (CD₃)₂SO d6, à une température de 393 K, δ en ppm) : 1,41 - 1,66 et de 1,95 à 2,25 (3 mts, respectivement 1H - 1H et 2H : CH₂CH₂) ; de 3,25 à 3,65 (mt, 3H : CH₂ en 1 et H en 9a) ; 3,43 (mt, 1H : H en 4) ; 3,60 et 4,05 (respectivement d et d large, J = 12,5 Hz, 1H chacun : CH₂ en 3) ; 3,72 (s, 3H : OCH₃) ; 5,53 et 5,66 (2 s larges, 1H chacun : =CH₂) ; 6,42 (d large, J = 7,5 Hz, 1H : H en 8) ; 6,90 (t large, J = 7,5 Hz, 1H : H aromatique en para du OCH₃) ; de 7,00 à 7,10 (mt, 2H : H aromatique en ortho du OCH₃ et H en 7) ; de 7,10 à 7,60 (mt, 9H : -H aromatiques en méta du OCH₃ - H en 5 - H en 6 et H aromatiques du phényle en 9).

### EXEMPLE 39

5,2 g (10,84 mmoles) d'acide 4,9-éthano-2-[2-(2-méthoxyphényl)prop-2-èn-1-oyl]-9-phényl-1,2,3,3a,9,9a-hexahydro-1H-benzo[f]isoindole-3a-carboxylique- (3aRS, 4SR, 9SR, 9aRS) sont dédoublés sur une colonne de silice chirale, porteuse de greffons (3,5-dinitrobenzyl)phénylalanine, en éluant avec un mélange dichlorométhane-isopropanol-n.heptane (85-10-5 en volumes). En recueillant la deuxième fraction éluée (temps de rétention = 21,8 minutes), on obtient 2,25 g d'acide 4,9-éthano-2-[2-(2-méthoxyphényl)prop-2-èn-1-oyl]-9-phényl-1,2,3,3a,9,9a-hexahydro-1H-benzo[f]isoindole-3a-carboxylique-(3aS, 4R, 9R, 9aS), énantiomère dextrogyre sous forme de cristaux blancs dont les caractéristiques sont les suivantes :
- point de fusion = 155°C
- pouvoir rotatoire [α]_{D}²⁰ = ⁺70,1 ± 1,3 (c = 0,5 ; dichlorométhane)
- spectre de masse (D.I.C. : NH₃) M/Z = 480 (MH⁺).

### EXEMPLE 40

A une solution de 10 g (0,04 mole) d'ester benzylique de l'acide (2-hydroxy phényl)acétique dans du tétrahydrofurane, on ajoute 8,25 cm3 (0,04 mole) de diisopropylazodicarboxylate, 10,82 g (0,04 mole) de triphénylphosphine et 3,1 cm3 (0,04 mole) d'isopropanol. Le mélange est chauffé au reflux pendant 3 heures puis lavé avec de l'eau distillée. La phase organique est séchée sur sulfate de magnésium. Après filtration le solvant est évaporé sous pression réduite. Le residu obtenu est chromatographié sur gel de silice en éluant avec un mélange cyclohexane-acétate d'éthyle (99-01 en volumes). On obtient 4,6 g (40 %) d'ester benzylique de l'acide (2-isopropoxyphényl)acétique sous forme d'un liquide dont les caractéristiques sont les suivantes :
- spectre de masse (E.I.) M/Z = 284 (M⁺).

On chauffe au reflux 2,8 g (9,86 mmoles) d'ester benzylique de l'acide (2-isopropoxyphényl)acétique dans 100 cm3 (0,1 mole) d'une solution d'hydroxyde de sodium 1N pendant 15 heures. Après acidification, le mélange réactionnel est extrait avec de I'acétate d'éthyle, séché sur sulfate de magnésium, puis filtré. Après évaporation du solvant sous pression réduite, on obtient 1,76 g (92 %) d'acide (2-isopropoxyphényl)-acétique sous forme d'une huile dont les caractéristiques sont les suivantes :
- spectre de masse (E.I.) M/Z = 194 (M⁺).

A partir de 1,5 g (4,5 mmoles) d'ester méthylique de l'acide 4,9-éthano-9-phényl-2,3,3a,4,9,9a-hexahydro-1H-benzo[f]-isoindole-3a-carboxylique-(3aRS, 4SR, 9SR, 9aRS) et de 1 g (5,4 mmoles) d' acide (2-isopropoxyphényl) acétique dans le dichlorométhane, on obtient 0,97 g (42 %) d'ester méthylique de l'acide 4,9-éthano-2-(2-isopropyloxyphényl)acétyl-9-phényl-2,3,3a,4,9,9a-hexahydro-1H-benzo[f]isoindole-3a-carboxylique-(3aRS, 4SR, 9SR, 9aRS) sous forme d'un solide blanc dont les caractéristiques sont les suivantes :
- point de fusion = 135-136°C
- spectre de masse (D.C.I. : NH₃) M/Z = 510 (MH⁺).

A partir de 970 mg (1,9 mmoles) d'ester méthylique de l'acide 4,9-éthano-2-(2-isopropyloxyphényl)acétyl-9-phényl-2,3,3a,4,9,9a-hexahydro-1H-benzo[f] isoindole-3a-carboxylique-(3aRS, 4SR, 9SR, 9aRS) dans 3,8 cm3 (3,8 mmoles) d'une solution d'hydroxyde de sodium 1N, on obtient 891 mg (94 %) d'acide 4,9-éthano-2-(2-isopropyloxyphényl)acétyl-9-phényl-2,3,3a,4,9,9a-hexahydro-1H-benzo[f]isoindole-3a-carboxylique-(3aRS 4SR, 9SR, 9aRS) dont les caractéristiques sont les suivantes :
- point de fusion = 260° C
- spectre de masse (L.S.I.M.S.) M/Z = 496 (MH⁺).

### EXEMPLE 41

A une suspension de 9,4 g (42,7 mmoles) de iodure de triméthyl-ulfoxonium dans le diméthylsulfoxyde, on ajoute par fraction et sous atmosphère d'argon, 1,22 g (42,7 mmoles) d'hydrure de sodium. On ajoute alors une solution de 8 g (38,8 mmoles) d'ester éthylique de l'acide 2-(2-méthoxyphényl)-acrylique dans le diméthylsulfoxyde. Le mélange réactionnel est agité à une temperature voisine de 20°C pendant 2 heures 30 minutes. Après hydrolyse avec de l'eau distillée, extraction avec de l'acétate d'éthyle, séchage sur sulfate de magnésium et évaporation du solvant sous pression réduite, le résidu est chromatographié sur gel de silice (70-230 mesh) en éluant avec un mélange cyclohexane-acétate d'éthyle (95-5 en volumes). On obtient ainsi 4,8 g (56 %) d'ester éthylique de l'acide 1-(2-méthoxyphényl)-cyclopropane-1-carboxylique sous forme d'une huile dont les caractéristiques sont les suivantes :
- spectre de masse (D.I.C. : NH₃) M/Z = 221 (MH⁺).

On chauffe au reflux 4,8 g (21,8 mmoles) d'ester éthylique de l'acide 1-(2-méthoxyphényl)-cyclopropane-1-carboxylique dans 33 cm3 (32,7 mmoles) d'une solution d'hydroxyde de sodium 1N pendant 4 heures. Après acidification du mélange réactionnel et filtration du précipité obtenu, on obtient 3,2 g (76 %) d'acide 1-(2-méthoxyphényl)-cyclopropane-1-carboxylique dont les caractéristiques sont les suivantes :
- point de fusion = 135°C
- spectre de masse (E.I.) M/Z = 192 (M⁺).

A partir de 2,6 g (7,8 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS) et 1,5 g (7,8 mmoles) d'acide 1-(2-méthoxyphényl)-cyclopropane-1-carboxylique dans le dichlorométhane, on obtient 2,5 g (63 %) d'ester méthylique de l'acide 4,9-éthano-2-[1-(2-méthoxyphényl)-cyclopropan-1-oyl]-9-phényl-2,3,3a,4,9, 9a-hexahydro-1H-benzo[f]isoindole-3a-carboxylique-(3aRS, 4SR, 9SR, 9aRS) sous forme d'un solide blanc dont les caractéristiques sont les suivantes :
- point de fusion = 162° C
- spectre de masse (E.I.) M/Z = 507 (M⁺).

A partir de 1 g (1,9 mmoles) d'ester méthylique de l'acide 4,9-éthano-2-[1-(2-méthoxyphényl)-cyclopropan-1-oyl]-9-phényl-2,3,3a,4,9,9a-hexahydro-1H-benzo [f]isoindole-3a-carboxylique-(3aRS, 4SR, 9SR, 9aRS) et 3,8 cm3 (3,8 mmoles) d'une solution d'hydroxyde de sodium 1N, on obtient 800 mg (82 %) d' acide 4,9-éthano-2-[1-(2-méthoxyphényl)-cyclopropan-1-oyl]-9-phényl-2,3,3a,4,9,9a-hexahydro-1H-benzo[f]isoindole-3a-carboxylique-(3aRS, 4SR, 9SR, 9aRS) sous forme d'un solide blanc dont les caractéristiques sont les suivantes :
- point de fusion = 146° C
- spectre de masse (D.C.I. : NH₃) M/Z = 494 (MH⁺).

### EXEMPLE 42

A une solution de 1 g (5,62 mmoles) d'acide 2-(2-méthoxyphényl)-acrylique, obtenu dans les conditions décrites dans l'exemple 38, dans le dichlorométhane, on fait barboter un courant d'ozone à -75°C pendant 3 heures 30 minutes. On ajoute 1,66 cm3 (22,5 mmoles) de diméthylsulfure et on poursuit l'agitation en laissant remonter la température au voisinage de 20°C. Après concentration à sec du mélange réactionnel, on obtient 0,7 g (69 %) d'acide (2-méthoxyphényl)-oxo-acétique sous forme de cristaux dont les caractéristiques sont les suivantes :
- spectre de masse (E.I.) M/Z = 180 (M⁺).

A partir de 1,26 g (3,78 mmoles) d'ester méthylique de l'acide 4,9-éthano-9-phényl-2,3,3a,4,9,9a-hexahydro-1H-benzo[f]-isoindole-3a-carboxylique-(3aRS, 4SR, 9SR, 9aRS) et de 620 mg (3,44 mmoles) d'acide (2-méthoxyphényl)-oxo-acétique dans le dichlorométhane, on obtient 1,3 g (76 %) d'ester méthylique de l'acide 4,9-éthano-2-[2-(2-méthoxyphényl)-2-oxo-acétyl)-9-phényl-2,3,3a,4,9,9a-hexahydro-1H-benzo[f]isoindole-3a-carboxylique-(3aRS, 4SR, 9SR, 9aRS) sous forme d'un solide blanc dont les caractéristiques sont les suivantes :
- point de fusion = 156° C
- spectre de masse (E.I.) M/Z = 495 (M⁺).

A partir de 500 mg (1,0 mmole) d'ester méthylique de l'acide 4,9-éthano-2-[2-(2-méthoxyphényl)-2oxo-acétyl]-9-phényl-2,3,3a,4,9,9a-hexahydro-1H-benzo[f] isoindole-3a-carboxylique-(3aRS, 4SR, 9SR, 9aRS) et de 1,6 cm3 (1,6 mmoles) d'une solution d'hydroxyde de sodium 1N, on obtient 168 mg (35 %) d'acide 4,9-éthano-2-[2-(2-méthoxyphényl)-2-oxo-acétyl]-9-phényl-2,3,3a,4,9,9a-hexahydro-1H-benzo[f]isoindole-3a-carboxylique-(3aRS, 4SR, 9SR, 9aRS) dont les caractéristiques sont les suivantes :
- point de fusion supérieur à 260°C
- spectre de masse (D.C.I. : NH₃) M/Z = 482 (MH⁺).

### EXEMPLE 43

A une température voisine de 20°C, on agite pendant 1 heure, 2 g (6 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS) avec 6 g (6 mmoles) de dit.butyldicarbonate en solution dans du dichlorométhane. Après évaporation du solvant, le résidu obtenu est cristallisé dans l'éther de pétrole. On obtient 2,3 g (87 %) d'ester méthylique de l'acide 4,9-éthano-9-phényl-2-(t.butoxycarbonyl)-2,3,3a,4,9,9a-hexahydro-1H-benzo[f]isoindole-3a-carboxylique-(3aRS, 4SR, 9SR, 9aRS) sous forme de cristaux dont les caractéristiques sont les suivantes :
- point de fusion = 142°C
- spectre de masse (D.C.I. : NH₃) M/Z = 434 (MH⁺).

On agite, à une température voisine de 20°C, 2,2 g (5 mmoles) d'ester méthylique de l'acide 4,9-éthano-9-phényl-2-(t.butoxycarbonyl)-2,3,3a,4,9,9a-hexahydro-1H-benzo[f]isoindole-3a-carboxylique-(3aRS, 4SR, 9SR, 9aRS) et 1,29 g d'iode (5 mmoles) dans du dichlorométhane. On ajoute, à 0°C, 2,29 g (5,25 mmoles) de bistrifluoroacétoxyiodobenzène et le mélange est maintenu à 0°C pendant 1 heure. Après hydrolyse à l'eau distillée, extraction avec de l'acétate d'éthyle, séchage sur sulfate de magnésium et évaporation du solvant sous pression réduite, le résidu obtenu est chromatographié sur gel de silice (70-230 mesh), en éluant avec un mélange cyclohexane-acétate d'éthyle (90-10 en volumes). On obtient ainsi 0,9 g (31,6 %) d'ester méthylique de l'acide 4,9-éthano-7-iodo-9-phényl-2-(t.butoxycarbonyl)-2,3,3a,4,9,9a-hexahydro-1H-benzo[f]isoindole-3a-carboxylique-(3aRS, 4SR, 9SR, 9aRS) sous forme de meringue blanche dont les caractéristiques sont les suivantes :
- spectre de R.M.N. ¹H (250 MHz, (CD₃)₂SO d6, à une température de 383 K, δ en ppm) : 1,47 (s, 9H : C(CH₃)₃) ; 1,45 - 1,78 - de 2,00 à 2,20 (3 mts, respectivement 1H - 1H et 2H : CH₂CH₂) ; de 3,20 à 3,40 (mt, 3H : CH₂ en 1 et H en 9a) ; 3,48 et 4,01 (2 d, J = 12,5 Hz, 1H chacun : CH₂ en 3) ; 3,52 (mt, 1H : H en 4) ; 3,60 (s, 3H : COOCH₃) ; 6,72 (s large, 1H : H en 8) ; 7,07 (d large, J = 7,5 Hz, 1H : H en 5) ; 7,45 (mt , 3H : H aromatiques en ortho et para du phényle en 9) ; 7,57 (mt, 3H : H en 6 et H aromatiques en méta du phényle en 9) et 0,8 g (28 %) d'ester méthylique de l'acide 4,9-éthano-6-iodo-9-phényl-2-(t.butoxycarbonyl)-2,3,3a,4,9,9a-hexahydro-1H-benzo[f]isoindole-3a-carboxylique-(3aRS, 4SR, 9SR, 9aRS) sous forme de meringue blanche dont les caractéristiques sont les suivantes :
- spectre de R.M.N. ¹H (400 MHz, (CD₃)₂SO d6, à une température de 393 K, δ en ppm) : 1,42 (s, 9H : C(CH₃)₃) ; 1,45 - 1,73 - de 2,00 à 2,20 (3 mts, respectivement 1H - 1H et 2H : CH₂CH₂) ; de 3,20 à 3,60 (mt, 5H : CH₂ en 1 - 1H du CH₂ en 3 - H en 4 et H en 9a) ; 3,58 (s, 3H : COOCH₃) ; 3,98 (d, J = 12,5 Hz, 1H : l'autre H du CH₂ en 3) ; 6,25 (d large, J = 7,5 Hz, 1H : H en 8) ; de 7,25 à 7,60 (mt , 7H : H en 5 - H en 7 et H aromatiques du phényle en 9).

On agite, à une température voisine de 20°C, pendant 24 heures une solution de 235 mg (0,42 mmole) d'ester méthylique de l'acide 4,9-éthano-7-iodo-9-phényl-2-(t.butoxycarbonyl)-2,3,3a,4,9,9a-hexahydro-1H-benzo[f]-isoindole-3a-carboxylique-(3aRS, 4SR, 9SR, 9aRS) avec un large excès de méthanol chlorhydrique 5N dans du méthanol. Après évaporation du solvant, on obtient 200 mg (100 %) de chlorhydrate de l'ester méthylique de l'acide 4,9-éthano-7-iodo-9-phényl-2,3,3a,4,9,9a-hexahydro-1H-benzo[f]isoindole-3a-carboxylique-(3aRS, 4SR, 9SR, 9aRS) sous forme d'un solide blanc dont les caractéristiques sont les suivantes :
- spectre de masse (D.I.C. : NH₃) M/Z = 560 (MH⁺).

A partir de 0,4 g (0,8 mmole) de chlorhydrate de l'ester méthylique de l'acide 4,9-éthano-7-iodo-9-phényl-2,3,3a,4,9,9a-hexahydro-1H-benzo[f]isoindole-3a-carboxylique-(3aRS, 4SR, 9SR, 9aRS) et de 0,16 g (0,96 mmole) d'acide (2-méthoxyphényl)acétique dans le dichlorométhane, on obtient 0,26 g (53 %) d'ester méthylique de l'acide 4,9-éthano-7-iodo-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) sous forme d'un solide blanc dont les caractéristiques sont les suivantes :
- spectre de masse (D.I.C. : NH₃) M/Z = 608 (MH⁺).

A partir de 260 mg (0,43 mmole) d'ester méthylique de l'acide 4,9-éthano-7-iodo-2-(2-méthoxyphényl)acétyl-9-phényl-2,3,3a,4,9,9a-hexahydro-1H-benzo[f] isoindole-3a-carboxylique-(3aRS, 4SR, 9SR, 9aRS) et de 0,42 cm3 (0,42 mmole) d'une solution d'hydroxyde de sodium 1N, on obtient 200 mg (80 %) d' acide 4,9-éthano-7-iodo-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) dont les caractéristiques sont les suivantes :
- point de fusion supérieur à 260°C
- spectre de R.M.N. ¹H (250 MHz, (CD₃)₂SO d6avec ajout de quelques gouttes de CD₃COOD d4, à une température de 383 K, δ en ppm) : 1,40 - 1,65 et de 1,80 à 2,20 (3 mts, respectivement 1H - 1H et 2H; CH₂CH₂) : de 3,30 à 3,65 (mt, 7H : CH₂ en 1 - COCH₂Ar - 1H du CH₂ en 3 - H en 4 et H en 9a) ; 3,72 (s, 3H : OCH₃) ; 4,18 (d, J = 12,5 Hz, 1H : l'autre H du CH₂ en 3) ; 6,72 (s large, 1H : H en 8) ; de 6,85 à 7,00 (mt, 2H : H aromatiques en ortho et para du OCH₃) ; 7,07 (d large, J = 7,5 Hz, 1H : H en 5) ; de 7,15 à 7,35 (mt, 2H : H aromatiques en méta du OCH₃) ; de 7,35 à 7,70 (mt, 6H : H en 6 et H aromatiques du phényl en 9).

### EXEMPLE 44

A une solution de 332 mg (0,69 mmole) d'énantiomère dextrogyre de l'acide 4,9-éthano-2-[2-(2-méthoxyphényl)prop-2-én-1-oyl]-9-phényl-2,3,3a,4,9,9a-hexahydro-1H-benzo[f]isoindole-3a-carboxylique-(3aRS, 4SR, 9SR, 9aRS) dans le dichlorométhane, on ajoute 66,5 mg (0,80 mmole) de chlorhydrate de méthoxylamine, 133 mg (0,69 mmole) de chlorhydrate de 1-éthyl-3-(3-diméthylamino-propyl)-carbodiimide, 10,6 mg (0,069 mmole) d'hydroxybenzotriazole et 0,1 cm3 (0,80 mmole) de triéthylamine. Le mélange réactionnel est agité à une température voisine de 20°C pendant 24 heures, lavé avec de l'acide chlorhydrique 0,1 N puis avec de l'eau distillée. Après séchage et concentration à sec de la phase organique, le produit brut obtenu est recristallisé dans un mélange de pentane et de méthanol. On obtient ainsi 63 mg (18 %) de N-méthoxy-4,9-éthano-2-[2-(2-méthoxyphényl)prop-2-én-1-oyl]-9-phényl-2,3,3a,4,9,9a-hexahydro-1H-benzo[f]isoindole-3a-carboxamide-(3aRS, 4SR, 9SR, 9aRS) sous forme d'un solide blanc dont les caractéristiques sont les suivantes :
- point de fusion = 160-162°C
- spectre de masse (D.I.C. : NH₃) M/Z = 509 (MH⁺).

### EXEMPLE 45

A une solution de 1 g (2,1 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) dans le dichlorométhane, on ajoute 0,41 g (2,6 mmoles) de chlorhydrate de O-benzylhydroxylamine, 0,48 g (2,6 mmoles) de chlorhydrate de 1-éthyl-3-(3-diméthylamino-propyl)-carbodiimide, 29 mg (0,214 mmole) d'hydroxybenzotriazole et 0,36 cm3 (2,6 mmoles) de triéthylamine. En opérant comme dans l'exemple 44, on obtient 0,83 g (68 %) de N-benzyloxy-4,9-éthano-2-(2-méthoxyphénylacétyl)-9-phényl-2,3,3a,4,9,9a-hexahydro-1H-benzo[f] isoindole-3a-carboxamide-(3aRS, 4SR, 9SR, 9aRS) sous forme d'un solide blanc dont les caractéristiques sont les suivantes :
- point de fusion = 188°C
- spectre de masse (D.I.C. : NH₃) M/Z = 573 (MH⁺).

### EXEMPLE 46

On agite une solution de 0,75 g (1,3 mmoles) de N-benzyloxy-4,9-éthano-2-(2-méthoxyphénylacétyl)-9-phényl-2,3,3a,4,9,9a-hexahydro-1H-benzo[f]isoindole-3a-carboxamide-(3aRS, 4SR, 9SR, 9aRS), obtenu dans les conditions de l'exemple 45, en présence de 75 mg de palladium sur charbon à 10 % (p/p) dans 40 cm3 d'éthanol avec de l'hydrogène à pression atmosphérique, à 20°C pendant 2 heures. Après filtration puis lavage du catalyseur à l'éthanol, le filtrat est concentré à sec sous pression réduite. Après purification par flash-chromatographie sur gel de silice (70-230 mesh), en éluant avec un mélange dichlorométhane-méthanol (90:10 en volumes) et après recristallisation dans un mélange éther isopropylique-éthanol, on obtient 0,34 g (54 %) d'acide 4,9-éthano-2-(2-méthoxyphénylacétyl)-9-phényl-2,3,3a,4,9,9a-hexahydro-1H-benzo[f]isoindole-3a-carbohydroxamique-(3aRS, 4SR, 9SR, 9aRS) sous forme d'un solide blanc dont les caractéristiques sont les suivantes :
- point de fusion = 234°C
- spectre de masse (L.S.I.M.S.) M/Z = 483 (MH⁺).

### EXEMPLE 47

3,7 g d'acide 4,9-éthano-2-[(2-méthoxyphényl)acétyl]-9-phényl-2,3,3a,4,9, 9a-hexahydro-1H-benzo-[f]isoindole-3a-carboxylique-(3aRS, 4SR, 9SR, 9aRS), obtenu dans les conditions de l'exemple 2, sont dédoublés par chromatographie sur une colonne de silice chirale, porteuse de greffons 3,5-dinitrobenzyl-phénylalanine, préparée dans les conditions analogues à celles décrites dans le brevet européen EP-0 625 153, en éluant avec un mélange dichlorométhane-2-propanol-n.heptane (30-5-65 en volumes). En concentrant les fractions contenant le second produit élué, on obtient 1,4 g d'acide 4,9-éthano-2-[(2-méthoxyphényl)acétyl]-9-phényl-2,3,3a(S), 4(R),9(R),9a(S)-hexahydro-1H-benzo[f]isoindole-3a-carboxylique dont les caractéristiques sont les suivantes :
- point de fusion = 115°C
- pouvoir rotatoire [α]_{D}²⁰ = + 55,1 (c = 1; méthanol)
- spectre de masse (DIC ; NH₃) : M/Z = 468 (MH⁺).

### EXEMPLE 48

A partir de 200 mg (0,43 mmole) d'acide 4,9-éthano-2-(2-méthoxyphényl)acétyl-9-phényl-2,3,3a(S),4(R),9(R),9a(S)-hexahydro-1H-benzo[f] isoindole-3a-carboxylique, de 62 mg (0,49 mmole) de chlorhydrate du glycinate de méthyle, de 0,068 cm3 (0,48 mmole) de triéthylamine, de 100 mg (0,52 mmole) de chlorhydrate de 1-éthyl-3-(3-diméthylamino-propyl)-carbodiimide et de 6 mg (0,04 mmole) d'hydroxybenzotriazole en solution dans 5 cm3 de dichlorométhane, on obtient, après 20 heures d'agitation à une température voisine de 20°C, décantation, lavage de la phase organique avec 2 fois 10 cm3 d'acide chlorhydrique, 3 fois 10 cm3 d'eau, séchage sur du sulfate de magnésium, puis concentration sous pression réduite, 200 mg (87 %) d'ester méthylique de la N-[4,9-éthano-2-(2-méthoxyphényl)acétyl-9-phényl-2,3,3a(S),4(R),9(R),9a(S)-hexahydro-1H-benzo[f] isoindol-3a-yl]carbonyl-glycine, sous forme d'une huile jaune.

200 mg (0,37 mmole) d'ester méthylique de la N-[4,9-éthano-2-(2-méthoxyphényl)acétyl-9-phényl-2,3,3a(S),4(R),9(R),9a(S)-hexahydro-1H-benzo[f] isoindol-3a-yl]carbonyl-glycine sont chauffés au reflux pendant 3 heures dans 8,6 cm3 d'eau, 18,6 cm3 d'éthanol et 0,22 cm3 de soude à 30 % (p/p). Après élimination de l'éthanol sous pression réduite, la solution est acidifiée par 0,3 cm3 d'acide chlorhydrique (d = 1,18), puis extraite avec 2 fois 30 cm3 de dichlorométhane. Après décantation et lavage avec 3 fois 10 cm3 d'eau, la phase organique est séchée sur du sulfate de magnésium, puis concentrée sous pression réduite. Le résidu obtenu est purifié par chromatographie sur plaque préparative de gel de silice en éluant avec un mélange dichlorométhane-méthanol (80-20 en volumes). On obtient ainsi 85 mg (45 %) de N-[4,9-éthano-2-(2-méthoxyphényl)acétyl-9-phényl-2,3,3a(S),4(R),9(R),9a(S)-hexahydro-1H-benzo[f]isoindol-3a-yl]carbonyl-glycine sous forme d'une poudre blanche dont les caractéristiques sont les suivantes :
- point de fusion = 150°C
- pouvoir rotatoire [α]_{D}²⁰ = + 90 (c = 1 ; méthanol)
- spectre de masse (DIC ; NH₃) : M/Z = 525 (MH⁺).

### EXEMPLE 49

A partir de 1,5 g (3,2 mmoles) d'acide 4,9-éthano-2-(2-méthoxyphényl) acétyl-9-phényl-2,3,3a,4,9,9a-hexahydro-1H-benzo[f]isoindole-3a-carboxylique (3aRS, 4SR, 9SR, 9aRS), de 0,7 g (3,85 mmoles) de chlorhydrate de l'ester tert-butylique de la N-méthyl glycine, de 0,5 cm3 (3,85 mmoles) de triéthylamine, de 0,74 g (3,85 mmoles) de chlorhydrate de 1-éthyl-3-(3-diméthylamino-propyl)-carbodiimide et de 0,04 g (0,3 mmole) d'hydroxybenzotriazole en solution dans 40 cm3 de dichlorométhane, on obtient, après 18 heures d'agitation à une température voisine de 20°C, décantation, lavage de la phase organique avec 2 fois 100 cm3 d'eau, séchage sur du sulfate de magnésium, puis concentration sous pression réduite, un résidu qui est purifié par flash-chromatographie sur gel de silice (70-230 mesh) en éluant avec un mélange dichlorométhane-méthanol (99-1 en volumes). On obtient ainsi 1 g (50 %) d'ester tert-butylique de la N-[4,9-éthano-2-(2-méthoxyphényl)acétyl-9-phényl-2,3,3a,4,9,9a-hexahydro-1H-benzo[f]isoindol-3a-yl] carbonyl-N-méthyl-glycine-(3aRS, 4SR, 9SR, 9aRS) sous forme d'une poudre blanche.

700 mg (1,18 mmoles) d'ester tert-butylique de la N-[4,9-éthano-2-(2-méthoxyphényl)acétyl-9-phényl-2,3,3a,4,9,9a-hexahydro-1H-benzo[f]isoindol-3a-yl] carbonyl-N-méthyl-glycine-(3aRS, 4SR, 9SR, 9aRS) sont chauffés au reflux pendant 4 heures dans 12 cm3 d'éthanol et 17,5 cm3 de soude 0,1N. Après élimination de l'éthanol sous pression réduite, la solution est acidifiée par l'acide chlorhydrique 1N, puis extraite avec 50 cm3 d'acétate d'éthyle. Après lavage par décantation avec 20 cm3 d'eau, la phase organique est séchée sur du sulfate de magnésium, puis concentrée sous pression réduite. Le résidu obtenu est trituré dans 20 cm3 d'éther de pétrole. On obtient ainsi 455 mg (72 %) de N-[4,9-éthano-2-(2-méthoxyphényl) acétyl-9-phényl-2,3,3a,4,9,9a-hexahydro-1H-benzo[f]isoindol-3a-yl]carbonyl-N-méthyl-glycine-(3aRS, 4SR, 9SR, 9aRS) sous forme d'une poudre blanche dont les caractéristiques sont les suivantes :
- point de fusion = 198°C
- spectre de masse (LSIMS) : M/Z = 539 (MH⁺).

### EXEMPLE 50

A partir de 880 mg (1,88 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 316 mg (2,26 mmoles) de chlorhydrate de 3-amino-propanoate de méthyle, de 0,32 cm3 (2,26 mmoles) de triéthylamine, de 440 mg (2,26 mmoles) de chlorhydrate de 1-éthyl-3-(3-diméthylamino-propyl)-carbodiimide et de 25 mg (0,188 mmole) d'hydroxybenzotriazole en solution dans 30 cm3 de dichlorométhane, on obtient, après 18 heures d'agitation à une température voisine de 20°C, lavage par décantation avec 2 fois 30 cm3 d'eau de la phase organique qui est séchée sur du sulfate de magnésium, puis concentrée sous pression réduite, un résidu solide qui est purifié par recristallisation dans l'acétate d'éthyle. On obtient 540 mg (50 %) de N-[4,9-éthano-2-(2-méthoxyphényl)acétyl-9-phényl-2,3,3a,4,9,9a-hexahydro-1H-benzo[f]isoindol-3a-yl]carbonyl-3-aminopropanoate-(3aRS, 4SR, 9SR, 9aRS) de méthyle sous forme d'une poudre blanche.

540 mg (0,98 mmole) de N-[4,9-éthano-2-(2-méthoxyphényl)acétyl-9-phényl-2,3,3a,4,9,9a-hexahydro-1H-benzo[f]isoindol-3a-yl]carbonyl-3-aminopropanoate-(3aRS, 4SR, 9SR, 9aRS) de méthyle sont chauffés au reflux pendant 6 heures dans 20 cm3 d'éthanol et 9,8 cm3 de soude 0,1N. Après élimination de l'éthanol sous pression réduite, la solution est acidifiée par l'acide chlorhydrique 1N, puis extraite avec 20 cm3 d'acétate d'éthyle. Après lavage par décantation avec 20 cm3 d'eau, la phase organique est séchée sur du sulfate de magnésium, puis concentrée sous pression réduite. Le solide obtenu est purifié par recristallisation dans l'éthanol. On obtient ainsi 230 mg (45 %) d'acide N-[4,9-éthano-2-(2-méthoxyphényl)acétyl-9-phényl-2,3,3a,4,9,9a-hexahydro-1H-benzo[f]isoindol-3a-yl] carbonyl-3-aminopropanoique-(3aRS, 4SR, 9SR, 9aRS) sous forme d'une poudre blanche dont les caractéristiques sont les suivantes :
- point de fusion = 150°C
- spectre de masse (DIC ; NH₃) : M/Z = 539 (MH⁺).

### EXEMPLE 51

A partir de 1,5 g (3,2 mmoles) d'acide 4,9-éthano-2-(2-méthoxyphényl) acétyl-9-phényl-2,3,3a,4,9,9a-hexahydro-1H-benzo[f]isoindole-3a-carboxylique-(3aRS, 4SR, 9SR, 9aRS), de 0,76 g (3,85 mmoles) de chlorhydrate de l'ester méthylique de la L-méthionine, de 0,54 ml (3,85 mmoles) de triéthylamine, de 0,74 g (3,85 mmoles) de chlorhydrate de 1-éthyl-3-(3-diméthylamino-propyl)-carbodiimide et de 0,043 g (0,32 mmole) d'hydroxybenzotriazole en solution dans 50 cm3 de dichlorométhane, on obtient, après 18 heures d'agitation à une température voisine de 20°C, lavage par décantation avec 2 fois 50 cm3 d'eau de la phase organique qui est séchée sur du sulfate de magnésium, puis concentrée sous pression réduite, un résidu qui est purifié par flash-chromatographie sur gel de silice (70-230 mesh) en éluant avec un mélange dichlorométhane-méthanol (99-1 en volumes). On obtient ainsi 0,86 g (45 %) d'ester méthylique de la N-[4,9-éthano-2-(2-méthoxyphényl)acétyl-9-phényl-2,3,3a,4,9,9a-hexahydro-1H-benzo[f]isoindol-3a-yl]carbonyl-L-méthionine-(3aRS, 4SR, 9SR, 9aRS) sous forme d'une poudre blanche dont les caractéristiques sont les suivantes :
- point de fusion = 192°C.

0,86 g (1,4 mmoles) d'ester méthylique de la N-[4,9-éthano-2-(2-méthoxyphényl)acétyl-9-phényl-2,3,3a,4,9,9a-hexahydro-1H-benzo[f]isoindol-3a-yl] carbonyl-L-méthionine(3aRS, 4SR, 9SR, 9aRS) sont chauffés au reflux pendant 3 heures dans 16 cm3 d'éthanol et 21,5 cm3 de soude 0,1N. Après élimination de l'éthanol sous pression réduite, la solution est acidifiée par l'acide chlorhydrique 1N, puis extraite avec 100 cm3 d'acétate d'éthyle. Après lavage par décantation avec 50 cm3 d'eau de la phase organique qui est séchée sur du sulfate de magnésium puis concentrée sous pression réduite, on obtient 0,54 g (60 %) de N-[4,9-éthano-2-(2-méthoxyphényl)acétyl-9-phényl-2,3,3a,4,9,9a-hexahydro-1H-benzo[f]isoindol-3a-yl] carbonyl-L-méthionine-(3aRS, 4SR, 9SR, 9aRS) sous forme d'une poudre blanche dont les caractéristiques sont les suivantes :
- point de fusion = 196°C
- spectre de masse (DIC ; NH₃) : M/Z = 599 (MH⁺).

### EXEMPLE 52

A partir de 1,5 g (3,2 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 1 g (3,8 mmoles) de chlorhydrate de l'ester méthylique de la S-benzyl-cystéine, de 0,53 cm3 (3,8 mmoles) de triéthylamine, de 0,73 g (3,8 mmoles) de chlorhydrate de 1-éthyl-3-(3-diméthylamino-propyl)-carbodiimide et de 0,043 g (0,32 mmole) d'hydroxybenzotriazole en solution dans 60 cm3 de dichlorométhane, on obtient, après 18 heures d'agitation à une température voisine de 20°C, lavage par décantation avec 2 fois 50 cm3 d'eau de la phase organique qui est séchée sur du sulfate de magnésium, puis concentrée sous pression réduite, un résidu qui est purifié par flash-chromatographie sur gel de silice (70-230 mesh) en éluant avec de l'acétate d'éthyle. On obtient ainsi 0,72 g (33 %) d'ester méthylique de la N-[4,9-éthano-2-(2-méthoxyphényl)acétyl-9-phényl-2,3,3a,4,9,9a-hexahydro-1H-benzo[f]isoindol-3a-yl]carbonyl-S-benzyl-L-cystéine-(3aRS, 4SR, 9SR, 9aRS) sous forme d'une meringue blanche.

0,5 g (0,74 mmole) d'ester méthylique de la N-[4,9-éthano-2-(2-méthoxyphényl)acétyl-9-phényl-2,3,3a,4,9,9a-hexahydro-1H-benzo[f]isoindol-3a-yl] carbonyl-S-benzyl-L-cystéine-(3aRS, 4SR, 9SR, 9aRS) sont chauffés au reflux pendant 3 heures dans 20 cm3 d'éthanol et 12,5 cm3 de soude 0,1N. Après élimination de l'éthanol sous pression réduite, la solution est acidifiée par l'acide chlorhydrique 1N, puis extraite avec 100 cm3 d'acétate d'éthyle. Après lavage par décantation avec 50 cm3 d'eau de la phase organique qui est séchée sur du sulfate de magnésium puis concentrée sous pression réduite, on obtient un résidu qui est purifié par flash-chromatographie sur gel de silice (70-230 mesh) en éluant avec un mélange dichlorométhane-méthanol-acide acétique (96-2-2 en volumes). Le produit solide obtenu est recristallisé dans l'acétonitrile. On obtient ainsi 0,24 g (50 %) de N-[4,9-éthano-2-(2-méthoxyphényl)acétyl-9-phényl-2,3,3a,4,9,9a-hexahydro-1H-benzo[f] isoindol-3a-yl]carbonyl-S-benzyl-L-cystéine-(3aRS, 4SR, 9SR, 9aRS) sous forme d'une poudre blanche dont les caractéristiques sont les suivantes :
- point de fusion = 190°C
- spectre de masse (DIC ; NH₃) : M/Z = 661 (MH⁺).

### EXEMPLE 53

A partir de 1,35 g (2,9 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 1,31 g (3,47 mmoles) d'ester méthylique de la S-triphénylméthyl-L-cystéine qui peut être obtenue selon Liebigs Ann Chem., 1563 (1984), de 0,66 g (3,47 mmoles) de chlorhydrate de 1-éthyl-3-(3-diméthylamino-propyl)-carbodiimide et de 0,039 g (0,29 mmole) d'hydroxybenzotriazole en solution dans 100 cm3 de dichlorométhane, on obtient, après 48 heures d'agitation à une température voisine de 20°C, lavage par décantation avec 2 fois 75 cm3 d'eau de la phase organique qui est séchée sur du sulfate de magnésium puis concentrée sous pression réduite, un résidu qui est purifié par flash-chromatographie sur gel de silice (70-230 mesh) en éluant avec un mélange cyclohexane-acétate d'éthyle (80-20 en volumes). On obtient ainsi 1,72 g (72 %) d'ester méthylique de la N-[4,9-éthano-2-(2-méthoxyphény])acétyl-9-phényl-2,3,3a,4,9,9a-hexahydro-1H-benzo[f]isoindol-3a-yl]carbonyl-S-triphénylméthyl-L-cystéine-(3aRS, 4SR, 9SR, 9aRS) sous forme d'un solide jaune pâle dont les caractéristiques sont les suivantes :
- spectre de masse (LSIMS) : M/Z = 827 (MH⁺).

1,66 g (2 mmoles) d'ester méthylique de la N-[4,9-éthano-2-(2-méthoxyphényl)acétyl-9-phényl-2,3,3a,4,9,9a-hexahydro-1H-benzo[f]isoindol-3a-yl] carbonyl-S-triphénylméthyl-L-cystéine-(3aRS, 4SR, 9SR, 9aRS) sont chauffés au reflux pendant 5 heures dans 50 cm3 de méthanol et 20 cm3 de soude 0,1N. Après élimination de l'éthanol sous pression réduite, la solution est acidifiée par l'acide chlorhydrique 1N, laissée sous agitation pendant 1 heure, puis extraite avec 50 cm3 d'acétate d'éthyle. Après lavage par décantation avec 2 fois 30 cm3 d'eau de la phase organique qui est séchée sur du sulfate de magnésium puis concentrée sous pression réduite, on obtient un résidu qui est lavé par de l'oxyde d'isopropyle puis purifié par chromatographie sur gel de Séphadex® en éluant avec du méthanol. On obtient ainsi 0,3 g (20 %) de N-[4,9-éthano-2-(2-méthoxyphényl)acétyl-9-phényl-2,3,3a,4,9,9a-hexahydro-1H-benzo[f]isoindol-3a-yl]carbonyl-L-cystéine-(3aRS, 4SR, 9SR, 9aRS) sous forme d'un solide jaune pâle dont les caractéristiques sont les suivantes :
- spectre de masse (LSIMS) : M/Z = 571 (MH⁺).

### EXEMPLE 54

A partir de 750 mg (1,6 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 130 mg (1,9 mmoles) de chlorhydrate de méthylamine, de 0,27 ml (1,9 mmoles) de triéthylamine, de 370 mg (1,9 mmoles) de chlorhydrate de 1-éthyl-3-(3-diméthylamino-propyl)-carbodiimide et de 21,5 mg (0,16 mmole) d'hydroxybenzotriazole en suspension dans 25 cm3 de dichlorométhane, on obtient, après 18 heures d'agitation à une température voisine de 20°C et addition de 520 mg (3,9 mmoles) de carbonate de potassium, une suspension qui est agitée pendant 24 heures à une température voisine de 20°C. Après lavage par décantation avec 2 fois 20 cm3 d'eau, la phase organique est séchée sur du sulfate de magnésium, puis concentrée sous pression réduite. Le résidu obtenu est purifié par flash-chromatographie sur gel de silice (70-230 mesh) en éluant avec un mélange dichlorométhane-méthanol (98-2 en volumes). On obtient ainsi 540 mg (70 %) de N-méthyl-4,9-éthano-2-(2-méthoxyphényl)acétyl-9-phényl-2,3,3a,4,9,9a-hexahydro-1H-benzo[f]isoindole-3a-carboxamide-(3aRS, 4SR, 9SR, 9aRS) sous forme d'une poudre blanche dont les caractéristiques sont les suivantes :
- point de fusion = 216°C
- spectre de masse (DIC ; NH₃) : M/Z = 481 (MH⁺).

### EXEMPLE 55

A partir de 500 mg (1,1 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 105 mg (1,3 mmoles) de chlorhydrate de diméthylamine, de 0,18 cm3 (1,3 mmoles) de triéthylamine, de 246 mg (1,3 mmoles) de chlorhydrate de 1-éthyl-3-(3-diméthylamino-propyl)-carbodiimide et de 14,4 mg (0,11 mmole) d'hydroxybenzotriazole en suspension dans 30 cm3 de dichlorométhane, on obtient, après 20 heures d'agitation à une température voisine de 20°C et lavage par décantation avec 2 fois 25 cm3 d'eau de la phase organique qui est séchée sur du sulfate de magnésium puis concentrée sous pression réduite, un résidu qui est purifié par flash-chromatographie sur gel de silice (70-230 mesh) en éluant avec un mélange dichlorométhane-méthanol (99-1 en volumes). On obtient ainsi 225 mg (40 %) de N,N-diméthyl-4,9-éthano-2-(2-méthoxyphényl)acétyl-9-phényl-2,3,3a,4,9, 9a-hexahydro-1H-benzo[f]isoindole-3a-carboxamide-(3aRS, 4SR, 9SR, 9aRS) sous forme d'une poudre blanche dont les caractéristiques sont les suivantes :
- point de fusion = 138°C
- spectre de masse (DIC ; NH₃) : M/Z = 495 (MH⁺).

### EXEMPLE 56

A partir de 467 mg (1 mmole) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 96 mg (1,15 mmoles) de chlorhydrate de O-méthyl-hydroxylamine, de 0,16 cm3 (1,15 mmoles) de triéthylamine, de 230 mg (1,2 mmoles) de chlorhydrate de 1-éthyl-3-(3-diméthylamino-propyl)-carbodiimide et de 13 mg (0,1 mmole) d'hydroxybenzotriazole en suspension dans 8,5 cm3 de dichlorométhane, on obtient, après 20 heures d'agitation à une température voisine de 20°C, lavage par décantation avec 2 fois 10 cm3 d'eau de la phase organique qui est séchée sur du sulfate de sodium puis concentrée sous pression réduite, un résidu qui est purifié par recristallisations successives dans l'éthanol, puis le toluène. On obtient ainsi 300 mg (60 %) de N-méthoxy-4,9-éthano-2-(2-méthoxyphényl)acétyl-9-phényl-2,3,3a,4,9,9a-hexahydro-1H-benzo-[f]isoindole-3a-carboxamide-(3aRS, 4SR, 9SR, 9aRS) sous forme d'une poudre blanche dont les caractéristiques sont les suivantes :
- point de fusion = 217°C
- spectre de masse (DIC ; NH₃) : M/Z = 497 (MH⁺).

### EXEMPLE 57

A partir de 4,2 g (10 mmoles) d'acide 4,9-éthano-2-benzyl-9-phényl-2,3,3a,4,9,9a-hexahydro-1H-benzo[f]isoindole-3a-carboxylique-(3aRS, 4SR, SR, 9aRS), de 1,41 g (11,5 mmole) de 1(R)-phényléthylamine, de 2,25 g (11,5 mmoles) de chlorhydrate de 1-éthyl-3-(3-diméthylamino-propyl)carbodiimide et de 0,13 g (1 mmole) d'hydroxybenzotriazole en solution dans 100 cm3 de dichlorométhane, on obtient, après 20 heures d'agitation à une température voisine de 20°C, lavage par décantation avec 2 fois 25 cm3 d'eau de la phase organique qui est séchée sur du sulfate de magnésium puis concentrée sous pression réduite, un résidu qui est purifié par recristallisation dans l'oxyde d'isopropyle. On obtient ainsi 4,5 g (90 %) de N-[(R)-1-phényléthyl]-4,9-éthano-2-benzyl-9-phényl-2,3,3a,4,9,9a-hexahydro-1H-benzo[f]isoindole-3a-carboxamide-(3aRS, 4SR, 9SR, 9aRS) sous forme d'une poudre blanche dont les caractéristiques sont les suivantes :
- point de fusion = 125°C
- spectre de masse (DIC ; NH₃) : M/Z = 513 (MH⁺).

A partir de 4,5 g de N-[(R)-1-phényléthyl]-4,9-éthano-2-benzyl-9-phényl-2,3,3a,4,9,9a-hexahydro-1H-benzo[f]isoindole-3a-carboxamide (3aRS, 4SR, 9SR, 9aRS), de 2,77 g de formiate d'ammonium, de 1,5 g de palladium sur charbon à 5 % (p/p), en suspension dans 75 cm3 de méthanol, on obtient, après chauffage au reflux pendant 3 heures, filtration du catalyseur après refroidissement et concentration du méthanol sous pression réduite, un résidu qui est mis en solution dans 100 cm3 de dichlorométhane. Après lavage par décantation avec 30 cm3 de soude 1N, puis avec 3 fois 30 cm3 d'eau, séchage sur sulfate de magnésium puis concentration sous pression réduite, on obtient 2,7 g (75 %) de N-[(R)-1-phényléthyl]-4,9-éthano-9-phényl-2,3,3a,4,9,9a-hexahydro-1H-benzo[f]isoindole-3a-carboxamide-(3aRS, 4SR, 9SR, 9aRS) sous forme d'une meringue.

A partir de 2,7 g (6,4 mmoles) de N-[(R)-1-phényléthyl]-4,9-éthano-9-phényl-2,3,3a,4,9,9a-hexahydro-1H-benzo[f]isoindole-3a-carboxamide-(3aRS, 4SR, 9SR, 9aRS), de 1,24 g (7,35 mmoles) d'acide (2-méthoxyphényl)acétique, de 1,47 g (7,66 mmoles) de chlorhydrate de 1-éthyl-3-(3-diméthylamino-propyl)-carbodiimide et de 0,086 g (0,64 mmole) d'hydroxybenzotriazole en solution dans 70 cm3 de dichlorométhane, on obtient, après 20 heures d'agitation à une température voisine de 20°C, lavage par décantation avec 2 fois 25 cm3 d'eau de la phase organique qui est séchée sur du sulfate de magnésium puis concentrée sous pression réduite, un résidu qui est purifié par flash-chromatographie sur gel de silice (70-230 mesh) en éluant avec un mélange dichlorométhane-méthanol (98-2 en volumes). Les diastéréoisomères sont séparés par chromatographie sur plaque préparative de gel de silice en éluant par un mélange chloroforme-acétate d'éthyle (60-40 en volumes). Après recristallisation dans l'éthanol, on obtient 1 g (33 %) de N-[(R)-1-phényléthyl]-4,9-éthano-2-(2-méthoxyphényl)acétyl-9-phényl-2,3,3a(S),4(R),9(R),9a(S))-hexahydro-1H-benzo[f]isoindole-3a-carboxamide sous forme d'une poudre blanche dont les caractéristiques sont les suivantes :
- point de fusion = 250°C
- pouvoir rotatoire [α]_{D}²⁰ = ⁺ 114 (c = 0,5 : chloroforme)
- spectre de masse (DIC ; NH₃) : M/Z = 571 (MH⁺).

### EXEMPLE 58

A partir de 750 mg (1,6 mmoles) d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 0,094 cm3 (1,93 mmoles) d'hydrate hydrazine, de 370 mg (1,93 mmoles) de chlorhydrate de 1-éthyl-3-(3-diméthylamino-propyl)-carbodiimide et de 22 mg (0,16 mmole) d'hydroxybenzotriazole en solution dans 40 cm3 de dichlorométhane, on obtient, après 20 heures d'agitation à une température voisine de 20°C, lavage par décantation avec 2 fois 25 cm3 d'eau de la phase organique qui est séchée sur du sulfate de magnésium puis concentrée sous pression réduite, un résidu qui est purifié par flash-chromatographie sur gel de silice (70-230 mesh) en éluant avec un mélange dichlorométhane-méthanol (95-5 en volumes). On obtient ainsi 385 mg (50 %) d'hydrazide de l'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 243°C
- spectre de R.M.N. ¹H (250 MHz, (CD₃)₂SO d6, à une température de 393 K, δ en ppm) : 1,37 - 1,63 et de1,85 à 2,15 (3 mts, respectivement 1H - 1H et 2H : CH₂CH₂) ; de 3,25 à 3,70 (mt, 6H : CH₂ en 1 - COCH₂Ar - 1H du CH₂ en 3 et H en 9a) ; 3,60 (mt, 1H : H en 4) ; 3,70 (mf, 3H : OCH₃) ; 4,00 (mf, 2H : NH₂) ; 4,22 (d, J = 12,5 Hz, 1H : l'autre H du CH₂ en 3) ; 6,39 (d large, J = 7,5 Hz, 1H : H en 8) ; 6,90 (t large, J = 7,5 Hz, 1H : H aromatique en para du OCH₃) ; 6,95 (d large, J = 7,5 Hz, 1H : H aromatique en ortho du OCH₃) ; 7,02 (dt, J =7,5 et 2 Hz, 1H : H en 7) ; de 7,05 à 7,30 (mt, 4H : H en 5 - H en 6 et H aromatiques en méta du OCH₃) ; de 7,30 à 7,55 (mt, 5H : H aromatiques du phényle en 9) ; 8,70 (mf, 1H : CONH).

### EXEMPLE 59

On prépare l'ester méthylique de l'acide 4,9-éthano-2-(2-méthoxyphényl) propionyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 1 sous forme de cristaux blancs dont les caractéristiques sont les suivantes :
- point de fusion = 171°C
- spectre de masse (DIC ; NH₃) : M/Z = 496 (MH⁺).

On prépare l'acide 4,9-éthano-2-(2-méthoxyphényl)propionyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 2 sous forme de cristaux blancs dont les caractéristiques sont les suivantes :
- point de fusion = 202°C
- spectre de R.M.N. ¹H (250 MHz, (CD₃)₂SO d6 avec ajout de quelques gouttes de CD₃COOD d4, à une température de 403 K, δ en ppm) : 1,39 - 1,66 - de 1,85 à 2,15 (3 mts, respectivement 1H - 1H et 2H : CH₂CH₂) ; 2,50 (mt, 2H : COCH₂) ; 2,85 (t, J=7,5 Hz, 2H : CH₂Ar) ; de 3,25 à 3,50 (mt, 3H : CH₂ en 1 et H en 9a) ; 3,48 (mt, 1H : H en 4) ; 3,58 et 4,10 (2 d, J = 12,5 Hz, 1H chacun : CH₂ en 3) ; 3,75 (s, 3H : OCH₃) ; 6,42 (d large, J = 7,5 Hz, 1H : H en 8) ; 6,85 (t large, J =7,5 Hz, 1H : H aromatique en para du OCH₃) ; 6,92 (d large, J = 7,5 Hz, 1H : H aromatique en ortho du OCH₃) ; 7,04 (dt, J = 7,5 et 2 Hz, 1H : H en 7) ; de 7,10 à 7,25 (mt, 4H : H en 5 - H en 6 et H aromatiques en méta du OCH₃) ; de 7,35 à 7,60 (mt, 5H : H aromatiques du phényle en 9).

### EXEMPLE 60

On prépare l'ester méthylique de l'acide 4,9-éthano-2-(2-hydroxyphényl) acétyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 1 sous forme de cristaux beiges dont les caractéristiques sont les suivantes :
- point de fusion = 205°C

On prépare l'acide 4,9-éthano-2-(2-hydroxyphényl)acétyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 2 sous forme de cristaux blancs dont les caractéristiques sont les suivantes :
- point de fusion = 151°C
- spectre de masse (DIC ; NH₃) : M/Z = 454 (MH⁺).

### EXEMPLE 61

On prépare l'ester méthylique de l'acide 4,9-éthano-2-(2-ctuorophényl)acétyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 1 sous forme de cristaux blancs dont les caractéristiques sont les suivantes :
- point de fusion = 147°C
- spectre de masse (DIC ; NH₃) : M/Z = 486 (MH⁺).

On prépare l'acide 4,9-éthano-2-(2-chlorophényl)acétyl-9-phényl-2,3,3a,4, 9,9a-hexahydro-1H-benzo[f]isoindole-3a-carboxylique-(3aRS, 4SR, 9SR, 9aRS) dans les conditions décrites dans l'exemple 2 sous forme de cristaux blancs dont les caractéristiques sont les suivantes :
- point de fusion = 223°C
- spectre de masse (DIC ; NH₃) : M/Z = 472 (MH⁺).

### EXEMPLE 62

On prépare l'ester méthylique de l'acide 4,9-éthano-2-(2-méthylphényl) acétyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 1 sous forme de cristaux blancs dont les caractéristiques sont les suivantes :
- point de fusion = 130°C.

On prépare l''acide 4,9-éthano-2-(2-méthylphényl)acétyl-9-phényl-2,3,3a,4, 9,9a-hexahydro-1H-benzo[f]isoindole-3a-carboxylique, (3aRS, 4SR, 9SR, 9aRS) dans les conditions décrites dans l'exemple 2 sous forme de cristaux blancs dont les caractéristiques sont les suivantes :
- point de fusion = 188°C
- spectre de masse (DIC ; NH₃) : M/Z = 452 (MH⁺).

### EXEMPLE 63

On prépare l'ester méthylique de l'acide 4,9-éthano-2-(2-fluorophényl) acétyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 1 sous forme de cristaux blancs dont les caractéristiques sont les suivantes :
- point de fusion = 132°C
- spectre de masse (DIC ; NH₃) : M/Z = 470 (MH⁺).

On prépare l'acide 4,9-éthano-2-(2-fluorophényl)acétyl-9-phényl-2,3,3a,4,9 9a-hexahydro-1H-benzo[f]isoindole-3a-carboxylique-(3aRS, 4SR, 9SR, 9aRS) dans les conditions décrites dans l'exemple 2 sous forme de cristaux blancs dont les caractéristiques sont les suivantes :
- point de fusion = 251°C
- spectre de masse (LSIMS) : M/Z = 456 (MH⁺).

### EXEMPLE 64

A une solution de 1 g d'ester méthylique de l'acide 4,9-éthano-2-(2-hydroxyphényl)acétyl-9-phényl-2,3,3a,4,9,9a-hexahydro-1H-benzo[f]isoindole-3a- carboxylique-(3aRS, 4SR, 9SR, 9aRS), préparé dans les conditions décrites dans l'exemple 58, 650 mg de carbonate de potassium et 8 mg d'iodure de potassium dans 20 cm3 d'acétonitrile, on ajoute, à une température voisine de 20°C, 280 cm3 de bromure de benzyle. Le mélange réactionnel est chauffé à une température voisine de 85°C pendant 3 heures 30 minutes puis il est concentré à sec sous pression réduite (2,7 kPa) à 40°C. On ajoute 100 cm3 d'acétate d'éthyle et 50 cm3 d'eau. La phase organique est décantée, séchée sur sulfate de magnésium, filtrée et concentrée à sec sous pression réduite (2,7 kPa) à 40°C. On obtient 1 g d'un produit qui cristallise dans l'oxyde d'isopropyle. Après filtration, on obtient 837 mg d'ester méthylique de l'acide 4,9-éthano-2-(2-benzyloxyphényl)acétyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 146°C.

On prépare l'acide 4,9-éthano-2-(2-benzyloxyphényl)acétyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 2 sous forme de cristaux blancs dont les caractéristiques sont les suivantes :
- point de fusion = 229°C
- spectre de masse (D.I.C. : NH₃) M/Z = 544 (MH⁺).

### EXEMPLE 65

A une solution de 243 mg d'ester méthylique de l'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), préparé dans les conditions décrites dans l'exemple 1, dans 10 cm3 de tétrahydrofurane, on ajoute, à une température voisine de 20°C, 101 mg de réactif de Lawesson. Après 17 heures d'agitation à une température voisine de 20°C, on ajoute 20 cm3 d'eau. La phase aqueuse est extraite avec 3 fois 50 cm3 d'acétate d'éthyle. Les phases organiques sont réunies, lavées avec 30 cm3 d'une solution aqueuse saturée de chlorure de sodium, séchées sur sulfate de magnésium, filtrées et concentrées à sec sous pression réduite (2,7 kPa) à 40°C. On obtient 220 mg d'un produit que l'on purifie par chromatographie préparative sur plaque de silice de 0,2 mm d'épaisseur en éluant avec un mélange acétate d'éthyle-cyclohexane (50-50 en volumes). On obtient ainsi 18,2 mg d'ester méthylique de l'acide 4,9-éthano-2-(2-méthoxyphényl)thioacétyl-9-phényl-2,3,3a,4, 9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 165°C.

On prépare l'acide 4,9-éthano-2-(2-méthoxyphényl)thioacétyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 2 sous forme de cristaux blancs dont les caractéristiques sont les suivantes :
- point de fusion = 224°C
- spectre de R.M.N. ¹H (300 MHz, (CD₃)₂SO d6 avec ajout de quelques gouttes de CD₃COOD d4, δ en ppm) : à température ambiante, on observe un mélange de rotamèresdans les proportions 65/35. 1,30 et de 1,60 à 2,00 (2 mts, 4H en totalité : CH₂CH₂) ; de 3,30 à 4.20 (mt, 10H : CH₂ en 1 - CSCH₂Ar - 1H du CH₂ en 3 - H en 4 - H en 9a et OCH₃) ; 4,48 et 4,70 (2 d, J = 14 Hz, 1H en totalité : l'autre H du CH₂ en 3) ; 6,30 et 6,35 (2 d large, J = 7,5 Hz, 1H en totalité : H en 8) ; 6,85 à 7,55 (mt, 12H : H en 5 - H en 6 - H en 7 - H aromatiques en ortho - méta et para du OCH₃ et H aromatiques du phényle en 9).

### EXEMPLE 66

A une solution de 1 g d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), préparé dans les conditions décrites dans l'exemple 1, et de 10 mg de 4-diméthylaminopyridine dans 15 cm3 de tétrahydrofurane, on ajoute à une température voisine de 20°C, 400 cm3 de 2-méthoxyphénylisocyanate. Le mélange réactionnel est agité à une température voisine de 20°C pendant 1 heure puis on ajoute 50 cm3 d'acétate d'éthyle et 50 cm3 d'eau. La phase organique est décantée, lavée avec 30 cm3 d'une solution aqueuse saturée de chlorure de sodium, séchée sur sulfate de magnésium, filtrée et concentrée à sec sous pression réduite (2,7 kPa) à 40°C. On obtient 2,3 g d'un produit qui cristallise dans l'oxyde d'isopropyle. Après filtration, on obtient 1,2 g d'ester méthylique de l'acide 4,9-éthano-2-(2-méthoxyphényl)aminocarbonyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion = 156°C
- spectre de masse (DIC ; NH₃) : M/Z = 483 (MH⁺).

On prépare l'acide 4,9-éthano-2-(2-méthoxyphényl)aminocarbonyl-9-phényl-2,3,3a,4,9,9a-hexahydro-1H-benzo[f]isoindole-3a-carboxylique-(3aRS, 4SR, 9SR, 9aRS) dans les conditions décrites dans l'exemple 2 sous forme de cristaux blancs dont les caractéristiques sont les suivantes :
- point de fusion supérieur à 260°C
- spectre de R.M.N. ¹H (300 MHz, (CD₃)₂SO d6 avec ajout de quelques gouttes de CD₃COOD d4, δ en ppm) : 1 ,41 - 1 ,69 et de 2,05 à 2,30 (3 mts, respectivement 1H - 1H et 2H : CH₂CH₂) ; de 3,25 à 3,65 (mt, 5H : CH₂ en 1 - 1H du CH₂ en 3 - H en 4 et H en 9a) ; 3,80 (s, 3H : OCH₃) ; 4,20 (d, J = 12 Hz, 1H : l'autre H du CH₂ en 3) ; 6,40 (d large, J = 7,5 Hz, 1H : H en 8) ; 6,90 (dt, J = 7,5 et 2 Hz, 1H : H en 7) ; de 6,90 à 7,00 (mt, 2H : H en 5 et H en 6) ; 7,07 (t large, J = 7,5 Hz, 1H : H aromatique en para du OCH₃) ; de 7,15 à 7,30 (mt, 2H : H aromatique en ortho du OCH₃ et H aromatique en para du CONH) : de 7,35 à 7,60 (mt, 5H : H aromatiques du phényle en 9) ; 7,65 (d large, J = 7,5 Hz, 1H : H aromatique en ortho du CONH).

### EXEMPLE 67

A une suspension de 2 g d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) dans 40 cm3 de dichlorométhane, on ajoute, goutte à goutte à une température voisine de 20°C, 2 cm3 de chlorure de thionyle. Le mélange réactionnel est chauffé à une température voisine de 43°C pendant 4 heures puis laissé sous agitation à une température voisine de 20°C pendant 15 heures. Le mélange réactionnel est ensuite évaporé à sec sous pression réduite (2,7 kPa). On obtient ainsi une huile qui cristallise dans 20 cm3 d'oxyde d'isopropyle. Après filtration, on obtient 1,9 g de chlorure de l'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) sous forme de cristaux blancs dont les caractéristiques sont les suivantes :
- point de fusion. : 140°C.

A une solution de 450 mg de glycinate de t-butyle dans 10 ml d'une solution aqueuse saturée d'hydrogénocarbonate de sodium, on ajoute une solution de 1,4 g de chlorure de l'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS) dans 10 cm3 de dichlorométhane à une température voisine de 20°C. Après 4 heures d'agitation à une température voisine de 20°C, on ajoute 50 cm3 de dichlorométhane et 50 cm3 d'eau. La phase organique est décantée, lavée avec 50 cm3 d'eau puis séchée sur sulfate de magnésium, filtrée et concentrée à sec sous pression réduite (2,7 kPa) à 40°C. On obtient ainsi 2,05 g d'une meringue blanche qui, triturée dans un mélange oxyde d'isopropyle-éther de pétrole, cristallise pour donner, après filtration, 1,54 g d'ester t-butylique de la N-[4,9-éthano-2-(2-méthoxyphényl)acétyl-9-phényl-2,3,3a,4,9,9a-hexahydro-1H-benzo[f]isoindol-3a-yl]carbonyl-glycine-(3aRS, 4SR, 9SR, 9aRS) sous forme de cristaux blancs dont les caractéristiques sont les suivantes :
- point de fusion = 213°C
- spectre de masse (DIC ; NH₃) : M/Z = 581 (MH⁺).

A une solution de 1,5 g d'ester t-butylique de la N-[4,9-éthano-2-(2-méthoxyphényl)acétyl-9-phényl-2,3,3a,4,9,9a-hexahydro-1H-benzo[f]isoindol-3a-yl] carbonyl-glycine-(3aRS, 4SR, 9SR, 9aRS) dans 25 cm3 de dichlorométhame, on ajoute, à une température voisine de 20°C, 2 cm3 d'acide trifluoroacétique. Le mélange réactionnel est agite pendant une nuit à une température voisine de 20°C puis on ajoute 20 cm3 de dichlorométhane et 50 cm3 d'eau. La phase organique est décantée, lavée avec 50 cm3 d'eau puis séchée sur sulfate de magnésium, filtrée et concentrée à sec sous pression réduite (2,7 kPa) à 40°C. On obtient ainsi 1,4 g d'un produit qui est recristallisé dans l'éthanol à chaud pour donner, après filtration, 350 mg de N-[4,9-éthano-2-(2-méthoxyphényl)acétyl-9-phényl-2,3,3a,4,9,9a-hexahydro-1H-benzo[f]isoindol-3a-yl]carbonyl-glycine-(3aRS, 4SR, 9SR, 9aRS) sous forme de cristaux blancs dont les caractéristiques sont les suivantes :
- point de fusion = 262°C
- spectre de R.M.N. ¹H (250 MHz, (CD₃)₂SO d6, à une température de 383 K, δ en ppm) : 1,39 - 1,66 - 1,85 et 2,10 (4 mts, 1H chacun : CH₂CH₂) ; de 3,25 à 3,75 (mt, 9H : CH₂ en 1 - COCH₂Ar - NCH₂COO - 1H du CH₂ en 3 - H en 4 et H en 9a) ; 3,72 (s, 3H : OCH₃) ; 4,28 (d, J = 13 Hz, 1H : l'autre H du CH₂ en 3) ; 6,38 (d large, J = 7,5 Hz, 1H : H en 8) ; 6,90 (t large, J = 7,5 Hz, 1H : H aromatique en para du OCH₃) ; 6,95 (d large, J = 7,5 Hz, 1H : H aromatique en ortho du OCH₃) ; 7,02 (dt, J = 7,5 et 2 Hz, 1H : H en 7) ; de 7,05 à 7,30 (mt, 4H : H en 5 - H en 6 et H aromatiques en méta du OCH₃) ; de 7,35 à 7,55 (mt, 5H : H aromatiques du phényle en 9) ; 7,63 (mf, 1H : CONH).

### EXEMPLE 68

A une solution de 340 mg d'acide 4,9-éthano-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), de 91 mg du chlorure de la N,O-diméthylhydroxylamine et de 130 cm3 de triéthylamine dans 15 cm3 de dichlorométhane, on ajoute 11 mg de 1-hydroxybenzotriazole hydraté et 150 mg de chlorhydrate de 1-éthyl-3-(3-diméthylamino-propyl)-carbodiimide à une température voisine de 20°C. Le mélange réactionnel est agité pendant toute la nuit à une température voisine de 20°C puis on ajoute 50 cm3 de dichlorométhane et 50 cm3 d'eau. La phase organique est décantée, séchée sur sulfate de magnésium filtrée et concentrée à sec sous pression réduite (2,7 kPa) à 40°C. On obtient 300 mg d'un solide blanc qui, trituré dans l'oxyde d'isopropyle cristallise pour donner, après filtration, 180 mg de N-méthoxy-N-méthyl-4,9-éthano-2-(2-méthoxyphényl)acétyl-9-phényl-2,3,3a,4,9,9a-hexahydro- 1H-benzo[f]isoindole-3a-carboxamide-(3aRS, 4SR, 9SR, 9aRS) sous forme de cristaux blancs dont les caractéristiques sont les suivantes :
- point de fusion = 124°C
- spectre de R.M.N. ¹H (300 MHz, (CD₃)₂SO d6, d en ppm) : à une température voisine de 20°C : mélange de rotamères dans les proportions approximatives 1/1 : de 1,20 à 1,95 (mts, 4H : CH₂CH₂) ; 3,02 (s, 3H : NCH₃) ; de 3,25 à 3,85 (mt, 7H : CH₂ en 1 - COCH₂Ar - 1H du CH₂ en 3 - H en 4 - H en 9a) ; 3,78 et 3,82 (2s, 3H en totalité : NOCH₃) ; 4,26 (mt, 1H : l'autre H du CH₂ en 3) ; 6,35 (d large, J = 7,5 Hz, 1H : H en 8) ; de 6,95 à 7,60 (mt, 13H : H en 5 - H en 6 - H en 7 - H aromatiques du benzyle et H aromatiques du phényle en 9).

### EXEMPLE 69

On prépare l'ester méthylique de la N-[4,9-éthano-2-(2-méthoxyphényl) acétyl-9-phényl-2,3,3a,4,9,9a-hexahydro-1H-benzo[f]isoindol-3a-yl]carbonyl-glycine-(3aRS, 4SR, 9SR, 9aRS) dans les conditions décrites dans l'exemple 66 sous forme de cristaux blancs dont les caractéristiques sont les suivantes :
- point de fusion = 227°C
- spectre de masse (D.I.C. : NH₃) M/Z = 539 (MH⁺).

### EXEMPLE 70

Sous atmosphère d'argon, à une solution de 1,1 g ( 1,97 mmole) d'ester méthylique de l'acide 4,9-éthano-7-iodo-9-phényl-2-(t.butoxycarbonyl)-2,3,3a,4,9, 9a-hexahydro-1H-benzo[f]isoindole-3a-carboxylique-(3aRS, 4SR, 9SR, 9aRS), obtenu comme dans l'exemple 43, dans le diméthylformamide, on ajoute 4 cm3 d'une solution 5 M, préparée extemporanément, de méthylate de sodium dans le méthanol et 87 mg (0,97 mmole) de chlorure cuivrique. Après 1 heure 30 minutes. d'agitation à 60°C puis pendant 20 heures à une température voisine de 20°C, on ajoute du chlorure d'ammonium en excès jusqu'à forte coloration bleue, puis on agite pendant 1 heure et on évapore le méthanol sous pression réduite. Le résidu est repris par de l'eau et de l'acétate d'éthyle. Après décantation, lavage à l'eau jusqu'à neutralité, séchage sur sulfate de magnésium, et concentration du solvant sous pression réduite, le résidu huileux brun est purifié par flash-chromatographie sur gel de silice (230-400 mesh), en éluant avec un mélange de dichlorométhane et de méthanol (99-1 en volumes). On obtient ainsi 0,7 g (79,5%) d'ester méthylique de l'acide 4,9-éthano-7-méthoxy-9-phényl-2-(t.butoxycarbonyl)-2,3,3a,4,9,9a-hexahydro-1H-benzo[f]isoindole-3a-carboxylique-(3aRS, 4SR, 9SR, 9aRS), sous forme d'une huile qui cristallise partiellement dont les caractéristiques sont les suivantes :
- spectre de R.M.N. ¹H (400 MHz, (CD₃)₂SO d6, à une température de 383°K, δ en ppm) : 1,45 (s, 9H : OC(CH₃)₃) ; 1,45 - 1,75 et de 2,00 à 2,20 (3 mts, respectivement 1H -1H et 2H : CH₂CH₂) ; de 3,20 à 3,35 (mt, 3H : CH₂ en 1 et H en 9a) ; 3,45 (mt, 1H : H en 4) ; 3,45 et 3,98 (2 d, J = 12,5 Hz, 1H chacun : CH₂ en 3) ; 355 (s, 3H : COOCH₃) ; 3,60 (s, 3H : OCH₃) ; 6,00 (d J = 1 Hz, 1H : H en 8) ; 6,75 (dd, J = 8 et 1 Hz, 1H : H en 6) ; 7,09 (d, J = 8 Hz, 1H : H en 5) ; 7,42 (t large, J = 7,5 Hz, 1H : H aromatique en para du phényle en 9) ; 7,45 (d large, J = 7,5 Hz, 2H : H aromatiques en ortho du phényle en 9) ; 7,53 (t large, J = 7,5 Hz, 2H : H aromatiques en para du phényle en 9).

A partir de 0,58 g (1,20 mmole) d'ester méthylique de l'acide 4,9-éthano-7-méthoxy-9-phényl-2-(t.butoxycarbonyl)-2,3,3a,4,9,9a-hexahydro-1H-benzo[f]-isoindole-3a-carboxylique-(3aRS, 4SR, 9SR, 9aRS) dans un large excès de méthanol chlorhydrique, on obtient 0,49 g (98 %) de chlorhydrate de l'ester méthylique de l'acide 4,9-éthano-7-méthoxy-9-phényl-2,3,3a,49,9a-hexahydro-1H-benzo[f]-isoindole-3a-carboxylique-(3aRS, 4SR, 9SR, 9aRS), sous forme d'une poudre blanche dont les caractéristiques sont les suivantes:
- point de fusion : supérieur à 260°C
- spectre de masse (D.I.C. : NH₃) M/Z = 363 (MH⁺).

A partir de 0,79 g (1,97 mmole) de chlorhydrate de l'ester méthylique de l'acide 4,9-éthano-7-méthoxy-9-phényl-2,3,3a,4,9,9a-hexahydro-1H-benzo[f]-isoindole-3a-carboxylique-(3aRS, 4SR, 9SR, 9aRS) et de 0,39 g (2,37 mmoles) d'acide 2-méthoxyphénylacétique, en présence de 0,2 g (1,97 mmole) de triéthylamine, de 0,45 g (2,37 mmoles) de chlorhydrate de 1-éthyl-3-(3-diméthylaminopropyl)-carbodiimide et de 26 mg (0,2 mmole) d'hydroxybenzotriazole en solution dans 20 cm3 de dichlorométhane, on obtient, après purification par flash-chromatographie sur gel de silice (230-400 mesh) en éluant avec un mélange de dichlorométhane et de méthanol (98-2 en volumes) suivie d'une recristallisation dans du méthanol aqueux à 50 %, 147 mg (15 %) d'ester méthylique de l'acide 4,9-éthano-7-méthoxy-2-(2-méthoxyphényl)acétyl-9-phényl-2,3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 146°C
- spectre de masse (D.I.C. : NH₃) M/Z = 511 (MH⁺).

A partir de 0,49 g (0,96 mmole) d'ester méthylique de l'acide 4,9-éthano-7-méthoxy-2-(2-méthoxyphényl)acétyl-9-phényl-2,3,3a,4,9,9a-hexahydro-1H-benzo[f]-isoindole-3a-carboxylique-(3aRS, 4SR, 9SR, 9aRS) et de 1 cm3 de soude 1N dans 30 cm3 d'éthanol, on obtient, après purification par recristallisation dans de l'éthanol aqueux à 80 %, 165 mg (35 %) d'acide 4,9-éthano-7-méthoxy-2-(2-méthoxyphényl) acétyl-9-phényl-2,3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 226°C
- spectre de R.M.N. ¹H (300 MHz, (CD₃)₂SO d6, δ en ppm), à une température de 393°K, on observe un mélange 50-50 de deux rotamères : de 1,30 à 2,30 (mts, 4H en totalité : CH₂CH₂): de 3,10 à 3,90 (mt, 7H : CH₂ en 1 - NCO CH₂ - 1H du CH₂ en 3 - H en 4 et H en 9a) ; 3,52 - 3,55 - 3,60 et 3,80 (4 s, 6H en totalité : ArOCH₃) ; 4,12 et 4,22 (2 d, J = 12,5 Hz, 1H en totalité : l'autre H du CH₂ en 3) ; 5,90 et 5,92 (2 d, J = 1 Hz, 1H en totalité : H en 8) ; 6,75 (mt, 1H : H en 6) ; de 6,90 à 7,60 (mt, 10H : H aromatiques).

### EXEMPLE 71

A partir de 150 mg (0,312 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 40 mg (0,375 mmole) de benzylamine, de 72 mg (0,375 mmole) de chlorhydrate de 1-éthyl-3-(3-diméthylaminopropyl)-carbodiimide et de 4,2 mg (0.03 mmole) d'hydroxybenzotriazole en solution dans 10 cm3 de dichlorométhane, on obtient, après 18 heures d'agitation à une tempe'rature voisine de 20°C et chromatographie du produit brut obtenu sur une plaque de silice préparative, en éluant avec un mélange de cyclohexane et d'acétate d'éthyle (50-50 en volumes), 160 mg (94 %) de N-[benzyl)]-[4,9-éthano-2-(2-méthoxyphényl)-2-méthylènyl-acétyl]-9-phényl-2,3,3a,4,9,9a-hexahydro-1H-benzo[f]isoindole-3a-carboxamide-(3aS, 4R, 9R, 9aS) sous forme d'une poudre blanche dont les caractéristiques sont les suivantes :
- point de fusion = 235°C
- spectre de masse (E.I.) M/Z = 568 (M⁺).

### EXEMPLE 72

A partir de 300 mg (0,625 mmole) d'acide 4,9-éthano-2-[(2-méthoxyphényl) prop-2-én-1-oyl]-9-phényl-2,3,3a,4,9,9a-hexahydro-1H-benzo[f]isoindole-3a-carboxylique-(3aS, 4R, 9R, 9aS) (exemple 39), de 151 mg (0,75 mmole) du chlorhydrate de l'ester méthylique de la (S)-phénylglycine, de 144 mg (0,75 mmole) de chlorhydrate de 1-éthyl-3-(3-diméthylamino-propyl)-carbodiimide, de 7 mg (0,065 mmole) d'hydroxy-benzotriazole et de 76 mg (0,75 mmole) de triéthylamine en solution dans 10 cm3 de dichlorométhane, on obtient, après 18 heures d'agitation à une température voisine de 20°C et chromatogaphie du produit brut obtenu sur une plaque de silice préparative, en éluant avec un mélange de cyclohexane et d'acétate d'éthyle (60-40 en volumes), 230 mg (59%) de N-[(S)-méthyl phénylglycinoyl)]-[4,9-éthano-2-(2-méthoxyphényl)-2-méthylényl-acétyl]-9-phényl-2,3,3a,4,9,9a-hexahydro-1H-benzo[f]isoindole-3a-carboxamide-(3aS, 4R, 9R, 9aS) sous forme d'une poudre blanche dont les caractéristiques sont les suivantes :
- point de fusion = 214-215°C
- spectre de masse (E.I.) M/Z = 626 (M⁺).

### EXEMPLE 73

A une solution de 0,3 g ( 0,53 mmole) d'ester méthylique de l'acide 4,9-éthano-7-iodo-9-phényl-2-(t.butoxycarbonyl)-2,3,3a,4,9,9a-hexahydro-1H-benzo[f] isoindole-3a-carboxylique-(3aRS, 4SR, 9SR, 9aRS), obtenu comme dans l'exemple 43, dans 10 cm3 de diméthylformamide, on ajoute, sous atmosphère d'argon, successivement 0,2 cm3 (0,67 mmole) de tri-n.butyl-vinyl-stannane et 50 mg (0,05 mmole) de tétrakis(triphénylphosphine)-palladium. Après 3 heures d'agitation à 90°C puis 20 heures à une température voisine de 20°C, on ajoute 50 cm3 d'une solution d'ammoniaque à 10 % et 50 cm3 de dichlorométhane et on agite vigoureusement pendant 30 minutes. Après décantation, lavage à l'eau jusqu'à neutralité, séchage sur sulfate de magnésium, et concentration du solvant sous pression réduite, le résidu huileux noir est purifié par flash-chromatographie sur gel de silice (230-400 mesh), en éluant avec un gradient de mélanges de cyclohexane et d'acétate d'éthyle (de 99-1 à 90-10 en volumes). On obtient ainsi 0,1 g (40,5 %) d'ester méthylique de l'acide 4,9-éthano-9-phényl-2-(t.butoxycarbonyl)-7-vinyl-2,3,3a,4,9,9a-hexahydro-1H-benzo [f]isoindole-3a-carboxylique-(3aRS, 4SR, 9SR, 9aRS), sous forme d'une huile incolore dont les caractéristiques sont les suivantes :
- spectre de R.M.N. ¹H (400 MHz, (CD₃)₂SO d6, δ en ppm) : 1,45 (s, 9H : C(CH₃)₃) ; 1,46 - 1,75 et de 2,05 à 2,20 (3 mts, respectivement 1H - 1H et 2H : CH₂CH₂) ; de 3,20 à 3,40 (mt, 3H : CH₂ en 1 et H en 9a) ; 3,47 et 4,00 (2d, J = 12,5 Hz, 1H chacun : CH₂ en 3) ; 3,50 (mt, 1H : H en 4) ; 3,58 (s, 3H : COOCH₃) ; 5,10 (d large, J = 11 Hz, 1H : H en cis du = CH₂) ; 5,45 (d large, J = 17 Hz, 1H : H en trans du =CH₂) ; 6,49 (d, J = 1 Hz, 1H : H en 8) ; 6,57 (dd, J = 17 et 11 Hz, 1H : ArCH=) ; 7,17 (d, J = 8 Hz, 1H : H en 5) ; 7,26 (dd, J = 8 et 1 Hz, 1H : H en 6) ; de 7,35 à 7,50 (mt, 3H : H aromatiques en ortho et para pour le phényle en 9) ; 7,56 (t large, J = 8 Hz, 2H : H aromatiques en méta pour le phényle en 9).

A partir de 0,7 g (1,50 mmole) d'ester méthylique de l'acide 4,9-éthano-9-phényl-2-(t.butoxycarbonyl)-7-vinyl-2,3,3a,4,9,9a-hexahydro-1H-benzo[f]isoindole-3a-carboxylique-(3aRS, 4SR, 9SR, 9aRS) dans un large excès de méthanol chlorhydrique, on obtient 0,44 g (73 %) de chlorhydrate de l'ester méthylique de l'acide 4,9-éthano-9-phényl-7-vinyl-2,3,3a,4,9,9a-hexahydro-1H-benzo[f]-isoindole-3a-carboxylique-(3aRS, 4SR, 9SR, 9aRS), sous forme d'une poudre blanche dont les caractéristiques sont les suivantes :
- spectre de R.M.N. ¹H (300 MHz, (CD₃)₂SO d6, δ en ppm) : 1,48 - 1,82 et de 2,15 à 2,40 (3 mts, respectivement 1H - 1H et 2H : CH₂CH₂) : de 2,80 à 3,80 (mt, 6H : CH₂ en 1 - CH₂ en 3 - H en 4 et H en 9a) ; 3,55 (s, 3H : COOCH₃) : 5,10 (d large, J = 11 Hz, 1H : H en cis du =CH₂) ; 5,45 (d large, J = 17 Hz, 1H : H en trans du =CH₂) : 6,42 (d, J = 1 Hz, 1H : H en 8) ; 6,55 (dd, J = 17 et 11 Hz, 1H : ArCH=) ; 7,15 (d, J = 8 Hz, 1H : H en 5) ; 7,29 (dd, J = 8 et 1 Hz, 1H : H en 6); de 7,35 à 7,50 (mt. 3H : H aromatiques en ortho et para pour le phényle en 9) ; 7,56 (t large, J = 8 Hz, 2H : H aromatiques en méta pour le phényle en 9).

A partir de 0,51 g (1.30 mmole) de chlorhydrate de l'ester méthylique de l'acide 4,9-éthano-9-phényl-7-vinyl-2,3,3a,4,9,9a-hexahydro-1H-benzo[f]-isoindole-3a-carboxylique-(3aRS, 4SR, 9SR, 9aRS) et de 0,26 g (1.50 mmole) d'acide 2-méthoxyphénylacétique, en présence de 0,132 g (1.30 mmole) de triéthylamine, de 0,30 g (1.50 mmole) de chlorhydrate de 1-éthyl-3-(3-diméthylamino-propyl)-carbodiimide et de 20 mg (0,13 mmole) d'hydroxybenzotriazole en solution dans 50 cm3 de dichlorométhane, on obtient, après purification par flash-chromatographie sur gel de silice (230-400 mesh) en éluant avec un mélange de dichlorométhane et de méthanol (98-2 en volumes). 230 mg (48 %) d'ester méthylique de l'acide 4.9-éthano-2-(2-méthoxyphényl)acétyl-9-phényl-7-vinyl-2,3,3a,4,9,9a-hexahydro-1H-benzo[f]-isoindole-3a-carboxylique-(3aRS, 4SR, 9SR, 9aRS), sous forme d'une poudre blanche dont les caractéristiques sont les suivantes :
- point de fusion = 45-50°C
- spectre de masse (D.I.C. : NH₃) M/Z = 507 (MH⁺).

A partir de 0,32 g (0,63 mmole) d'ester méthylique de l'acide 4,9-éthano-2-(2-méthoxyphényl)acétyl-9-phényl-7-vinyl-2,3,3a,4.9,9a-hexahydro-1H-benzo[f]-isoindole-3a-carboxylique-(3aRS, 4SR, 9SR, 9aRS) et de 0,65 cm3 de soude 1N dans 40 cm3 d'éthanol, on obtient, après purification par chromatographie liquide à haute performance sur gel de silice greffée C₁₈ 〈〈 Hyperprep 〉〉 en éluant par un mélange acétonitrile-eau (50-50 en volumes) contenant 0,05 % d'acide trifluoroacétique, 138 mg (46 %) d'acide 4,9-éthano-2-(2-méthoxyphényl)acétyl-9-phényl-7-vinyl-2,3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 220°C
- spectre de R.M.N. ¹H (400 MHz, (CD₃)₂SO d6, à une température de 383°K, δ en ppm) : 1,40 - 1,68 - 1,95 et 2,10 (4 mts, 1H chacun : CH₂CH₂) ; de 3,30 à 3,65 (mt, 5H : CH₂ en 1 - NCO CH₂ - 1H du CH₂ en 3 et H en 9a) ; 3,48 (mt, 1H : H en 4) ; 3,72 (s large, 3H : OCH₃) ; 4,18 (d, J = 12,5 Hz, 1H: l'autre H du CH₂ en 3) ; 5,08 (d large, J = 11 Hz, 1H : H en cis du =CH₂) ; 5,42 (d large, J = 17 Hz, 1H : H en trans du =CH₂) ; 6,47 (d, J = 1 Hz, 1H : H en 8) ; 6,57 (dd, J = 17 et 11 Hz, 1H : ArCH=) ; 6,90 (t large, J = 7,5 Hz, 1H : H aromatique en para du OCH₃) ; 6,96 (d large, J = 7,5 Hz, 1H : H aromatique en ortho du OCH₃) ; de 7,15 à 7,30 (mt, 4H : H en 5 - H en 6 et H aromatiques en méta du OCH₃) ; 7,42 (mt, 3H : H aromatiques en ortho et para pour le phényle en 9). ; 7,54 (t large, J = 8 Hz, 2H : H aromatiques en méta pour le phényle en 9).

### EXEMPLE 74

A partir de 0,93 g (2,8 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 0,51 g (2,8 mmoles) d'acide 2-éthoxyphénylacétique, de 0,64 g (3,3 mmoles) de chlorhydrate de 1-éthyl-3-(3-diméthylamino-propyl)-carbodiimide et de 37 mg (0,27 mmole) d'hydroxybenzotriazole en solution dans 30 cm3 de dichlorométhane, on obtient, après 18 heures d'agitation à une température voisine de 20°C et purification du produit brut obtenu par flash-chromatographie sur gel de silice (230-400 mesh), en éluant avec un mélange cyclohexane-acétate d'éthyle (70-30 en volumes), 1,18 g (86 %) d'ester méthylique de l'acide 4,9-éthano-2-(2-éthoxyphényl)acétyl-9-phényl-2,3,3a,4,9,9a-hexahydro-1H-benzo[f]isoindole-3a- carboxylique-(3aRS, 4SR, 9SR, 9aRS) sous forme d'une poudre blanche dont les caractéristiques sont les suivantes :
- point de fusion = 142-144°C
- spectre de masse (D.C.I. : NH₃) M/Z = 495 (MH⁺).

A partir de 1,17 g (2,4 mmoles) d'ester méthylique de l'acide 4,9-éthano-2-(2-éthoxyphényl)acétyl-9-phényl-2,3,3a,4,9,9a-hexahydro-1H-benzo[f]isoindole-3a-carboxylique-(3aRS, 4SR, 9SR, 9aRS) chauffé au reflux pendant 4 heures dans 30 cm3 de soude 1N et 30 cm3 d'éthanol, on obtient après purification par recristallisation dans de l'éthanol à 90 %, 890 mg (86 %) d'acide 4,9-éthano-2-(2-éthoxyphényl)acétyl-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 251-253°C
- spectre de masse (D.C.I. : NH₃) M/Z = 481 (MH⁺).

### EXEMPLE 75

460 mg (0,96 mmole) d'acide 4,9-éthano-2-(2-éthoxyphényl)acétyl-9-phényl-2,3,3a,4,9,9a-hexahydrol-1H-benzo[f]isoindole-3a-carboxylique (3aRS, 4SR, 9SR, 9aRS) sont dedoublés sur une colonne de silice chirale, porteuse de greffons (3,5-dinitrobenzyl)phénylalanine préparée comme à l'exmple 31, en éluant par un mélange dichlorométhane-isopropanol-n.heptane (75-20-2,5 en volumes). En recueillant la deuxième fraction éluée (temps de rétention = 17,2 minutes), on obtient 210 mg d'acide 4,9-éthano-2-(2-éthoxyphényl)acétyl-9-phényl-2,3,3a,4,9,9a-hexahydro-1H-benzo[f]isoindole-3a-carboxylique-(3aS, 4R, 9R, 9S), énantiomère dextrogyre, sous forme de cristaux blancs dont les caractéristiques sont les suivantes:
- point de fusion = 221-223°C
- pouvoir rotatoire [α]₃₆₅²⁰ = +536 ± 1 (c = 0,5 ; dichlorométhane)
- spectre de masse (D.C.I. : NH₃) M/Z = 481 (MH⁺).

### EXEMPLE 76

A partir de 1,04 g (3,12 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 0,60 g (3,12 mmoles) d'acide 8-chromanylacétique, de 0,72 g (3,75 mmoles) de chlorhydrate de 1-éthyl-3-(3-diméthylamino-propyl)-carbodiimide et de 40 mg (0,38 mmole) d'hydroxybenzotriazole en solution dans 30 cm3 de dichlorométhane, on obtient, après 18 heures d'agitation à une température voisine de 20°C et purification du produit brut obtenu par flash-chromatographie sur gel de silice (230-400 mesh), en éluant avec un mélange cyclohexane-acétate d'éthyle (80-20 en volumes), 1,41 g (89 %) d'ester méthylique de l'acide 2-(8-chromanyl)acétyl-4,9-éthano-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 176-178°C
- spectre de masse (D.C.I. : NH₃) M/Z = 507 (MH⁺).

L'acide 8-chromanylacétique peut être préparé en trois étapes à partir de chromane, avec un rendement global de 60 %, en s'inspirant de Synth. Comm., 12, 763-70 (1982) par traitement au n.butyllithium puis au diméthylformamide dans le tétrahydrofurane, le chromane conduit au 8-chromanal, qui est ensuite traité par du cyanure de triméthylsilane en présence d'iodure de zinc dans le dichlorométhane. La cyanhydrine triméthylsilylée ainsi obtenue est traitée par du chlorure stanneux dans un mélange d'acide acétique et d'acide chlorhydrique pour fournir l'acide 8-chromanylacétique.

A partir de 0,92 g (1,8 mmole) d'ester méthylique de l'acide 2-(8-chromanyl)acétyl-4,9-éthano-9-phényl-2,3,3a,4,9,9a-hexahydro-1H-benzo[f] isoindole-3a-carboxylique-(3aRS, 4SR, 9SR, 9aRS) chauffé au reflux pendant 4 heures dans 2,3 cm3 de soude 1N et 30 cm3 d'éthanol, on obtient après purification par recristallisation dans de l'éthanol à 90 %, 500 mg (56 %) d'acide 2-(8-chromanyl)acétyl-4,9-éthano-9-phényl-2,3,3a,4,9,9a-hexahydro-1H-benzo[f] isoindole-3a-carboxylique-(3aRS, 4SR, 9SR, 9aRS) sous forme d'une poudre blanche dont les caractéristiques sont les suivantes :
- point de fusion = 265-266°C
- spectre de masse (D.C.I. : NH₃) M/Z = 493 (MH⁺).

### EXEMPLE 77

A une solution refroidie vers 0°C de 1,86 g (5 mmoles) de bromure de triphénylphosphonio-éthane dans 25 cm3 d'oxyde de diéthyle on ajoute successivement 2,6 cm3 (5,2 mmoles) d'une solution 2M de diisopropylamidure de lithium et 1,04 g (5 mmoles) d'ester éthylique de l'acide (2-méthoxyphényl)-oxo-acétique dans 10 cm3 d'oxyde de diéthyle. Après 20 heures d'agitation à une température voisine de 20°C, on ajoute 30 cm3 d'eau. Après décantation, la phase organique est lavée avec une solution à 10 % de dithionite de sodium, séchée sur sulfate de magnésium et concentrée sous pression réduite. Après purification par flash-chromatographie sur gel de silice (230-400 mesh) en éluant par un mélange de cyclohexane et d'acétate d'éthyle (90-10 en volumes), on obtient 0,5 g (46 %) d'ester éthylique de l'acide 2-(2-méthoxyphényl)-butèn-2-oïque sous forme d'une huile incolore contenant un mélange 80-20 des isomères E et Z, dont les caractéristiques sont les suivantes :
- spectre de R.M.N. ¹H (300 MHz, CDCl₃, δ en ppm) . (mélange d'isomères E et Z dans les proportions 80/20) : 1,23 et 1,25 (2 t, J = 7 Hz, 3H en totalité : CH₃ de l'éthyle des deux isomères) ; 1,70 et 2.,10 (2 d, J = 6,5 Hz, 3H en totalité : respectivement CH₃ de l'isomère majoritaire et CH₃ de l'isomère minoritaire) ; 3,78 (s, 3H : COOCH₃ des deux isomères) ; 4,20 et 4,22 (2 q, J = 7 Hz, 2H en totalité : respectivement CH₂ de l'isomère majoritaire et CH₂ de l'isomère minoritaire) ; 6,12 et 7,10 (2 q, J = 6,5 Hz, 1H en totalité : respectivement =CH de l'isomère minoritaire et =CH de l'isomère majoritaire) ; de 6,80 à 7,35 (mt, 4H : H aromatiques).

L'ester éthylique de l'acide (2-méthoxyphényl)-oxo-acétique peut être obtenu selon Synth. Comm., 20, 1781-91 (1990).

A partir de 0,3 g (1,3 mmole) d'ester éthylique de l'acide 2-(2-méthoxyphényl)-butén-2-oique dans 13 cm3 de soude 1N et 13 cm3 d'éthanol, on obtient 0,24 g (96 %) d'acide 2-(2-méthoxyphényl)-butén-2-oïque sous forme d'une poudre blanche dont les caractéristiques sont les suivantes :
- point de fusion = 205-206°C
- spectre de masse (D.C.I. : NH₃) M/Z = 192 (MH⁺).

A partir de 0,42 g (1,26 mmole) d'ester méthylique de l'acide 4,9-éthano-9-phényl-2,3,3a.4,9,9a-hexahydro-1H-benzo[f]isoindole-3a-carboxylique-(3aRS, 4SR, 9SR, 9aRS), de 0,25 g (1,26 mmole) d'acide 2-(2-méthoxyphényl)-butén-2-oique, de 0,25 g (1,26 mmole) de chlorhydrate de 1-éthyl-3-(3-diméthylamino-propyl)-carbodiimide et de 17 mg (0,126 mmole) d'hydroxybenzotriazole en solution dans 20 cm3 de dichlorométhane, on obtient, après 18 heures d'agitation à une température voisine de 20°C et purification du produit brut obtenu par flash-chromatographie dur gel de silice (230-400 mesh) en éluant avec un mélange cyclohexane-acétate d'éthyle (40-60 en volumes) 0,39 g (61%) d'ester méthylique de I'acide 4,9-éthano-2-[2(E)-(2-méthoxyphényl)-butén-2-oyl]-9-phényl-2,3,3a,4,9,9a-hexahydro-1H-benzo[f]isoindole-3a-carboxylique-(3aRS, 4SR, 9SR, 9aRS) sous forme d'une poudre blanche dont les caractéristiques sont les suivantes :
- point de fusion = 177-178°C
- spectre de R.M.N. ¹H (400 MHz, (CD₃)₂SO d6, à une température de 393°K, δ en ppm) : 1,37 - 1,60 - 1,88 et 2,02 (4 mts, 1H chacun : CH₂CH₂) ; 1,58 (d, J = 6,5 Hz, 3H : CH₃) ; 3,28 et 3,36 (respectivement dd et d large, J = 11 et 8 Hz et J = 11 Hz, 1H chacun : CH₂ en 1) ; 3,42 (mt, 2H : H en 9a et H en 4) ; 3,55 et 4,15 (2 d, J = 12,5 Hz, 1H chacun : CH₂ en 3) ; 3,55 (s, 3H : COOCH₃) ; 3,63 (s, 3H : OCH₃) ; 6,19 (q, J = 6,5 Hz, 1H : =CH) ; 6,41 (d, J = 7,5 Hz, 1H : H en 8) ; 6,95 (t large, J = 7,5 Hz, 1H : H aromatique en para du OCH₃) ; 7,00 (d large, J = 7,5 Hz, 1H : H aromatique en ortho du OCH₃) ; de 7,00 à 7,45 (mt, 5H : H en 5 - H en 6 - H en 7 et H aromatiques en méta du OCH₃) ; de 7,30 à 7,45 (mt, 3H : H aromatiques en ortho et para pour le phényle en 9) ; 7,53 (t large, J = 8 Hz, 2H : H aromatiques en méta pour le phényle en 9).

### EXEMPLE 78

A partir de 0,35 g (0,69 mmole) d'ester méthylique de l'acide 4,9-éthano-2-[2(E)-(2-méthoxyphényl)-butén-2-oyl]-9-phényl-2,3,3a,4,9,9a-hexahydro-1H-benzo[f]isoindole-3a-carboxylique (3aRS, 4SR, 9SR, 9aRS) dans 7 cm3 de soude 1N et 7 cm3 d'éthanol, on obtient, après recristallisation dans un mélange de dichlorométhane et de pentane 0,18 g (52 %) d'acide 4,9-éthano-2-[2(E)-(2-méthoxyphényl)-butén-2-oyl]-9-phényl-2,3,3a,4,9,9a-hexahydro-1H-benzo[f] isoindole-3a-carboxylique (3aRS, 4SR, 9SR, 9aRS), sous forme d'une poudre blanche dont les caractéristiques sont les suivantes :
- point de fusion = 190-193°C
- spectre de R.M.N. ¹H (400 MHz, (CD₃)₂SO d6, à une température de 403°K, δ en ppm) : 1,37 - 1,60 - 1,88 et 2,05 (4 mts, 1H chacun : CH₂CH₂) ; 1,60 (d, J = 6,5 Hz, 3H : CH₃) ; 3,25 et 3,35 (respectivement dd et d large, J = 12 et 8 Hz et J = 12 Hz, 1H chacun : CH₂ en 1) ; 3,44 (mt, 2H : H en 9a et H en 4) ; 3,55 et 4,15 (2 d, J = 12,5 Hz, 1H chacun : CH₂ en 3) ; 3,65 (s, 3H : OCH₃) ; 6,19 (q, J = 6,5 Hz, 1H : =CH) ; 6,39 (d, J = 7,5 Hz, 1H : H en 8) ; 6,95 (t large, J = 7,5 Hz, 1H : H aromatique en para du OCH₃) ; 7,00 (d large, J = 7,5 Hz, 1H : H aromatique en ortho du OCH₃) ; de 6,95 à 7,45 (mt, 5H : H en 5 - H en 6 - H en 7 et H aromatiques en méta du OCH₃) ; de 7,30 à 7,45 (mt, 3H : H aromatiques en ortho et para pour le phényle en 9) ; 7,52 (t large, J = 8 Hz, 2H : H aromatiques en méta pour le phényle en 9).

### EXEMPLE 79

A partir d'une solution de 2,16 g (5 mmoles) d'iodure de triphénylphosphonio-i.propane dans 25 cm3 d'oxyde de diéthyle, de 2,6 cm3 (5,2 mmoles) d'une solution 2M de diisopropylamidure de lithium et 1,04 g (5 mmoles) d'ester éthylique de l'acide (2-méthoxyphényl)-oxo-acétique dans 10 cm3 d'oxyde de diéthyle, on obtient après purification par flash-chromatographie sur gel de silice (230-400 mesh) en éluant par un mélange cyclohexane-acétate d'éthyle (90-10 en volumes), on obtient 0,31 g (25 %) d'ester éthylique de l'acide 2-(2-méthoxyphényl)-3-méthyl-butén-2-oique sous forme d'une huile incolore, dont les caractéristiques sont les suivantes :
- spectre de masse (D.C.I. : NH₃) M/Z = 234 (MH⁺).

A partir de 0,3 g (1,28 mmole) d'ester éthylique de l'acide 2-(2-méthoxyphényl)-3-méthyl-butén-2-oique dans 13 cm3 de soude 1N et 13 cm3 d'éthanol, on obtient 0,21 g (80 %) d'acide 2-(2-méthoxyphényl)-3-méthyl-butén-2-oique sous forme d'une poudre blanche dont les caractéristiques sont les suivantes :
- point de fusion = 142-144°C
- spectre de masse (D.C.I : NH₃) M/Z = 206 (MH⁺).

A partir de 0,32 g (0,9 mmole) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 0,21 g (0,9 mmole) d'acide 2-(2-méthoxyphényl)-3-méthyl-butén-2-oique, de 0,19 g (0,9 mmole) de chlorhydrate de 1-éthyl-3-(3-diméthylamino-propyl)-carbodiimide et de 13,5 mg (0,1 mmole) d'hydroxybenzotriazole en solution dans 20 cm3 de dichlorométhane, on obtient, après 18 heures d'agitation à une température voisine de 20°C et purification du produit brut obtenu par flash-chromatographie sur gel de silice (230-400 mesh), en éluant avec un mélange cyclohexane-acétate d'éthyle (10-90 en volumes), puis recristallisation dans du pentane, 0,29 g (62 %) d'ester méthylique de l'acide 4,9-éthano-2-[2-(2-méthoxyphényl)-3-méthyl-butén-2-oyl]-9-phényl-2,3,3a,4,9,9a-hexahydro-1H-benzo [f]isoindole-3a-carboxylique (3aRS, 4SR, 9SR, 9aRS) sous forme d'une poudre blanche dont les caractéristiques sont les suivantes :
- point de fusion = 132-134°C
- spectre de masse (D.C.I. : NH₃) M/Z = 521 (MH⁺).

A partir de 0,20g (0,38 mmole) d'ester méthylique de l'acide 4,9-éthano-2-[2-(2-méthoxyphényl)-3-méthyl-butén-2-oyl]-9-phényl-2,3,3a,4,9,9a-hexahydro-1H-benzo[f]isoindole-3a-carboxylique-(3aRS, 4SR, 9SR, 9aRS) dans 4 cm3 de soude 1N et 8 cm3 d'éthanol, on obtient, après recristallisation dans un mélange de dichlorométhane et de pentane, 0,16 g (83 %) d'acide 4,9-éthano-2-[2-(2-méthoxyphényl)-3-méthyl-butén-2-oyl]-9-phényl-2,3,3a,4,9,9a-hexahydro-1H-benzo [f]isoindole-3a-carboxylique-(3aRS, 4SR, 9SR, 9aRS), sous forme d'une poudre blanche dont les caractéristiques sont les suivantes :
- point de fusion = 170-172°C
- spectre de masse (D.C.I. : NH₃) M/Z = 507 (MH⁺).

### EXEMPLE 80

A partir de 0,69 g (2,1 mmoles) d'ester méthylique de l'acide 4,9-éthano-9-phényl-2,3,3a,4,9,9a-hexahydro-1H-benzo[f]isoindole-3a-carboxylique-(3aRS, 4SR, 9SR, 9aRS), de 0,40 g (2,1 mmoles) d'acide 2-n.propoxyphénylacétique, de 0,47 g (2,47 mmoles) de chlorhydrate de 1-éthyl-3-(3-diméthylamino-propyl)-carbodiimide et de 30 mg (0,29 mmole) d'hydroxybenzotriazole en solution dans 20 cm3 de dichlorométhane, on obtient, après 18 heures d'agitation à une température voisine de 20°C et purification du produit brut obtenu pur flash-chromatographie sur gel de silice (230-400 mesh), en éluant avec un mélange cyclohexane-acétate d'éthyle (80-20 en volumes), 0,95 g (91 %) d'ester méthylique de l'acide 4,9-éthano-9-phényl-2-(2-n.propoxyphényl)acétyl-2,3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 140-145°C
- spectre de masse (D.C.I. : NH₃) M/Z = 495 (MH⁺).

L'acide 2-n.propoxyphénylacétique peut être préparé en deux étapes à partir de l'ester méthylique de l'acide 2-hydroxyphénylacétique, avec un rendement global de 86 %, par traitement à l'iodure de n.propyle en présence de carbonate de potassium dans le diméthylformamide suivie d'une saponification de l'ester obtenu par de la soude 1N dans l'éthanol.

A partir de 0,95 g (1,8 mmole) d'ester méthylique de l'acide 4,9-éthano-9-phényl-2-(2-n.propoxyphényl)acétyl-2,3,3a,4,9,9a-hexahydro-1H-benzo[f]isoindole-3a-carboxylique-(3aRS, 4SR, 9SR, 9aRS) chauffé au reflux pendant 4 heures dans 2,3 cm3 de soude 1N et 30 cm3 d'éthanol, on obtient après purification par recristallisation dans de l'éthanol à 90 %, 260 mg (36 %) d'acide 4,9-éthano-9-phényl-2-(2-n.propoxyphényl)acétyl-2,3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 243-246°C
- spectre de masse (D.C.I. : NH₃) M/Z = 509 (MH⁺).

### EXEMPLE 81

A partir de 350 mg (0,73 mmole) d'acide 4,9-éthano-2-[(2-méthoxyphényl) prop-2-én-1-oyl]-9-phényl-2,3,3a,4,9,9a-hexahydro-1H-benzo[f]isoindole-3a-carboxylique-(3aS, 4R, 9R, 9aS) (exemple 39), de 214 mg (0,87 mmole) de chlorhydrate de (S)-phénylglycinate de t.butyle, de 168 mg (0,87 mmole) de chlorhydrate de 1-éthyl-3-(3-diméthylamino-propyl)-carbodiimide et de 10 mg (0,07 mmole) d'hydroxybenzotriazole en solution dans 10 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (80-20 en volumes), 470 mg (94 %) de N-[(S)-t.butyl-phénylglycinoyl)]-4,9-éthano-2-[(2-méthoxyphényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une meringue blanche dont les caractéristiques sont les suivantes :
- point de fusion = 170-175°C
- spectre de masse (E.I.) M/Z = 668 (M⁺).

On agite pendant 48 heures à une température voisine de 20°C 460 mg (0,687 mmole) de N-[(S)-t.butyl-phénylglycinoyl)]-[4,9-éthano-2-(2-méthoxyphényl prop-2-én-1-oyl]-9-phényl-2,3,3a,4,9,9a-hexahydro-1H-benzo[f]isoindole-3a-carboxamide-(3aS, 4R, 9R, 9aS) et 1,6 cm3 (2,06 mmole) d'acide trifluoroacétique en solution dans 10 cm3. Après concentration sous pression réduite, le résidu est repris par 10 cm3 d'éther de pétrole. Le précipité formé est essoré, puis recristallisé dans de l'éthanol à 90 %. On obtient ainsi 330 mg (79 %) de N-[(S)-phénylglycinoyl)]-4,9-éthano-2-[(2-méthoxy-phényl)prop-2-én-1-oyl]-9-phényl- 2,3,3a,4,9,9a-hexahydro-1H-benzo[f]isoin-dole-3a-carboxamide-(3aS, 4R, 9R, 9aS), sous forme d'une poudre blanche dont les caractéristiques sont les suivantes :
- point de fusion = 205-210°C
- spectre de masse (E.I.) M/Z = 612 (M⁺).

### EXEMPLE 82

A partir de 200 mg (0,417 mmole) d'acide 4,9-éthano-2-[(2-méthoxyphényl) prop-2-én-1-oyl]-9-phényl-2,3,3a,4,9,9a-hexahydro-1H-benzo[f]isoindole-3a-carboxylique-(3aS, 4R, 9R, 9aS) (exemple 39), de 69 mg (0,5 mmole) de 2-méthoxybenzylamine, de 96 mg (0,5 mmole) de chlorhydrate de 1-éthyl-3-(3-diméthylamino-propyl)-carbodiimide et de 6 mg (0,045 mmole) d'hydroxybenzotriazole en solution dans 10 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (50-50 en volumes), 210 mg (84 %) de N-[2-méthoxybenzyl)]-4,9-éthano-2-[(2-méthoxy-phényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 114-116°C
- spectre de masse (E.I.) M/Z = 598 (M⁺).

### EXEMPLE 83

A partir de 200 mg (0,417 mmole) d'acide 4,9-éthano-2-[(2-méthoxyphényl)prop-2-én-1-oyl]-9-phényl-2,3,3a,4,9,9a-hexahydro-1H-benzo[f]isoindole-3a-carboxylique-(3aS, 4R, 9R, 9aS) (exemple 39), de 89 mg (0,5 mmole) de 1-(R)-(1-naphtyl)éthylamine, de 96 mg (0,5 mmole) de chlorhydrate de 1-éthyl-3-(3-diméthylamino-propyl)-carbodiimide et de 6 mg (0,045 mmole) d'hydroxybenzotriazole en solution dans 10 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (50-50 en volumes), 200 mg (77 %) de N-[1-(R)- (1-naphtyl)éthyl]-4,9-éthano-2-[(2-méthoxyphényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 139-143°C
- spectre de masse (E.I.) M/Z = 632 (M⁺).

### EXEMPLE 84

A partir de 250 mg (0,52 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 83 mg (0,625 mmole) de 1-(R,S)-aminoindane, de 120 mg (0,625 mmole) de chlorhydrate de 1-éthyl-3-(3-diméthylamino-propyl)-carbodiimide et de 7 mg (0,052 mmole) d'hydroxybenzotriazole en solution dans 10 cm3 de dichlorométhane, on obtient, après 18 heures d'agitation à une température voisine de 20°C et purification par flash-chromatogaphie sur gel de silice (230-400 mesh), en éluant avec du dichlorométhane contenant 0,25 % de méthanol, 270 mg (90 %) de N-[1-(R,S)-indanyl]-4,9-éthano-2-[(2-méthoxyphényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 174-175°C
- spectre de masse (E.I.) M/Z = 594 (M⁺).

### EXEMPLE 85

A partir de 250 mg (0,52 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-) carboxylique-(3aS, 4R, 9R, 9aS) (exemple 39), de 70 mg (0,52 mmole) de (S)-2-phénylglycinol, de 120 mg (0,625 mmole) de chlorhydrate de 1-éthyl-3-(3-diméthylamino-propyl)-carbodiimide et de 7 mg (0,052 mmole) d'hydroxybenzotriazole en solution dans 10 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (80-20 en volumes), 170 mg (55 %) de N-[1-(S)-1-phényl-2-hydroxy-éthyl]-4,9-éthano-2[-(2-méthoxyphényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 230-231°C
- spectre de masse (E.I.) M/Z = 598 (M⁺).

### EXEMPLE 86

A partir de 240 mg (0,5 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 81 mg (0,6 mmole) de cumylamine, de 115 mg (0,6 mmole) de chlorhydrate de 1-éthyl-3-(3-diméthylamino-propyl)-carbodiimide et de 13 mg (0,1 mmole) d'hydroxybenzotriazole en solution dans 10 cm3 de dichlorométhane, on obtient, après 18 heures d'agitation à une température voisine de 20°C et purification par flash-chromatogaphie sur gel de silice (230-400 mesh), en éluant avec des mélanges de cyclohexane et d'acétate d'éthyle (95-5 puis 80-20 en volumes), 69 mg (23 %) de N-[2,2-diméthylbenzyl]-4,9-éthano-2[-(2-méthoxy-phényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 142- 143°C
- spectre de masse (E.I.) M/Z = 596 (M⁺).

### EXEMPLE 87

A partir de 250 mg (0,52 mmole) d'acide 4,9-éthano-2-[(2-méthoxyphényl) prop-2-én-1-oyl]-9-phényl-2,3,3a,4,9,9a-hexahydro-1H-benzo[f]isoindole-3a-carboxylique-(3aS, 4R, 9R, 9aS) (exemple 39), de 84 mg (0,52 mmole) de (R,S)-2-phénylglycinonitrile, de 115 mg (0,6 mmole) de chlorhydrate de 1-éthyl-3-(3-diméthylamino-propyl)-carbodiimide et de 10 mg (0,075 mmole) d'hydroxybenzotriazole en solution dans 10 cm3 de dichlorométhane, on obtient, après 18 heures d'agitation à une température voisine de 20°C et purification par flash-chromatogaphie sur gel de silice (230-400 mesh), en éluant avec des mélanges cyclohexane-acétate d'éthyle (80-20 puis 50-50 en volumes), 70 mg (23 %) de N-[1-(R,S)-1-cyano-1-phényl-méthyl]-4,9-éthano-2-[(2-méthoxyphényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 98-100°C
- spectre de masse (E.I.) M/Z = 593 (M⁺).

### EXEMPLE 88

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 100 mg (0,5 mmole) de chlorhydrate de (R,S)-4-amino-benzo[b]thiopyranne, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 10 cm3 de dichlorométhane, on obtient, après 18 heures d'agitation à une temperature voisine de 20°C et purification par flash-chromatogaphie sur gel de silice (230-400 mesh), en éluant avec un mélange cyclohexane-acétate d'éthyle (60-40 en volumes), 170 mg (65 %) de N-[(R,S)-4-benzo[b]thiopyrannyl]-4,9-éthano-2-[(2-méthoxyphényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 143-146°C
- spectre de masse (E.I.) M/Z = 626 (M⁺).

### EXEMPLE 89

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 88 mg (0,5 mmole) de 3-trifluorobenzylamine, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (50-50 en volumes), 217 mg (81 %) de N-[3-trifluorobenzyl]-4,9-éthano-2-[(2-méthoxyphényl)prop-2-én-1-oyl)-9-phényl-2,3,3a,4,9,9a-hexahydro-1H-benzo[f]isoindole-3a-carboxamide-(3aS, 4R, 9R, 9aS), sous forme d'une poudre jaune-pâle dont les caractéristiques sont les suivantes :
- point de fusion = 118-120°C
- spectre de masse (E.I.) M/Z = 636 (M ⁺).

### EXEMPLE 90

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 84 mg (0,5 mmole) de vératrylamine, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (80-20 en volumes), 190 mg (73 %) de N-[3,4-diméthoxybenzyl]-4,9-éthano-2-[(2-méthoxyphényl)prop-2-én-1-oyl]-9-phényl-2,3,3a,4,9,9a-hexahydro-1H-benzo[f]isoindole-3a-carboxamide-(3aS, 4R, 9R, 9aS), sous forme d une poudre blanche dont les caractéristiques sont les suivantes :
- point de fusion = 102-105°C
- spectre de masse (E.I.) M/Z = 628 (M⁺).

### EXEMPLE 91

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 54 mg (0,5 mmole) de 3-picolylamine, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec un mélange acétate d'éthyle-méthanol (90-10 en volumes), 176 mg (74 %) de N-[(3-pyridyl)méthyl]-4,9-éthano-2-[(2-méthoxy-phényl)prop-2-én-1-oyl]-9-phényl-2,3,3a,4,9,9a-hexahydro-1H-benzo[f]isoindole-3a-carboxamide-(3aS, 4R, 9R, 9aS), sous forme d'une poudre blanche dont les caractéristiques sont les suivantes :
- point de fusion = 150-154°C
- spectre de masse (E.I.) M/Z = 569 (M⁺).

### EXEMPLE 92

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 88 mg (0,5 mmole) de 4-trifluorobenzylamine, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (60-40 en volumes). 200 mg (77 %) de N-[4-trifluorobenzyl]-[4,9-éthano-2-(2-méthoxyphényl)prop-2-én-1-oyl]-9-phényl-2,3,3a, 4,9,9a-hexahydro-1H-benzo[f]isoindole-3a-carboxamide-(3aS, 4R, 9R, 9aS), sous forme d'une poudre jaune-pâle dont les caractéristiques sont les suivantes :
- point de fusion = 149-151°C
- spectre de masse (E.I.) M/Z = 636 (M⁺).

### EXEMPLE 93

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 54 mg (0,5 mmole) de 2-picolylamine, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec des mélanges acétate d'éthyle-méthanol (95-5 puis 90-10 en volumes), 210 mg (91 %) de N-[(2-pyridyl)méthyl]-4,9-éthano-2-[(2-méthoxy-phényl)prop-2-én-1-oyl]-9-phényl-2,3,3a,4,9,9a-hexahydro-1H-benzo[f]isoin-dole-3a-carboxamide-(3aS, 4R, 9R, 9aS), sous forme d'une poudre blanche dont les caractéristiques sont les suivantes :
- point de fusion = 137-139°C
- spectre de masse (E.I.) M/Z = 569 (M⁺).

### EXEMPLE 94

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 49 mg (0,5 mmole) de furfurylamine, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (50-50 en volumes), 200 mg (87 %) de N-[(2-furyl)méthyl]-4,9-éthano-2-[(2-méthoxyphényl)prop-2-én-1-oyl]-9-phényl-2,3,3a, 4,9,9a-hexahydro-1H-benzo[f]isoindole-3a-carboxamide-(3aS, 4R, 9R, 9aS), sous forme d'une poudre jaune orangée dont les caractéristiques sont les suivantes :
- point de fusion = 170-171°C
- spectre de masse (E.I.) M/Z = 558 (M⁺).

### EXEMPLE 95

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 56 mg (0,5 mmole) de 2-aminométhyl-thiophène, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (50-50 en volumes), 180 mg (75 %) de N-[(2-thiényl)méthyl]-4,9-éthano-2-[(2-méthoxy-phényl)prop-2-én-1-oyl]-9-phényl-2,3,3a, 4,9,9a-hexahydro-1H-benzo[f]isoindole-3a-carboxamide-(3aS, 4R, 9R, 9aS), sous forme d'une poudre beige dont les caractéristiques sont les suivantes :
- point de fusion = 133-135°C
- spectre de masse (E.I.) M/Z = 574 (M⁺).

### EXEMPLE 96

A partir de 200 mg (0,42 mmole) d'acide 4,9-éthano-2-[(2-méthoxyphényl)prop-2-én-1-oyl]-9-phényl-2,3,3a,4,9,9a-hexahydro-1H-benzo[f] isoindole-3a-carboxylique-(3aS, 4R, 9R, 9aS) (exemple 39), de 56 mg (0,5 mmole) de 4-aminométhyl-thiazole, de 96 mg (0,5 mmole) de chlorhydrate de 1-éthyl-3-(3-diméthylamino-propyl)-carbodiimide et de 6 mg (0,042 mmole) d'hydroxybenzotriazole en solution dans 15 cm3 de dichlorométhane, on obtient, après 18 heures d'agitation à une température voisine de 20°C et purification par flash-chromatogaphie sur gel de silice (230-400 mesh), en éluant avec un mélange cyclohexane-acétate d'éthyle (60-40 en volumes), 115 mg (48%) de N-[(4-thiazolyl)méthyl]-4,9-éthano-2-[(2-méthoxy-phényl)prop-2-én-1-oyl]-9-phényl- 2,3,3a,4,9,9a-hexahydro-1H-benzo[f]isoindole-3a-carboxamide-(3aS, 4R, 9R, 9aS), sous forme d'une poudre beige dont les caractéristiques sont les suivantes :
- point de fusion = 136-138°C
- spectre de masse (E.I.) M/Z = 573 (M⁺).

Le 4-aminométhyl-thiazole peut être préparé selon Zhur. Obshchei. Khim., 31, 1356-1361 (1961).

### EXEMPLE 97

A partir de 2 g (3,6 mmoles) d'ester méthylique de l'acide 4,9-éthano-6-iodo-9-phényl-2-(t.butoxycarbonyl)-2,3,3a,4,9,9a-hexahydro-1H-benzo[f]isoindole-3a-carboxylique-(3aRS, 4SR, 9SR, 9aRS), obtenu comme dans l'exemple 43, de 1,3 cm3 de tri-n.butyl-vinyl-stannane et de 310 mg (0,27 mmole) de tétrakis (triphénylphosphine)-palladium dans 65 cm3 de diméthylformamide en opérant comme à l'exemple 73, on obtient, après purification par flash-chromatogaphie sur gel de silice (230-400 Mesh), en éluant avec un mélange cyclohexane-acétate d'éthyle (95-5 en volumes), 740 mg (45 %) d'ester méthylique de l'acide 4,9-éthano-9-phényl-2-(t.butoxy-carbonyl)-6-vinyl-2,3,3a,4,9,9a-hexahydro-1H-benzo[f]isoindole-3a-carboxylique-(3aRS, 4SR, 9SR, 9aRS), sous forme d'une meringue jaune pâle dont les caractéristiques sont les suivantes :
- spectre de masse (E.I.) M/Z = 459 (M⁺).

A partir de 280 mg (0,62 mmole) d'ester méthylique de l'acide 4,9-éthano-9-phényl-2-(t.butoxycarbonyl)-6-vinyl-2,3,3a,4,9,9a-hexahydro-1H-benzo[f]isoindole-3a-carboxylique-(3aRS, 4SR, 9SR, 9aRS) dans un excès d'acide chlorhydrique dans le méthanol, on obtient 235 mg (98 % ) du chlorhydrate de l'ester méthylique de l'acide 4,9-éthano-9-phényl-6-vinyl-2,3,3a,4,9,9a-hexahydro-1H-benzo[f]isoindole-3a-carboxylique(3aRS, 4SR, 9SR, 9aRS), sous forme d'une poudre blanche dont les caractéristiques sont les suivantes :
- spectre de masse (E.I.) M/Z = 395 (M⁺).

A partir de 230 mg (0,58 mmole) d'ester méthylique de l'acide 4,9-éthano-9-phényl-6-vinyl-2,3,3a,4,9,9a-hexahydro-1H-benzo[f]isoindole-3a-carboxylique-(3aRS, 4SR, 9SR, 9aRS), de 116 mg (0,7 mmole) d'acide 2-méthoxyphénylacétique, de 137 mg (0,7 mmole) de chlorhydrate de 1-éthyl-3-(3-diméthylamino-propyl)-carbodiimide et de 8 mg (0,06 mmole) d'hydroxybenzotriazole en solution dans 350 cm3 de dichlorométhane, on obtient, après 18 heures d'agitation à une température voisine de 20°C et purification du produit brut obtenu par flash-chromatographie sur gel de silice (230-400 mesh), en éluant avec du dichlorométhane, 260 mg (88 %) d'ester méthylique de l'acide 4,9-éthano-2-[(2-méthoxyphenyl)acétyl]-9-phényl-6-vinyl-2,3,3a,4,9,9a-hexahydro-1H-benzo[f] isoindole-3a-carboxylique-(3aRS, 4SR, 9SR, 9aRS), sous forme d'une poudre blanche dont les caractéristiques sont les suivantes :
- point de fusion = 54-56°C
- spectre de masse (D.C.I. : NH₃) M ³/Z = 507 (MH⁺).

Dans un tricol de 100 cm3 relié à un ozoniseur, on dissous 350 mg (0,69 mmole) d'ester méthylique de l'acide 4,9-éthano-2-[(2-méthoxyphenyl)acétyl]-9-phényl-6-vinyl-2,3,3a,4,9,9a-hexahydro-1H-benzo[f]isoindole-3a-carboxylique (3aRS, 4SR, 9SR, 9aRS) dans 35 cm3 de dichlorométhane puis on ajoute 0,35 cm3 de méthanol et on refroidit à -70°C. On fait alors barbotter de l'ozone à -70°C, pendant environ 1 heure 45 minutes, jusqu'à l'apparition d'une coloration bleutée stable. Après 1 heure d'agitation supplémentaire à cette température, on fait barbotter de l'air comprimé pour chasser l'excès d'ozone, puis on ajoute 170 mg (2,8 mmoles) de sulfure de diméthyl et on laisse revenir à température ambiante. Après concentration sous pression réduite, le résidu huileux est repris par de l'acétate d'éthyle, lavé à l'eau jusqu'à neutralité. Après séchage sur sulfate de magnésium et concentration sous pression réduite, l'huile jaune obtenue est purifiée par flash-chromatographie sur gel de silice (230-400 mesh) en éluant par un mélange cyclohexane-acétate d'éthyle (80-20 en volumes). On obtient ainsi 68 mg (19 %) d'ester méthylique de l'acide 4,9-éthano-6-formyl-2-[(2-méthoxyphenyl)acétyl]-9-phényl-2.3,3a,4,9,9a-hexahydro-1H-benzo[f]isoindole-3a-carboxylique-(3aRS, 4SR, 9SR, 9aRS), sous forme d'une poudre blanche dont les caractéristiques sont les suivantes :
- point de fusion = 109-110°C
- spectre de masse (D.C.I. : NH₃) M/Z = 509 (MH⁺).

L'activité inhibitrice de la farnésyltransférase et de la farnésylation de la protéine Ras peut être mise en évidence dans le test suivant :

L'activité farnésyltransférase est déterminée par la quantité de (³H) farnésyl transférée à partir du (³H) farnésylpyrophosphate [(³H) FPP] sur la protéine p21 H-ras. Le mélange réactionnel standard est composé, pour un volume final de 60 ml, de Tris-HCl 50mM, MgCl₂ 5mM, dithiotréitol 5 mM, octyl-b-D-glucopyranoside 0,2 %, p21 H-ras 200 picomoles, (³H) FPP (à 61000 dpm/picomole) 4,5 picomoles.

La réaction est initiée par addition d'environ 5 ng de farnésyltransférase humaine purifiée à partir de cultures de cellules THP1. Après incubation pendant 20 minutes à 37°C en plaque de microtitration contenant 96 trous de 1 cm3 par plaque (Titer Plate®, Beckman), la réaction est stoppée par addition de 0,4 cm3 de SDS à 0,1 % dans le méthanol à 0°C. Le mélange est ensuite additionné de 0,4 cm3 d'acide ®trichloroacétique (TCA) à 30 % dans le méthanol. Les plaques sont laissées pendant 1 heure dans la glace. Le contenu précipité est alors retenu sur membrane en fibre de® verte Filtermat®, Pharmacia) avec l'unité de filtration (Combi Cell Harvester®, Skatron) et rincé avec de l'acide trichloroacétique à 6 % dans l'eau distillée. Les membranes sont séchées au four à micro-ondes puis imprégnées de scintillant par fusion sous air chaud de Meltilex® (Pharmacia) et enfin comptées en cpm dans un compteur b-Plate® (LKB). Chaque essai est répété 3 fois.

L'unité d'activité est définie par 1 picomole de (³H) FPP transférée sur p21 H-ras en 20 minutes.

Les pourcentages d'inhibition sont obtenus par comparaison des essais avec et sans inhibiteur après déduction des blancs, les IC₅₀ étant mesurées à partir des inhibitions obtenues avec 9 concentrations différentes en utilisant les logiciels Enzfitter® ou Grafit®.

Les résultats obtenus sont rassemblés dans le tableau I.

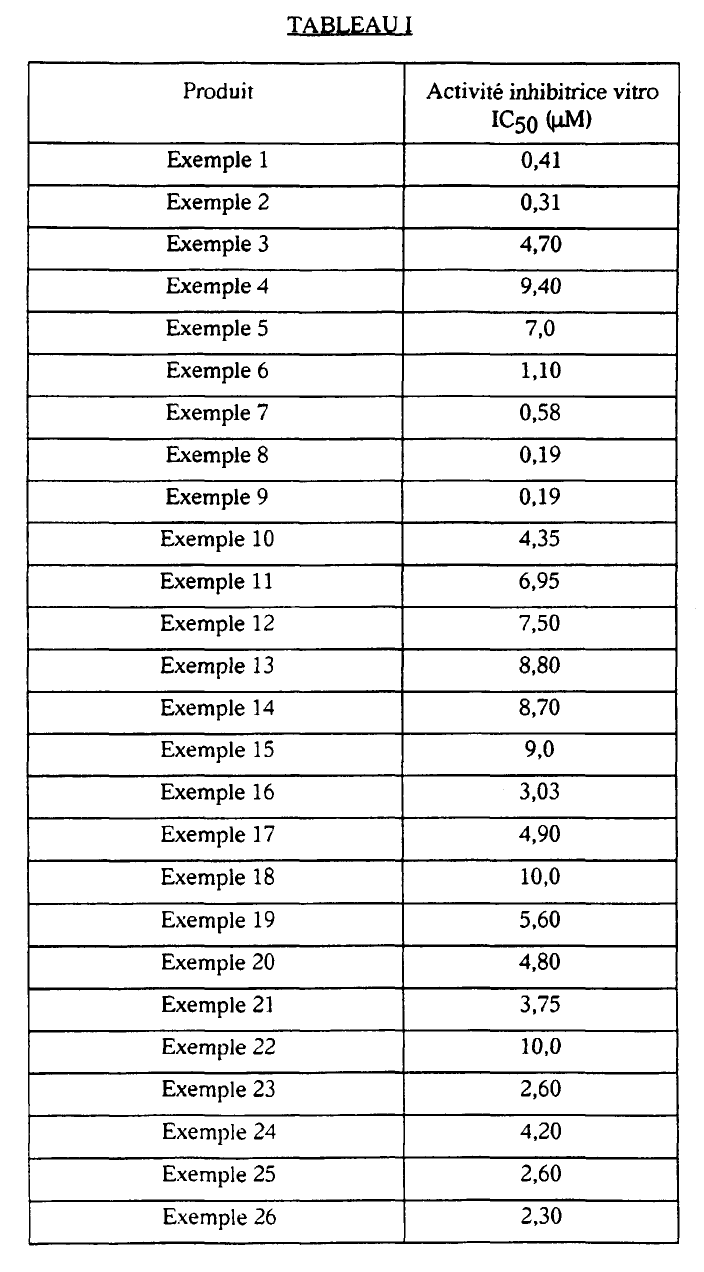

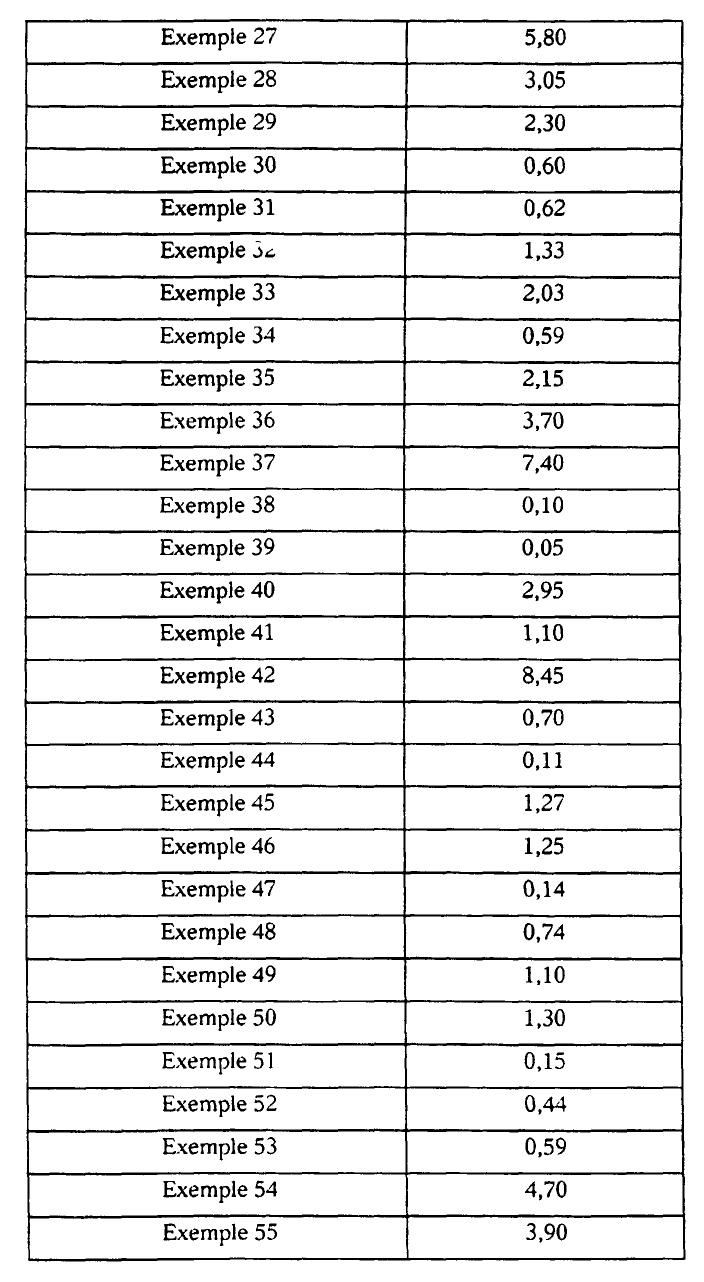

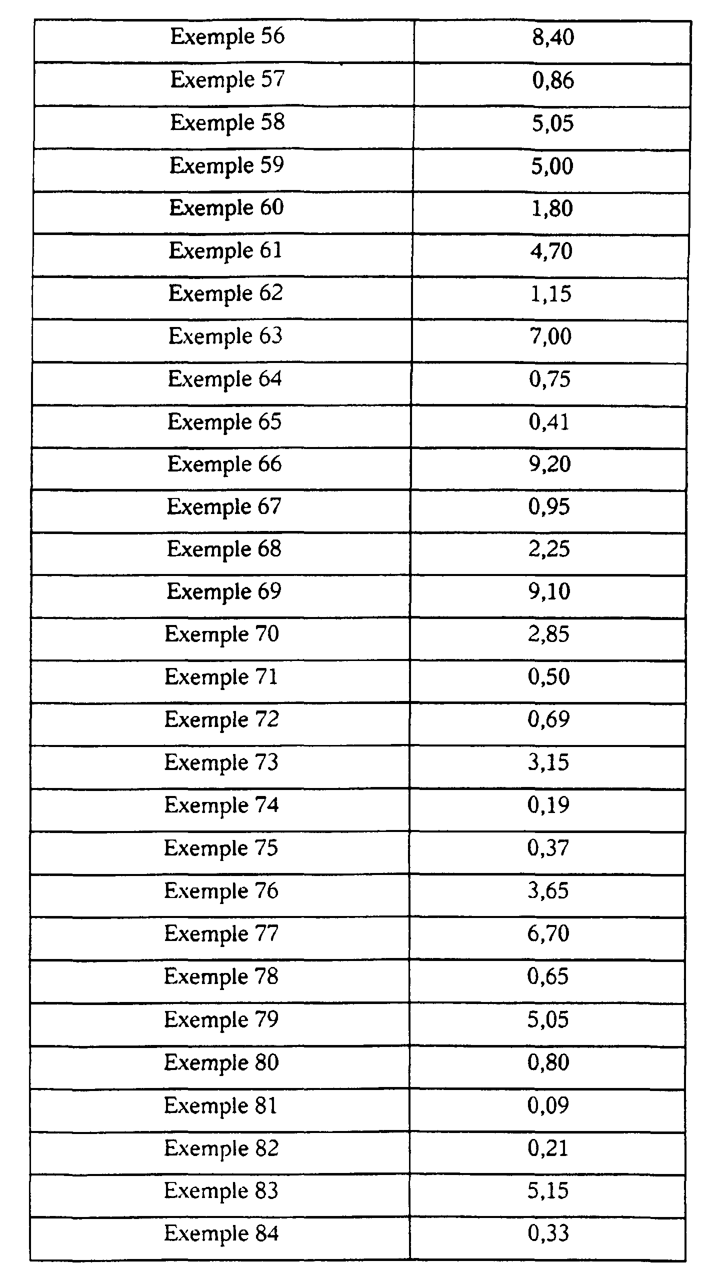

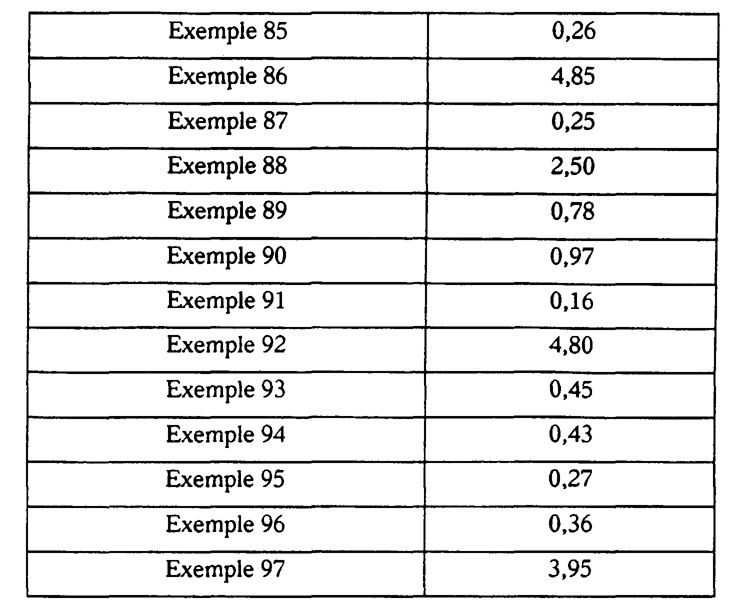

L'activité sur cellules peut être déterminée de la manière suivante :

La lignée cellulaire est la lignée THAC (cellules CCL 39 transfectées par Ha-Ras activé) selon K. Seuwen et coll., EMBO J., 7(1) 161-168 (1988). Les cellules sont ensemencées dans des boîtes de Petri de 6 cm de diamètre contenant un milieu DMEM, sérum de veau foetal 5 %, G418 1 %.

Après 24 heures de culture, le milieu de culture est changé (avec ou sans sérum) et le produit à étudier est ajouté en solution dans le diméthylformamide (DMF), en présence ou en absence de DTT (concentrations finales de 0,5 % en DMF et 0,1mM en DTT). Après 24 heures de culture à 37°C, les cellules sont lysées dans 1 cm3 de tampon de lyse (Tris, HCl 20mM, Triton X114 1 %, MgCl₂ 5 mM, DTT 7 mM, NaCl 150 mM, pH = 7,4). Les lysats sont clarifiés par centrifugation à 4.000 tours/minute pendant 10 minutes. L'extraction par le Triton X114 permet de séparer la protéine Ras farnésylée de la protéine Ras non farnésylée [C. BORDIER, J. Biol. Chem., 256 (4), 1604-1607 (1981)]. La protéine Ras farnésylée qui est plus hydrophobe se trouve dans la phase détergente tandis que la protéine Ras non farnésylée est dans la phase aqueuse. Les échantillons sont dénaturés par chauffage à 95°C dans du tampon de dénaturation pour électrophorèse et déposées sur un gel de polyacrylamide à 14 %. Lorsque le colorant atteint le bas du gel, les protéines du gel sont transférées sur une membrane PVDF. La protéine Ras est révélée par la technique de Western blot : la membrane est incubée avec un anticorps monoclonal spécifique anti-Ras (pan-Ras Ab3, Oncogène Science) puis avec de la protéine A marquée à ¹²⁵I. Après autoradiographie, les bandes sont identifiées, découpées et comptées dans un compteur γ. La radioactivité des bandes correspondant à Ras farnésylée et à Ras non farnésylée permet de déterminer le pourcentage d'inhibition de la farnésylation de la protéine Ras.

Les produits selon l'invention inhibent de 50 % la farnésylation de la protéine Ras à des concentrations comprises entre 100 nM et 100 µM.

Les nouveaux produits de formule générale (I) peuvent se présenter sous forme de sels non toxiques et pharmaceutiquement acceptables. Ces sels non toxiques comprennent les sels avec les acides minéraux (acides chlorhydrique, sulfurique, bromhydrique, phosphorique, nitrique) ou avec les acides organiques (acides acétique, propionique, succinique, maléique, hydroxymaléique, benzoïque, fumarique, méthanesulfonique, trifluoroacétique ou oxalique) ou avec les bases minérales (soude, potasse, lithine, chaux) ou organiques (amines tertiaires comme la triéthylamine, la pipéridine, la benzylamine) selon la nature des aminoacides qui constituent le peptide de formule générale (I).

Les nouveaux produits selon l'invention, qui inhibent la farnésyltransférase et la farnésylation de la protéine Ras, sont des agents anticancéreux remarquables qui agissent tant au niveau des tumeurs solides que liquides.

D'un intérêt tout particulier sont les produits de formule générale (I) dans laquelle l'un des symboles R₁ et R₂ représente un atome d'hydrogène et l'autre représente un radical alcoyloxy contenant 1 à 4 atomes de carbone, R₃ représente un atome d'hydrogène, X représente un radical méthylène, Y représente un atome d'oxygène et R représente un radical de formule générale -(CH₂)ₘ-X₁-(CH₂)ₙ-Z dans laquelle m est égal à 0 ou 1, X₁ représente une liaison et n est égal à 0 et Z représente un radical carboxy ou un radical -COOR₄ pour lequel R₄ représente un radical alcoyle contenant 1 à 6 atomes de carbone ou un radical CON(R₅)(R₆) dans lequel R₅ et R₆ sont définis comme précédemment.

Plus particulèrement encore peuvent être cités l'acide 4,9-éthano-2-[2-(2-méthoxyphényl)prop-2-én-1-oyl]-9-phényl]-1,2,3,3a,9,9a-hexahydro-1H-benzo[f] soindole-3a-carboxylique-(3aS, 4R, 9R, 9aS), énantiomère dextrogyre, éventuellement sous forme d'ester contenant 1 à 3 atomes de carbone, d'amide ou d'amide N-substitué et le N-[(S)-t.butyl-phénylglycinoyl)]-4,9-éthano-2-[(2-méthoxyphényl) prop-2-én-1-oyl]-9-phényl-2,3,3a,4,9,9a-hexahydro-1H-benzo[f]isoindole-3a-carboxamide-(3aS, 4R, 9R, 9aS).

La présente invention concerne également les compositions pharmaceutiques qui contiennent au moins un produit de formule générale (I) en association avec un ou plusieurs diluants ou adjuvants pharmaceutiquement acceptables qu'ils soient inertes ou physiologiquement actifs.

Ces compositions peuvent être administrées par voie orale, parentérale ou rectale.

Les compositions pour administration orale comprennent des comprimés, des pilules, des poudres ou des granulés. Dans ces compositions le produit actif selon l'invention est mélangé à un ou plusieurs diluants inertes tels que saccharose, lactose ou amidon. Ces compositions peuvent comprendre des substances autres que les diluants, par exemple un lubrifiant tel que le stéarate de magnésium.

Comme compositions liquides pour administration orale peuvent être utilisées des émulsions pharmaceutiquement acceptables, des solutions, des suspensions, des sirops des élixirs contenant des diluants inertes tel que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants ou par chauffage. Elles peuvent être également préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao.

Les compositions selon l'invention sont particulièrement utiles en thérapeutique humaine dans le traitement de cancers d'origines diverses.

En thérapeutique humaine, les doses dépendent de l'effet recherché, de la durée du traitement et des facteurs propres au sujet à traiter.

Généralement, les doses sont comprises, chez l'homme, entre 0,1 et 20 mg/kg par jour par voie intra-péritonéale.

## Revendications

1. Produits de formule générale : 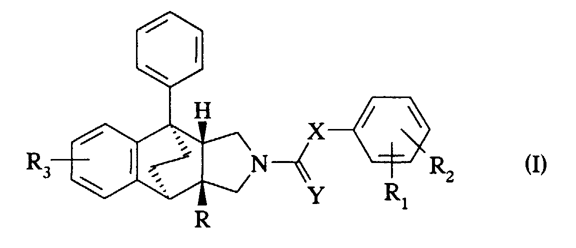 dans laquelle :
R représente :
- un radical de formule générale -(CH₂)ₘ-X₁-(CH₂)ₙ-Z dans lequel
- X₁ représente une simple liaison ou un atome d'oxygène ou de soufre, m représente un nombre entier égal à 0 ou 1 et n représente un nombre entier égal à 0, 1 ou 2, les radicaux méthylènes pouvant être substitués par un radical carboxy, alcoxycarbonyle dont la partie alcoyle contient 1 à 4 atomes de carbone, carbamoyle, alcoylcarbamoyle dont la partie alcoyle contient 1 à 4 atomes de carbone, dialcoylcarbamoyle dont chaque partie alcoyle contient 1 à 4 atomes de carbone, amino, alcoylamino contenant 1 à 4 atomes de carbone, dialcoylamino dont chaque partie alcoyle contient 1 à 4 atomes de carbone et
- Z représente
- un radical carboxy,
- un radical COOR₄, dans lequel R₄ représente un radical alcoyle droit ou ramifié contenant 1 à 3 atomes de carbone, ou
- un radical CON(R₅)(R₆) dans lequel R₅ représente un atome d'hydrogène ou un radical alcoyle droit ou ramifié contenant 1 à 6 atomes de carbone et R₆ représente un atome d'hydrogène ou un radical alcoyle droit ou ramifié contenant 1 à 6 atomes de carbone éventuellement substitué par un radical amino, alcoylamino contenant 1 à 4 atomes de carbone, dialcoylamino dont chaque partie alcoyle contient 1 à 4 atomes de carbone, hydroxy, alcoxy contenant 1 à 4 atomes de carbone, mercapto, alcoylthio contenant 1 à 4 atomes de carbone, alcoyloxycarbonyle dont la partie alcoyle contient 1 à 4 atomes de carbone, carboxy, cyano, phényle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone, dialcoylamino dont chaque partie alcoyle contient 1 à 4 atomes de carbone, trifluorométhyle ou cyano, naphtyl-1 ou -2, furyl-2 ou -3, thiényl-2 ou -3, imidazolyl-4 ou -5, thiazolyl-2, -4 ou - 5, thiazolidinyl-2, -4 ou -5 ou pyridyl-2, -3 ou -4, ou un radical indanyle ou thiochromanyle ou bien R₅ représente un atome d'hydrogène ou un radical alcoyle droit ou ramifié contenant 1 à 6 atomes de carbone et R₆ représente un radical hydroxy, amino ou alcoyloxy contenant 1 à 6 atomes de carbone en chaîne droite ou ramifiée éventuellement substitué par un radical phényle, ou
- un radical PO(OR₇)₂ dans lequel R₇ représente un atome d'hydrogène ou un radical alcoyle droit ou ramifié contenant 1 à 6 atomes de carbone, ou
- un radical -NH-CO-T dans lequel T représente un atome d'hydrogène ou un radical alcoyle droit ou ramifié contenant 1 à 6 atomes de carbone éventuellement substitué par un radical amino, carboxy, alcoyloxycarbonyle dont la partie alcoyle contient 1 à 4 atomes de carbone, hydroxy, alcoyloxy contenant 1 à 4 atomes de carbone, mercapto ou alcoylthio contenant 1 à 4 atomes de carbone, ou bien
- un radical 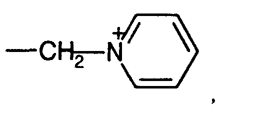
R₁ et R₂, identiques ou différents, représentent un atome d'hydrogène ou d'halogène ou un radical alcoyle contenant 1 à 4 atomes de carbone, alcoyloxy contenant 1 à 4 atomes de carbone éventuellement substitué par un radical dialcoylamino dont chaque partie alcoyle contient 1 à 4 atomes de carbone ou forme avec l'atome d'azote un hétérocycle saturé contenant 5 ou 6 atomes de carbone, alcoylthio contenant 1 à 4 atomes de carbone, alcoyloxycarbonyle dont la partie alcoyle contient 1 à 4 atomes de carbone, ou bien R₁ et R₂, situés en ortho l'un par rapport à l'autre, forment un hétérocycle saturé ou non saturé contenant 1 ou 2 hétéroatomes choisis parmi l'azote et l'oxygène, éventuellement substitué par un atome d'halogène ou par un radical alcoyle contenant 1 à 4 atomes de carbone ou alcoyloxy contenant 1 à 4 atomes de carbone, ou bien R₁ représente un atome d'hydrogène ou d'halogène ou un radical alcoyle contenant 1 à 4 atomes de carbone, alcoyloxy contenant 1 à 4 atomes de carbone éventuellement substitué par un radical dialcoylamino dont chaque partie alcoyle contient 1 à 4 atomes de carbone, alcoylthio contenant 1 à 4 atomes de carbone, alcoyloycarbonyle dont la partie alcoyle contient 1 à 4 atomes de carbone, et R₂ représente un radical thioalcoyle contenant 1 à 4 atomes de carbone, étant entendu que dans ce cas le produit de formule générale (I) se présente sous forme d'un produit doublé par l'intermédiaire d'un pont disulfure,
R₃ représente un atome d'hydrogène ou d'halogène ou un radical alcoyle contenant 1 à 4 atomes de carbone, alcényle contenant 2 à 4 atomes de carbone, alcoyloxy contenant 1 à 4 atomes de carbone ou alcoylthio contenant 1 à 4 atomes de carbone, X représente un atome d'oxygène ou de soufre ou un groupement -NH-, -CO-, méthylène, éthylène, alcoylidène tel que vinylidène ou 1,1-cycloalcoyle contenant 3 à 6 atomes de carbone, et
Y représente un atome d'oxygène ou de soufre,
sous forme racémique ainsi que les isomères optiques du produit de formule générale (I).

2. Produits selon la revendication 1 pour lesquels l'un des symboles R₁ et R₂ représente un atome d'hydrogène et l'autre représente un radical alcoyloxy contenant 1 à 4 atomes de carbone, R₃ représente un atome d'hydrogène, X représente un radical méthylène, Y représente un atome d'oxygène et R représente un radical de formule générale -(CH₂)ₘ-X₁-(CH₂)ₙ-Z dans laquelle m est égal à 0 ou 1, X₁ représente une liaison et n est égal à 0 et Z représente un radical carboxy ou unradical -COOR₄ pour lequel R₄ représente un radical alcoyle contenant 1 à 3 atomes de carbone ou un radical CON(R₅)(R₆) dans lequel R₅ et R₆ sont définis comme dans la revendication 1.

3. Produit selon la revendication 1 caractérisé en ce qu'il s'agit de l'acide 4,9-éthano-2-[2-(2-méthoxyphényl)prop-2-én-1-oyl]-9-phényl-1,2,3,3a,9,9a-hexahydro-1H-benzo[f]isoindole-3a-carboxylique-(3aS, 4R, 9R, 9aS), énantiomère dextrogyre, éventuellement sous forme d'ester contenant 1 à 3 atomes de carbone.

4. Produit selon la revendication 1 caractérisé en ce qu'il s'agit du N-[(S)-t.butyl-phénylglycinoyl)]-4,9-éthano-2-[(2-méthoxyphényl) prop-2-én-1-oyl]-9-phényl-2,3,3a,4,9,9a-hexahydro-1H-benzo[f]isoindole-3a-carboxamide-(3aS, 4R, 9R, 9aS).

5. Produit selon la revendication 1 caractérisé en ce qu'il s'agit du N-[(3-pyridyl)méthyl]-4,9-éthano-2-[(∼-méthoxy-phényl)prop-2-én-1-oyl]-9-phényl-2,3,3a,4,9,9a-hexahydro-1H-benzo[f]-isoindole-3a-carboxamide-(3aS, 4R, 9R, 9aS).

6. Produit selon la revendication 1 caractérisé en ce qu'il s'agit du N-[(2-pyridyl)méthyl]-4,9-éthano-2-[(2-méthoxyphényl)prop-2-én-1-oyl]-9-phényl-2,3,3a,4,9,9a-hexahydro-1H-benzo[f]isoindole-3a-carboxamide- (3aS,4R,9R,9aS).

7. Procédé de préparation d'un produit selon l'une des revendications 1, 2, 3, 4, 5 ou 6 pour lesquels R, R₁, R₂ et R₃ sont définis comme dans l'une des revendications 1 ou 2, Y représente un atome d'oxygène ou de soufre et X représente un groupement -CO-, méthylène, éthylène, alcoylidène ou 1,1-cycloalcoyle, caractérisé en ce que l'on fait agir un acide de formule générale : 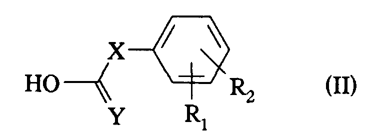 dans laquelle R₁, R₂ et X sont définis comme précédemment et Y représente un atome d'oxygène ou de soufre, ou son ester méthylique ou d'un dérivé de cet acide tel qu'un halogénure ou l'anhydride, sur un produit de formule générale : 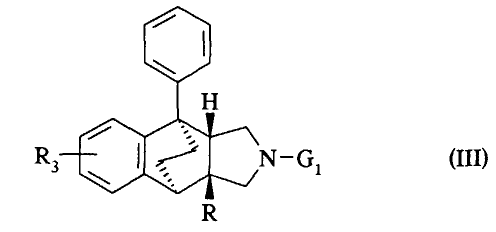 dans laquelle R et R₃ sont définis comme précédemment et G₁ représente un atome d'hydrogène, puis éventuellement, lorsque R représente ou contient un radical -COOR₄ ou -PO(OR₇)₂ dans lesquels R₄ et R₇ représentent un radical alcoyle, de la saponification du produit obtenu pour obtenir un produit de formule générale (I) dans laquelle R représente ou contient un radical carboxy ou transforme au moyen d'agent nucléophile le produit obtenu en un produit de formule générale (I) dans laquelle R représente ou contient un radical -PO₃H₂.

8. Procédé de préparation d'un produit selon l'une des revendications 1 ou 2 pour lequel Y représente un atome d'oxygène ou de soufre et X représente un atome d'oxygéne, caractérisé en ce que l'on fait réagir un halogénoformiate ou halogénothioformiate de formule générale : 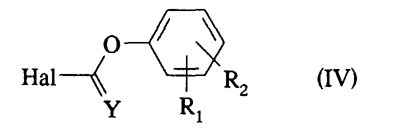 dans laquelle Y représente un atome d'oxygène ou de soufre, R₁ et R₂ sont définis comme dans l'une des revendications 1 ou 2 et Hal représente un atome d'halogène, sur un produit de formule générale (III), puis éventuellement, lorsque R représente ou contient un radical -COOR₄ ou -PO₃(R₇)₂ dans lesquels R₄ et R₇ représentent un radical alcoyle, saponifie le produit obtenu pour obtenir un produit de formule générale (I) dans laquelle R représente ou contient un radical carboxy ou transforme au moyen d'agent nucléophile le produit obtenu en un produit de formule générale (I) dans laquelle R représente ou contient un radical -PO₃H₂.

9. Procédé de préparation d'un produit selon l'une des revendications 1 ou 2 pour lequel Y représente un atome d'oxygène ou de soufre et X représente un groupement NH caractérisé en ce que l'on fait réagir un isocyanate ou un isothiocyanate de formule générale : 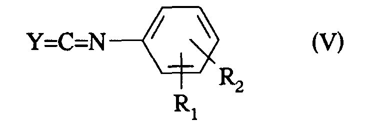 dans laquelle Y représente un atome d'oxygène ou de soufre, R₁ et R₂ sont définis comme dans l'une des revendications 1 ou 2 sur un produit de formule générale (III) dans laquelle G₁ représente un atome d'hydrogène, puis éventuellement, lorsque R représente ou contient un radical -COOR₄ ou -PO₃(R₇)₂ dans lesquels R₄ et R₇ représentent un radical alcoyle, saponifie le produit obtenu pour obtenir un produit de formule générale (I) dans laquelle R représente ou contient un radical carboxy ou transforme au moyen d'agent nucléophile le produit obtenu en un produit de formule générale (I) dans laquelle R représente ou contient un radical -PO₃H₂.

10. Procédé de préparation d'un produit selon l'une des revendications 1 ou 2 pour lequel R₁, R₂ et R₃ sont définis comme dans l'une des revendications 1 ou 2, X représente un atome d'oxygène ou de soufre ou un groupement -NH-, -CO-, méthylène, éthylène, alcoylidène ou 1,1-cycloalcoyle et Y représente un atome d'oxygène ou de soufre et R représente un radical de formule générale -(CH₂)ₘ-X₁-(CH₂)ₙ -Z dans lequel X₁, m et n sont définis comme dans l'une des revendications 1 ou 2 et Z représente un radical -COOR₄ dans lequel R₄ représente un radical alcoyle droit ou ramifié contenant 1 à 3 atomes de carbone caractérisé en ce que l'on estérifie un produit de formule générale (I) dans laquelle R₁, R₂, R₃, X, Y, X₁, m et n sont définis comme précédemment et Z représente un radical carboxy.

11. Procédé de préparation d'un produit selon la revendication 1 pour lequel R₁, R₂ et R₃ sont définis comme dans la revendication 1, X représente un atome d'oxygène ou de soufre ou un groupement -NH-, -CO-, méthylène, éthylène, alcoylidène ou 1,1-cycloalcoyle et Y représente un atome d'oxygène ou de soufre et R représente un radical de formule générale -(CH₂)ₘ-X₁-(CH₂)ₙ -Z dans laquelle X₁, m et n sont définis comme dans la revendication 1 et Z représente un radical -CON(R₅)(R₆) dans lequel lequel R₅ et R₆, identiques ou différents, représentent un atome d'hydrogène ou un radical alcoyle droit ou ramifié contenant 1 à 6 atomes de carbone et R₆ représente un atome d'hydrogène ou un radical alcoyle droit ou ramifié contenant 1 à 6 atomes de carbone éventuellement substitué par un radical amino, alcoylamino contenant 1 à 4 atomes de carbone, dialcoylamino dont chaque partie alcoyle contient 1 à 4 atomes de carbone, alcoxy contenant 1 à 4 atomes de carbone, alcoylthio contenant 1 à 4 atomes de carbone, alcoyloxycarbonyle dont la partie alcoyle contient 1 à 4 atomes de carbone, carboxy, cyano, phényle éventuellement substitué par un ou plusieurs radicaux identiques ou différents choisis parmi les radicaux alcoxy contenant 1 à 4 atomes de crabone ou trifluorométhyle, naphtyl-1 ou -2, furyl-2 ou 3, thiényl-2 ou -3, imidazolyl-4 ou -5 ou thiazolyl-4 ou -5, pyridyl-2, - 3 ou -4, ou un radical indanyle ou chromanyle, ou bien R₅ représente un atome d'hydrogène ou un radical alcoyle droit ou ramifié contenant 1 à 6 atomes de carbone et R₆ représente un radical hydroxy, amino ou alcoyloxy contenant 1 à 6 atomes de carbone en chaîne droite ou ramifiée éventuellement substitué par un radical phényl, caractérisé en ce que l'on fait réagir un produit de formule générale HN(R₅)(R₆) dans laquelle R₅ et R₆ sont définis comme ci-dessus sur un produit de formule générale (I) dans laquelle R₁, R₂, R₃, X, Y, X₁, m et n sont définis comme précédemment et Z représente un radical carboxy.

12. Procédé de préparation d'un produit selon la revendication 1 pour lequel R₁, R₂ et R₃ sont définis comme dans la revendication 1, X représente un atome d'oxygène ou de soufre ou un groupement -NH-, -CO-, méthylène, éthylène, alcoylidène ou 1,1-cycloalcoyle et Y représente un atome d'oxygène ou de soufre et R représente un radical de formule générale -NHCO-T dans laquelle T représente un atome d'hydrogène ou un radical alcoyle contenant 1 à 6 atomes de carbone éventuellement substitué par un radical amino, carboxy, alcoyloxycarbonyle, hydroxy, alcouyloxy, mercapto ou alcoylthio caractérisé en ce que l'on fait réagir un acide de formule générale T-CO-OH dans laquelle T est défini comme ci-dessus sur un produit de formule générale : 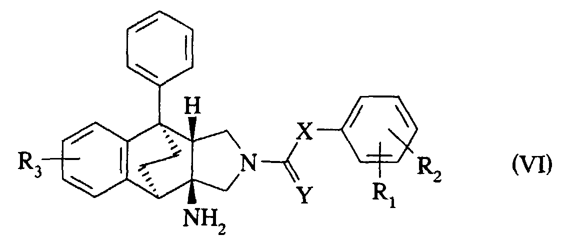 dans laquelle R₁, R₂, R₃, X et Y sont définis comme précédemment, puis éventuellement remplace les fonctions esters ou les fonctions amines protégées portées par T respectivement par des radicaux carboxy ou amino.

13. Procédé de préparation d'un produit selon la revendication 1 pour lequel R représente un radical de formule générale 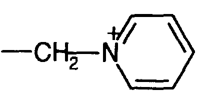 caractérisé en ce que l'on fait réagir un excès d'une amine tertiaire et d'un acide fort ou d'un dérivé de cet acide sur un produit de formule générale : 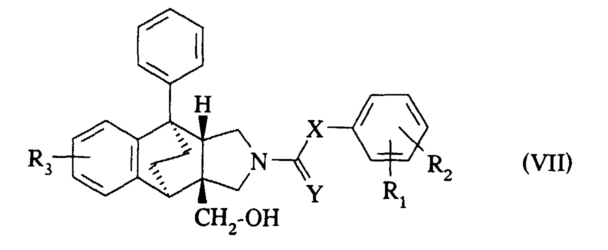 dans laquelle R₁, R₂, R₃, X et Y sont définis comme dans la revendication 1.

14. Procédé de préparation d'un produit selon la revendication 1 pour lequel Y représente un atome de soufre caractérisé en ce que l'on effectue la thionation d'un produit selon la revendication 1 pour lequel Y représente un atome d'oxygène.

15. Composition pharmaceutique caractérisée en ce qu'elle contient au moins un produit selon l'une des revendications 1, 2, 3, 4, 5 ou 6 en association avec un ou plusieurs diluants ou adjuvants pharmaceutiquements acceptables qu'ils soient inertes ou biologiquement actifs.

## Claims

1. Products of general formula: 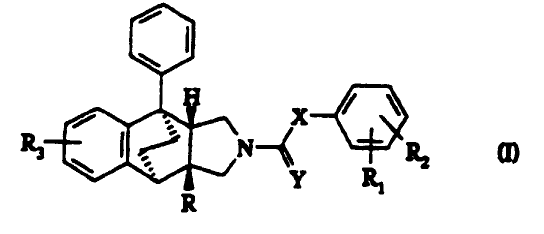 in which:
R represents:
- a radical of general formula -(CH₂)ₘ-X₁-(CH₂)ₙ-Z in which
- X₁ represents a single bond or an oxygen or sulphur atom, m represents an integer equal to 0 or 1 and n represents an integer equal to 0, 1 or 2, it being possible for the methylene radicals to be substituted by a carboxyl radical, an alkoxycarbonyl radical in which the alkyl part contains 1 to 4 carbon atoms, a carbamoyl radical, an alkylcarbamoyl radical in which the alkyl part contains 1 to 4 carbon atoms, a dialkylcarbamoyl radical in which each alkyl part contains 1 to 4 carbon atoms, an amino radical, an alkylamino radical containing 1 to 4 carbon atoms or a dialkylamino radical in which each alkyl part contains 1 to 4 carbon atoms and
- Z represents
- a carboxyl radical,
- a COOR₄ radical, in which R₄ represents a straight or branched alkyl radical containing 1 to 3 carbon atoms, or
- a CON(R₅)(R₆) radical in which R₅ represents a hydrogen atom or a straight or branched alkyl radical containing 1 to 6 carbon atoms and R₆ represents a hydrogen atom or a straight or branched alkyl radical containing 1 to 6 carbon atoms optionally substituted by an amino, alkylamino containing 1 to 4 carbon atoms, dialkylamino in which each alkyl part contains 1 to 4 carbon atoms, hydroxyl, alkoxy containing 1 to 4 carbon atoms, mercapto, alkylthio containing 1 to 4 carbon atoms, alkyloxycarbonyl in which the alkyl part contains 1 to 4 carbon atoms, carboxyl, cyano, phenyl optionally substituted by one or a number of identical or different atoms or radicals chosen from halogen atoms and alkyl radicals containing 1 to 4 carbon atoms, alkoxy radicals containing 1 to 4 carbon atoms, dialkylamino radicals in which each alkyl part contains 1 to 4 carbon atoms, trifluoromethyl radicals or cyano radicals, 1- or 2-naphthyl, 2- or 3-furyl, 2- or 3-thienyl, 4- or 5-imidazolyl, 2-, 4- or 5-thiazolyl, 2-, 4- or 5-thiazolidinyl or 2-, 3- or 4-pyridyl radical or an indanyl or thiochromanyl radical or else R₅ represents a hydrogen atom or a straight or branched alkyl radical containing 1 to 6 carbon atoms and R₆ represents a hydroxyl radical, an amino radical or an alkyloxy radical containing 1 to 6 straight- or branched-chain carbon atoms optionally substituted by a phenyl radical, or
- a PO(OR₇)₂ radical in which R₇ represents a hydrogen atom or a straight or branched alkyl radical containing 1 to 6 carbon atoms, or
- an -NH-CO-T radical in which T represents a hydrogen atom or a straight or branched alkyl radical containing 1 to 6 carbon atoms optionally substituted by an amino, carboxyl, alkyloxycarbonyl, in which the alkyl part contains 1 to 4 carbon atoms, hydroxyl, alkyloxy containing 1 to 4 carbon atoms, mercapto or alkylthio, containing 1 to 4 carbon atoms, radical, or else
- a 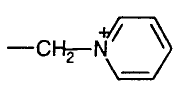 radical,
R₁ and R₂, which are identical or different, represent a hydrogen or halogen atom or an alkyl radical containing 1 to 4 carbon atoms, an alkyloxy radical containing 1 to 4 carbon atoms optionally substituted by a dialkylamino radical in which each alkyl part contains 1 to 4 carbon atoms or forms, with the nitrogen atom, a saturated heterocycle containing 5 or 6 carbon atoms, an alkylthio radical containing 1 to 4 carbon atoms or an alkyloxycarbonyl radical in which the alkyl part contains 1 to 4 carbon atoms, or else R₁ and R₂, situated in the ortho position with respect to one another, form a saturated or unsaturated heterocycle containing 1 or 2 heteroatoms chosen from nitrogen and oxygen, optionally substituted by a halogen atom or by an alkyl radical containing 1 to 4 carbon atoms or an alkyloxy radical containing 1 to 4 carbon atoms, or else R₁ represents a hydrogen or halogen atom or an alkyl radical containing 1 to 4 carbon atoms, an alkyloxy radical containing 1 to 4 carbon atoms optionally substituted by a dialkylamino radical in which each alkyl part contains 1 to 4 carbon atoms, an alkylthio radical containing 1 to 4 carbon atoms or an alkyloycarbonyl radical in which the alkyl part contains 1 to 4 carbon atoms and R₂ represents a thioalkyl radical containing 1 to 4 carbon atoms, it being understood that, in this case, the product of general formula (I) exists in the form of a product twinned via a disulphide bridge,
R₃ represents a hydrogen or halogen atom or an alkyl radical containing 1 to 4 carbon atoms, an alkenyl radical containing 2 to 4 carbon atoms, an alkyloxy radical containing 1 to 4 carbon atoms or an alkylthio radical containing 1 to 4 carbon atoms,
X represents an oxygen or sulphur atom or an -NH-, -CO-, methylene, ethylene, alkylidene, such as vinylidene, or 1,1-cycloalkyl group containing 3 to 6 carbon atoms, and
Y represents an oxygen or sulphur atom,
in the racemic form, and the optical isomers of the product of general formula (I).

2. Products according to claim 1, for which one of the R₁ and R₂ symbols represents a hydrogen atom and the other represents an alkyloxy radical containing from 1 to 4 carbon atoms, R₃ represents a hydrogen atom, X represents a methylene radical, Y represents an oxygen atom and R represents a radical of general formula -(CH₂)ₘ-X₁-(CH₂)ₙ-Z, in which m is equal to 0 or 1, X₁ represents a bond and n is equal to 0 and Z represents a carboxyl radical or a -COOR₄ radical for which R₄ represents an alkyl radical containing 1 to 3 carbon atoms or a CON(R₅)(R₆) radical in which R₅ and R₆ are defined as in claim 1.

3. Product according to claim 1, characterized in that it is (3aS,4R,9R,9aS)-4,9-ethano-2-[2-(2-methoxyphenyl)prop-2-en-1-oyl]-9-phenyl-1,2,3,3a,9,9a-hexahydro-1H-benzo[f]isoindole-3a-carboxylic acid, the dextrorotatory enantiomer, optionally in the form of an ester containing 1 to 3 carbon atoms.

4. Product according to claim 1, characterized in that it is N-[(S)-t-butyl-phenylglycinoyl)]-(3aS,4R,9R,9aS)-4,9-ethano-2-[(2-methoxyphenyl)prop-2-en-1-oyl]-9-phenyl-2,3,3a,4,9,9a-hexahydro-1H-benzo[f]isoindole-3a-carboxamide.

5. Product according to claim 1, characterized in that it is (3aS,4R,9R,9aS)-N-[(3-pyridyl)methyl]-4,9-ethano-2-[(∼-methoxyphenyl)prop-2-en-1-oyl]-9-phenyl-2,3,3a,4,9,9a-hexahydro-1H-benzo[f]isoindole-3a-carboxamide.

6. Product according to claim 1, characterized in that it is (3aS,4R,9R,9aS)-N-[(2-pyridyl)methyl]-4,9-ethano-2-[(2-methoxyphenyl)prop-2-en-1-oyl]-9-phenyl-2,3,3a,4,9,9a-hexahydro-1H-benzo[f]isoindole-3a-carboxamide.

7. Process for the preparation of a product according to one of claims 1, 2, 3, 4, 5 or 6 in which R, R₁, R₂ and R₃ are defined as in either of claims 1 and 2, Y represents an oxygen or sulphur atom and X represents a -CO-, methylene, ethylene, alkylidene or 1,1-cycloalkyl group, characterized in that an acid of general formula: 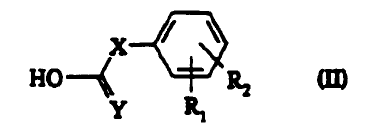 in which R₁, R₂ and X are defined as above and Y represents an oxygen or sulphur atom, or its methyl ester or of a derivative of this acid, such as a halide or the anhydride, is reacted with a product of general formula: 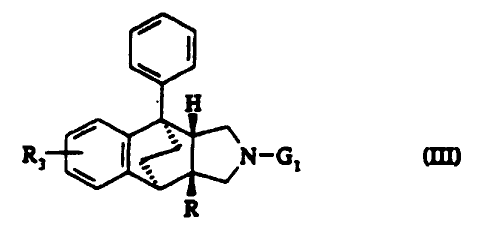 in which R and R₃ are defined as above and G₁ represents a hydrogen atom, and then optionally, when R represents or contains a -COOR₄ or -PO(OR₇)₂ radical in which R₄ and R₇ represent an alkyl radical, by the saponification of the product obtained, to obtain a product of general formula (I) in which R represents or contains a carboxyl radical, or the product obtained [lacuna] converted, by means of a nucleophilic agent, to a product of general formula (I) in which R represents or contains a -PO₃H₂ radical.

8. Process for the preparation of a product according to either of claims 1 and 2 in which Y represents an oxygen or sulphur atom and X represents an oxygen atom, characterized in that a haloformate or halothioformate of general formula: 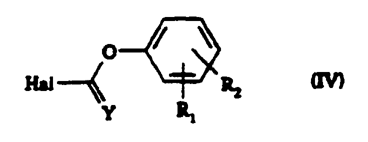 in which Y represents an oxygen or sulphur atom, R₁ and R₂ are defined as in either of claims 1 and 2 and Hal represents a halogen atom, is reacted with a product of general formula (III), and then, optionally, when R represents or contains a -COOR₄ or PO₃(R₇)₂ radical in which R₄ and R₇ represent an alkyl radical, the product obtained is saponified, to obtain a product of general formula (I) in which R represents or contains a carboxyl radical, or the product obtained is converted, by means of a nucleophilic agent, to a product of general formula (I) in which R represents or contains a -PO₃H₂ radical.

9. Process for the preparation of a product according to either of claims 1 and 2 in which Y represents an oxygen or sulphur atom and X represents an NH group, characterized in that an isocyanate or an isothiocyanate of general formula: 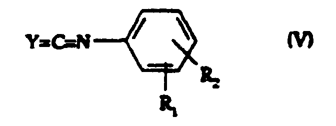 in which Y represents an oxygen or sulphur atom and R₁ and R₂ are defined as in either of claims 1 and 2, is reacted with a product of general formula (III) in which G₁ represents a hydrogen atom and then optionally, when R represents or contains a -COOR₄ or -PO₃(R₇)₂ radical in which R₄ and R₇ represent an alkyl radical, the product obtained is saponified, to obtain a product of general formula (I) in which R represents or contains a carboxyl radical, or the product obtained is converted, by means of a nucleophilic agent, to a product of general formula (I) in which R represents or contains a -PO₃H₂ radical.

10. Process for the preparation of a product according to either of claims 1 and 2 in which R₁, R₂ and R₃ are defined as in either of claims 1 and 2, X represents an oxygen or sulphur atom or an -NH-, -CO-, methylene, ethylene, alkylidene or 1,1-cycloalkyl group and Y represents an oxygen or sulphur atom and R represents a radical of general formula -(CH₂)ₘ-X₁-(CH₂)ₙ-Z in which X₁, m and n are defined as in either of claims 1 and 2 and Z represents a -COOR₄ radical in which R₄ represents a straight or branched alkyl radical containing 1 to 3 carbon atoms, characterized in that the esterification is carried out of a product of general formula (I) in which R₁, R₁, R₃, X, Y, X₁, m and n are defined as above and Z represents a carboxyl radical.

11. Process for the preparation of a product according to claim 1 in which R₁, R₂ and R₃ are defined as in claim 1, X represents an oxygen or sulphur atom or an -NH-, -CO-, methylene, ethylene, alkylidene or 1,1-cycloalhyl group and Y represents an oxygen or sulphur atom and R represents a radical of general formula -(CH₂)ₘ-X₁-(CH₂)ₙ-Z in which X₁, m and n are defined as in claim 1 and Z represents a CON(R₅)(R₆) radical in which which R₅ and R₆, which are identical or different, represent a hydrogen atom or a straight or branched alkyl radical containing 1 to 6 carbon atoms and R₆ represents a hydrogen atom or a straight or branched alkyl radical containing 1 to 6 carbon atoms optionally substituted by an amino, alkylamino containing 1 to 4 carbon atoms, dialkylamino in which each alkyl part contains 1 to 4 carbon atoms, alkoxy containing 1 to 4 carbon atoms, alkylthio containing 1 to 4 carbon atoms, alkyloxycarbonyl in which the alkyl part contains 1 to 4 carbon atoms, carboxyl, cyano, phenyl optionally substituted by one or a number of identical or different radicals chosen from alkoxy radicals containing 1 to 4 crabon atoms or trifluoromethyl radicals, 1- or 2-naphthyl, 2- or 3-furyl, 2- or 3-thienyl, 4- or 5-imidazolyl, 4- or 5-thiazolyl or 2-, 3- or 4-pyridyl radical or an indanyl or chromanyl radical or else R₅ represents a hydrogen atom or a straight or branched alkyl radical containing 1 to 6 carbon atoms and R₆ represents a hydroxyl radical, an amino radical or an alkyloxy radical containing 1 to 6 straight- or branched-chain carbon atoms optionally substituted by a phenyl radical, characterized in that a product of general formula HN(R₅)(R₆), in which R₅ and R₆ are defined as above, is reacted with a product of general formula (I) in which R₁, R₂, R₃, X, Y, X₁, m and n are defined as above and Z represents a carboxyl radical.

12. Process for the preparation of a product according to claim 1 in which R₁, R₂ and R₃ are defined as in claim 1, X represents an oxygen or sulphur atom or an -NH-, -CO-, methylene, ethylene, alkylidene or 1,1-cycloalkyl group and Y represents an oxygen or sulphur atom and R represents a radical of general formula -NHCO-T in which T represents a hydrogen atom or an alkyl radical containing 1 to 6 carbon atoms optionally substituted by an amino, carboxyl, alkyloxycarbonyl, hydroxyl, alkuyloxy, mercapto or alkylthio radical, characterized in that an acid of general formula T-CO-OH, in which T is defined as above, is reacted with a product of general formula: 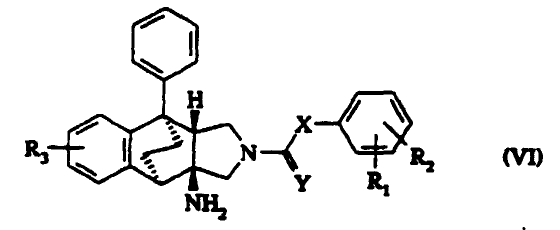 in which R₁, R₂, R₃, X and Y are defined as above, and then, optionally, the protected ester functional groups or protected amine functional groups carried by T are replaced by carboxyl or amino radicals respectively.

13. Process for the preparation of a product according to claim 1 in which R represents a radical of general formula 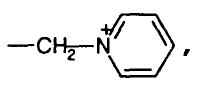 characterized in that an excess of a tertiary amine and of a strong acid or of a derivative of this acid are reacted with a product of general formula: 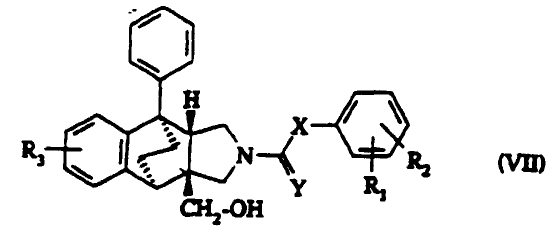 in which R₁, R₂, R₃, X and Y are defined as in claim 1.

14. Process for the preparation of a product according to claim 1 in which Y represents a sulphur atom, characterized in that the thionation is carried out of a product according to claim 1 in which Y represents an oxygen atom.

15. Pharmaceutical composition, characterized in that it contains at least one product according to one of claims 1, 2, 3, 4, 5 or 6, in combination with one or a number of pharmaceutically acceptable diluents or adjuvants, which may be either inert or biologically active.

## Patentansprüche

1. Produkte der allgemeinen Formel (I) 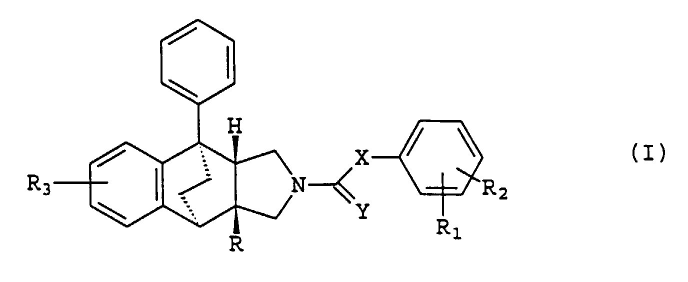 in der
R darstellt:
- einen Rest der allgemeinen Formel -(CH₂)ₘ-X₁-(CH₂)ₙ-Z, worin
- X₁ eine einfache Bindung oder ein Sauerstoffatom oder ein Schwefelatom darstellt, m eine ganze Zahl von 0 oder 1 ist und n eine ganze Zahl von 0, 1 oder 2 ist, die methylenischen Reste durch einen Rest Carboxy, Alkoxycarbonyl, dessen Teil Alkyl 1 bis 4 Kohlenstoffatome enthält, Carbamoyl, Alkylcarbamoyl, dessen Teil Alkyl 1 bis 4 Kohlenstoffatome enthalt, Dialkylcarbamoyl, bei dem jeder Teil Alkyl 1 bis 4 Kohlenstoffatome enthält, Amino, Alkylamino mit 1 bis 4 Kohlenstoffatomen, Dialkylamino, bei dem jeder Teil Alkyl 1 bis 4 Kohlenstoffatome enthalt, substituiert sein können, und
- Z bedeutet
- einen Rest Carboxy,
- einen Rest COOR₄, worin R₄ ein gerader oder verzweigter Rest Alkyl mit 1 bis 3 Kohlenstoffatomen ist, oder
- einen Rest CON(R₅)(R₆), worin R₅ ein Wasserstoffatom oder einen geraden oder verzweigten Rest Alkyl mit 1 bis 6 Kohlenstoffatomen darstellt und R₆ ein Wasserstoffatom oder einen geraden oder verzweigten Rest Alkyl mit 1 bis 6 Kohlenstoffatomen bedeutet, gegebenenfalls substituiert durch einen Rest Amino, Alkylamino mit 1 bis 4 Kohlenstoffatomen, Dialkylamino, bei dem jeder Teil Alkyl 1 bis 4 Kohlenstoffatome enthalt, Hydroxy, Alkoxy mit 1 bis 4 Kohlenstoffatomen, Mercapto, Alkylthio mit 1 bis 4 Kohlenstoffatomen, Alkyloxycarbonyl, dessen Teil Alkyl 1 bis 4 Kohlenstoffatome enthalt, Carboxy, Cyano, Phenyl, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen, Dialkylamino, bei dem jeder Teil Alkyl 1 bis 4 Kohlenstoffatome enthalt, Trifluormethyl oder Cyano,
1-Naphthyl oder 2-Naphthyl, 2-Furyl oder 3-Furyl, 2-Thienyl oder 3-Thienyl, 4-Imidazolyl oder 5-Imidazolyl, 2-Thiazolyl, 4-Thiazolyl oder 5-Thiazolyl, 2-Thiazolidinyl, 4-Thiazolidinyl oder 5-Thiazolidinyl oder 2-Pyridyl, 3-Pyridyl oder 4-Pyridyl, oder einen Rest Indanyl oder Thiochromanyl, oder auch R₅ ein Wasserstoffatom oder einen geraden oder verzweigten Rest Alkyl mit 1 bis 6 Kohlenstoffatomen darstellt und R₆ einen Rest Hydroxy, Amino oder Alkyloxy mit 1 bis 6 Kohlenstoffatomen in gerader oder verzweigter Kette bedeutet, gegebenenfalls substituiert durch einen Rest Phenyl, oder
- einen Rest PO(OR₇)₂, worin R₇ ein Wasserstoffatom oder ein gerader oder verzweigter Rest Alkyl mit 1 bis 6 Kohlenstoffatomen ist, oder
- einen Rest -NH-CO-T, worin T ein Wasserstoffatom oder einen geraden oder verzweigten Rest Alkyl mit 1 bis 6 Kohlenstoffatomen darstellt, gegebenenfalls substituiert durch einen Rest Amino, Carboxy, Alkyloxycarbonyl, dessen Teil Alkyl 1 bis 4 Kohlenstoffatome enthalt, Hydroxy, Alkyloxy mit 1 bis 4 Kohlenstoffatomen, Mercapto oder Alkylthio mit 1 bis 4 Kohlenstoffatomen, oder auch
- einen Rest 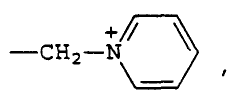
R₁ und R₂, gleich oder verschieden, ein Wasserstoffatom oder ein Halogenatom oder einen Rest Alkyl mit 1 bis 4 Kohlenstoffatomen, Alkyloxy mit 1 bis 4 Kohlenstoffatomen, gegebenenfalls substituiert durch einen Rest Dialkylamino, bei dem jeder Teil Alkyl 1 bis 4 Kohlenstoffatome enthalt oder auch mit dem Stickstoffatom einen gesättigten Heterocyclus mit 5 oder 6 Kohlenstoffatomen bildet, Alkylthio mit 1 bis 4 Kohlenstoffatomen, Alkyloxycarbonyl, dessen Teil Alkyl 1 bis 4 Kohlenstoffatome enthält, darstellen, oder
R₁ und R₂, zueinander in Stellung ortho liegend, einen gesättigten oder ungesättigten Heterocyclus mit 1 oder 2 Heteroatomen, ausgewählt unter Stickstoff und Sauerstoff, bilden, gegebenenfalls substituiert durch ein Halogenatom oder durch einen Rest Alkyl mit 1 bis 4 Kohlenstoffatomen oder Alkyloxy mit 1 bis 4 Kohlenstoffatomen, oder
R₁ ein Wasserstoffatom oder Halogenatom oder einen Rest Alkyl mit 1 bis 4 Kohlenstoffatomen, Alkyloxy mit 1 bis 4 Kohlenstoffatomen, gegebenenfalls substituiert durch einen Rest Dialkylamino, bei dem jeder Teil Alkyl 1 bis 4 Kohlenstoffatome enthält, Alkylthio mit 1 bis 4 Kohlenstoffatomen, Alkyloxycarbonyl, dessen Teil Alkyl 1 bis 4 Kohlenstoffatome enthalt, darstellt, und
R₂ einen Rest Thioalkyl mit 1 bis 4 Kohlenstoffatomen bedeutet, mit der Maßgabe, daß in diesem Fall das Produkt der allgemeinen Formel (I) in Form eines Produktes vorliegt, das über das Zwischenglied einer Disulfid-Brücke verdoppelt ist,
R₃ ein Wasserstoffatom oder ein Halogenatom oder einen Rest Alkyl mit 1 bis 4 Kohlenstoffatomen, Alkenyl mit 2 bis 4 Kohlenstoffatomen, Alkyloxy mit 1 bis 4 Kohlenstoffatomen oder Alkylthio mit 1 bis 4 Kohlenstoffatomen darstellt,
X ein Sauerstoffatom oder ein Schwefelatom oder eine Gruppe -NH-, -CO-, Methylen, Ethylen, Alkyliden wie Vinyliden oder 1,1-Cycloalkyl mit 3 bis 6 Kohlenstoffatomen bedeutet, und
Y ein Sauerstoffatom oder ein Schwefelatom ist,
in racemischer Form sowie die optischen Isomeren des Produktes der allgemeinen Formel (I).

2. Produkte nach Anspruch 1, worin eines der Symbole R₁ und R₂ ein Wasserstoffatom ist und das andere einen Rest Alkyloxy mit 1 bis 4 Kohlenstoffatomen darstellt, R₃ ein Wasserstoffatom ist, X einen Rest Methylen bedeutet, Y ein Sauerstoffatom ist und R einen Rest der allgemeinen Formel -(CH₂)ₘ-X₁-(CH₂)ₙ-Z darstellt, worin m gleich 0 oder 1 ist, X₁ eine Bindung bedeutet und n gleich 0 ist und Z einen Rest Carboxy oder einen Rest -COOR₄ darstellt, worin R₄ ein Rest Alkyl mit 1 bis 3 Kohlenstoffatomen oder ein Rest CON(R₅)(R₆) ist, worin R₅ und R₆ wie in Anspruch 1 definiert sind.

3. Produkt nach Anspruch 1, dadurch gekennzeichnet, daß es sich um die 4,9-Ethano-2-[2-(2-methoxyhenyl)-prop-2-en-1-oyl]-9-phenyl-1,2,3,3a,9,9a-hexahydro-1H-benzo[f]-isoindol-3a-carbonsäure-(3aS,4R,9R,9aS), rechtsdrehendes Enantiomer, handelt, gegebenenfalls in Form von Ester mit 1 bis 3 Kohlenstoffatomen.

4. Produkt nach Anspruch 1, dadurch gekennzeichnet, daß es sich um das N-[(S)-tert.-Butyl-phenylglycinoyl]-4,9-ethano-2-[(2-methoxyphenyl)-prop-2-en-1-oyl]-9-phenyl-2,3,3a,4,9,9a-hexahydro-1H-benzo[f]-isoindol-3a-carboxamid-(3aS,4R,9R,9aS) handelt.

5. Produkt nach Anspruch 1, dadurch gekennzeichnet, daß es sich um das N-[(3-Pyridyl)-methyl]-4,9-ethano-2-[(2-methoxyphenyl)-prop-2-en-1-oyl]-9-phenyl-2,3,3a,4,9,9a-hexahydro-1H-benzo[f]-isoindol-3a-carboxamid-(3aS,4R,9R,9aS) handelt.

6. Produkt nach Anspruch 1, dadurch gekennzeichnet, daß es sich um das N-[(2-Pyridyl)-methyl]-4,9-ethano-2-[(2-methoxyphenyl)-prop-2-en-1-oyl]-9-phenyl-2,3,3a,4,9,9a-hexahydro-1H-benzo[f]-isoindol-3a-carboxamid-(3aS,4R,9R,9aS) handelt.

7. Verfahren zur Herstellung eines Produktes nach einem der Ansprüche 1, 2, 3, 4, 5 oder 6, worin R, R₁, R₂ und R₃ wie in einem der Ansprüche 1 oder 2 definiert sind, Y ein Sauerstoffatom oder ein Schwefelatom darstellt und X eine Gruppe -CO-, Methylen, Ethylen, Alkyliden oder 1,1-Cycloalkyl bedeutet, dadurch gekennzeichnet, daß man eine Saure der allgemeinen Formel (II) 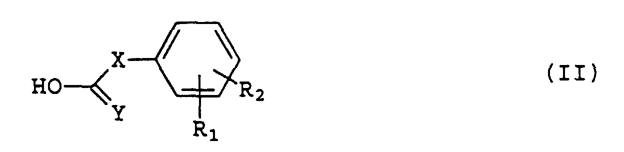 in der R₁, R₂ und X wie vorstehend definiert sind und Y ein Sauerstoffatom oder ein Schwefelatom ist, oder ihren Methylester oder ein Derivat dieser Saure wie ein Halogenid oder das Anhydrid, mit einem Produkt der allgemeinen Formel (III) 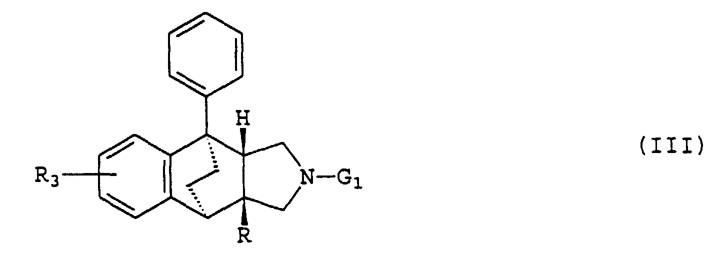 zur Reaktion bringt, in der R und R₃ wie vorstehend definiert sind und G₁ ein Wasserstoffatom ist, und man anschließend gegebenenfalls, wenn R einen Rest -COOR₄ oder -PO(OR₇)₂, worin R₄ und R₇ einen Rest Alkyl bedeuten, darstellt oder einen solchen enthält, die Verseifung des erhaltenen Produktes durchführt, um ein Produkt der allgemeinen Formel (I) zu erhalten, in der R einen Rest Carboxy darstellt oder einen solchen enthält, oder das erhaltene Produkt mit Hilfe eines nucleophilen Mittels in ein Produkt der allgemeinen Formel (I) umwandelt, in der R einen Rest -PO₃H₂ darstellt oder einen solchen enthält.

8. Verfahren zur Herstellung eines Produktes nach einem der Ansprüche 1 oder 2, worin Y ein Sauerstoffatom oder ein Schwefelatom darstellt und X ein Sauerstoffatom ist, dadurch gekennzeichnet, daß man einen Halogenameisensäureester oder einen Halogenthioameisensäureester der allgemeinen Formel (IV) 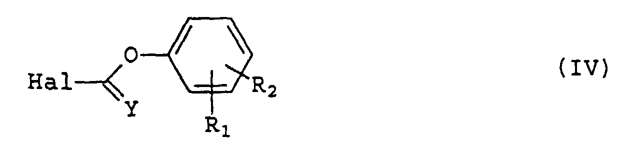 in der Y ein Sauerstoffatom oder ein Schwefelatom ist, R₁ und R₂ wie in einem der Ansprüche 1 oder 2 definiert sind und Hal ein Halogenatom bedeutet, mit einem Produkt der allgemeinen Formel (III) zur Reaktion bringt, und man anschließend gegebenenfalls, wenn R einen Rest -COOR₄ oder -PO₃(R₇)₂, worin R₄ und R₇ einen Rest Alkyl bedeuten, darstellt oder einen solchen enthalt, die Verseifung des erhaltenen Produktes durchführt, um ein Produkt der allgemeinen Formel (I) zu erhalten, in der R einen Rest Carboxy darstellt oder einen solchen enthält, oder das erhaltene Produkt mit Hilfe eines nucleophilen Mittels in ein Produkt der allgemeinen Formel (I) umwandelt, in der R einen Rest -PO₃H₂ darstellt oder einen solchen enthält.

9. Verfahren zur Herstellung eines Produktes nach einem der Ansprüche 1 oder 2, worin Y ein Sauerstoffatom oder ein Schwefelatom ist und X eine Gruppe NH darstellt, dadurch gekennzeichnet, daß man ein Isocyanat oder ein Isothiocyanat der allgemeinen Formel (V) 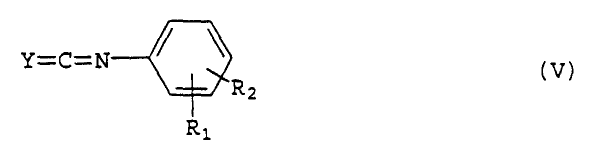 in der Y ein Sauerstoffatom oder ein Schwefelatom ist, R₁ und R₂ wie in einem der Ansprüche 1 oder 2 definiert sind, mit einem Produkt der allgemeinen Formel (III) zur Reaktion bringt, in der G₁ ein Wasserstoffatom ist, und man anschließend gegebenenfalls, wenn R einen Rest -COOR₄ oder -PO₃(R₇)₂, worin R₄ und R₇ einen Rest Alkyl bedeuten, darstellt oder einen solchen enthält, die Verseifung des erhaltenen Produktes durchführt, um ein Produkt der allgemeinen Formel (I) zu erhalten, in der R einen Rest Carboxy darstellt oder einen solchen enthält, oder das erhaltene Produkt mit Hilfe eines nucleophilen Mittels in ein Produkt der allgemeinen Formel (I) umwandelt, in der R einen Rest -PO₃H₂ darstellt oder einen solchen enthält.

10. Verfahren zur Herstellung eines Produktes nach einem der Ansprüche 1 oder 2, worin R₁, R₂ und R₃ wie in einem der Ansprüche 1 oder 2 definiert sind, X ein Sauerstoffatom oder ein Schwefelatom oder eine Gruppe -NH-, -CO-, Methylen, Ethylen, Alkyliden oder 1,1-Cycloalkyl darstellt und Y ein Sauerstoffatom oder ein Schwefelatom ist und R einen Rest der allgemeinen Formel -(CH₂)ₘ-X₁-(CH₂)ₙ-Z bedeutet, in der X₁, m und n wie in einem der Ansprüche 1 oder 2 definiert sind und Z einen Rest -COOR₄ darstellt, worin R₄ ein gerader oder verzweigter Rest Alkyl mit 1 bis 3 Kohlenstoffatomen ist, dadurch gekennzeichnet, daß man ein Produkt der allgemeinen Formel (I), in der R₁, R₂, R₃, X, Y, X₁, m und n wie vorstehend definiert sind und Z ein Rest Carboxy ist, verestert.

11. Verfahren zur Herstellung eines Produktes nach Anspruch 1, worin R₁, R₂ und R₃ wie in Anspruch 1 definiert sind, X ein Sauerstoffatom oder ein Schwefelatom oder eine Gruppe -NH-, -CO-, Methylen, Ethylen, Alkyliden oder 1,1-Cycloalkyl darstellt und Y ein Sauerstoffatom oder ein Schwefelatom ist und R einen Rest der allgemeinen Formel -(CH₂)ₘ-X₁-(CH₂)ₙ-Z bedeutet, in der X₁, m und n wie in Anspruch 1 definiert sind und Z einen Rest -CON(R₅)(R₅) darstellt, worin R₅ und R₆, gleich oder verschieden, ein Wasserstoffatom oder einen geraden oder verzweigten Rest Alkyl mit 1 bis 6 Kohlenstoffatomen darstellen und R₆ ein Wasserstoffatom oder einen geraden oder verzweigten Rest Alkyl mit 1 bis 6 Kohlenstoffatomen bedeutet, gegebenenfalls substituiert durch einen Rest Amino, Alkylamino mit 1 bis 4 Kohlenstoffatomen, Dialkylamino, bei dem jeder Teil Alkyl 1 bis 4 Kohlenstoffatome enthält, Alkoxy mit 1 bis 4 Kohlenstoffatomen, Alkylthio mit 1 bis 4 Kohlenstoffatomen, Alkyloxycarbonyl, dessen Teil Alkyl 1 bis 4 Kohlenstoffatome enthält, Carboxy, Cyano, Phenyl, gegebenenfalls substituiert durch ein oder mehrere, gleiche oder verschiedene Reste, ausgewählt unter den Resten Alkoxy mit 1 bis 4 Kohlenstoffatomen oder Trifluormethyl, 1-Naphthyl oder 2-Naphthyl, 2-Furyl oder 3-Furyl, 2-Thienyl oder 3-Thienyl, 4-Imidazolyl oder 5-Imidazolyl oder 4-Thiazolyl oder 5-Thiazolyl, 2-Pyridyl, 3-Pyridyl oder 4-Pyridyl, oder einen Rest Indanyl oder Chromanyl, oder auch R₅ ein Wasserstoffatom oder einen geraden oder verzweigten Rest Alkyl mit 1 bis 6 Kohlenstoffatomen darstellt und R₆ einen Rest Hydroxy, Amino oder Alkyloxy mit 1 bis 6 Kohlenstoffatomen in gerader oder verzweigter Kette bedeutet, gegebenenfalls substituiert durch einen Rest Phenyl, dadurch gekennzeichnet, daß man ein Produkt der allgemeinen Formel HN(R₅)(R₆), in der R₅ und R₆ wie oben definiert sind, mit einem Produkt der allgemeinen Formel (I) zur Reaktion bringt, in der R₁, R₂, R₃, X, Y, X₁, m und n wie vorstehend definiert sind und Z ein Rest Carboxy ist.

12. Verfahren zur Herstellung eines Produktes nach Anspruch 1, worin R₁, R₂ und R₃ wie in Anspruch 1 definiert sind, X ein Sauerstoffatom oder ein Schwefelatom oder eine Gruppe -NH-, -CO-, Methylen, Ethylen, Alkyliden oder 1,1-Cycloalkyl darstellt und Y ein Sauerstoffatom oder ein Schwefelatom ist und R einen Rest der allgemeinen Formel -NHCO-T darstellt, in der T ein Wasserstoffatom oder einen Rest Alkyl mit 1 bis 6 Kohlenstoffatomen darstellt, gegebenenfalls substituiert durch einen Rest Amino, Carboxy, Alkyloxycarbonyl, Hydroxy, Alkyloxy, Mercapto oder Alkylthio, dadurch gekennzeichnet, daß man eine Saure der allgemeinen Formel T-CO-OH, in der T wie oben definiert ist, mit einem Produkt der allgemeinen Formel (VI) 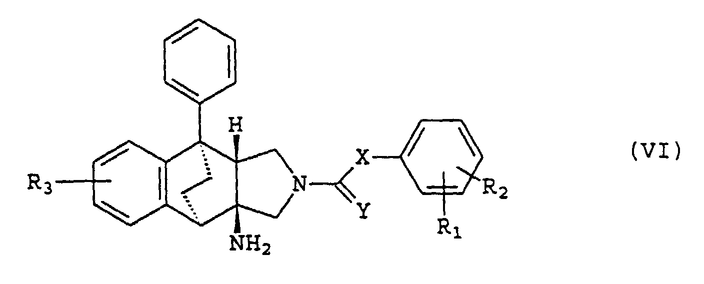 zur Reaktion bringt, in der R₁, R₂, R₃, X und Y wie vorstehend definiert sind, und man anschließend gegebenenfalls die von T getragenen, geschützten Esterfunktionen oder Aminfunktionen jeweils durch Reste Carboxy oder Amino ersetzt.

13. Verfahren zur Herstellung eines Produktes nach Anspruch 1, worin R einen Rest der allgemeinen Formel 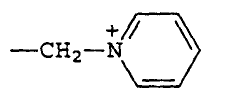 darstellt, dadurch gekennzeichnet, daß man einen Überschuß eines tertiären Amins und einer starken Säure oder einem Derivat dieser Säure mit einem Produkt der allgemeinen Formel (VII) 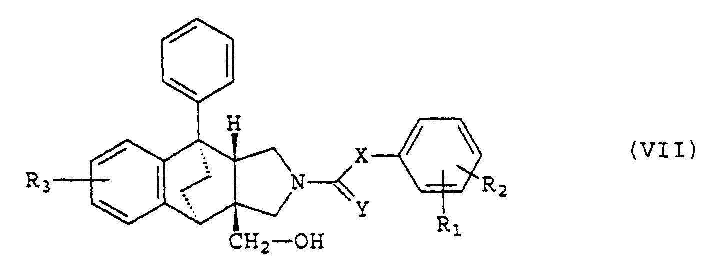 zur Reaktion bringt, in der R₁, R₂, R₃, X und Y wie in Anspruch 1 definiert sind.

14. Verfahren zur Herstellung eines Produktes nach Anspruch 1, worin Y ein Schwefelatom ist, dadurch gekennzeichnet, daß man die Thionierung eines Produktes nach Anspruch 1 durchführt, worin Y ein Sauerstoffatom ist.

15. Pharmazeutische Zusammensetzung, dadurch gekennzeichnet, daß sie mindestens ein Produkt nach einem der Ansprüche 1, 2, 3, 4, 5 oder 6 in Assoziation mit einem oder mehreren pharmazeutisch akzeptablen Verdünnungsmitteln oder Zusatzstoffen enthält, die inert oder biologisch aktiv sind.
